(19) Europäisches Patentamt
European Patent Office
Office européen des brevets

(11) EP 2 429 292 B1

(12) **EUROPEAN PATENT SPECIFICATION**

(45) Date of publication and mention of the grant of the patent:
**03.04.2019 Bulletin 2019/14**

(21) Application number: **10772877.6**

(22) Date of filing: **07.05.2010**

(51) Int Cl.:
***A01N 43/16*** (2006.01) ***A61K 31/35*** (2006.01)
***A61P 35/00*** (2006.01)

(86) International application number:
**PCT/US2010/034020**

(87) International publication number:
**WO 2010/129858 (11.11.2010 Gazette 2010/45)**

(54) **COMPOUNDS AND COMPOSITIONS COMPRISING CDK INHIBITORS AND THEIR USE IN THE TREATMENT OF CANCER**

VERBINDUNGEN UND ZUSAMMENSETZUNGEN MIT CDK-HEMMERN UND DEREN VERWENDUNG ZUR BEHANDLUNG VON KREBS

COMPOSÉS ET COMPOSITIONS COMPRENANT DES INHIBITEURS DES CDK ET LEURS UTILISATION DANS LE TRAITEMENT DU CANCER

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **08.05.2009 US 176760 P**

(43) Date of publication of application:
**21.03.2012 Bulletin 2012/12**

(73) Proprietors:
- **Georgia State University Research Foundation Atlanta, GA 30303 (US)**
- **Shandong University Jinan, Shandong 250100 (CN)**

(72) Inventors:
- **LI, Yan Ling Qingdao Shandong Province 266510 (CN)**
- **HOA, Fang Ji'nan 250012 (CN)**
- **XU, Wen, Fang Ji'nan 250010 (CN)**
- **WANG, Binghe Marietta, GA 30062 (US)**

(74) Representative: **Leißler-Gerstl, Gabriele et al**
**Hoefer & Partner**
**Patentanwälte mbB**
**Pilgersheimer Strasse 20**
**81543 München (DE)**

(56) References cited:
**EP-A1- 0 366 061**
**US-A- 5 234 951**
**US-A1- 2004 106 581**
**US-A1- 2006 247 305**
**US-A1- 2007 249 051**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 429 292 B1

## Description

## FIELD OF THE DISCLOSURE

Disclosed herein are compounds suitable for use as antitumor agents. Further disclosed are methods for treating cancer wherein the disclosed compounds arc used in making a medicament for the treatment of cancer. Also disclosed herein are methods for treating a tumor comprising, administering to a subject a composition comprising one or more of the disclosed cytotoxic agents. Yet further disclosed are methods for preparing the disclosed antitumor agents.

## BACKGROUND

The naturally occurring class of compounds, which can be loosely thought of as derivatives or variants of 2-phenyl-1,4-benzopyrone and 2-phenyl-4*H*-chromene, are known generically as "flavonoids." These compounds are ubiquitous throughout the plant kingdom and are responsible for many of the diverse properties of plants, for example, the coloring of fruits, *e.g.,* the red or blue of grape and berry skins. It is, however, the biological properties of flavonoids that have drawn the attention of pharmaceutical researchers. Foods containing various flavonoids have been long understood to play a central role in providing cellular anti-oxidant protection and scientists, as well as nutritionists have sought to take advantage of these properties.

Ren and co-workers have shown that morelloflavone, a biflavonoid extracted from *Garcinia dulcis,* was shown to "inhibit tumor growth *in vivo* via their anti-angiogenic activity at a much lower concentration and much earlier than their cytotoxicity effects on tumor cells." (Ren, W., et al., "Flavonoids: promising anticancer agents." Med Res Rev, 2003, 23(4): pp. 519-34.) Other studies have investigated flavonoids for their potential inhibition of HIV-1 reverse transcriptase, protease, and integrase (Wang, H.K., et al., "Recent advances in the discovery and development of flavonoids and their analogues as antitumor and anti-HIV agents." Adv. Exp. Med. Biol., 1998. 439: pp. 191-225). As such, the potential of flavonoids as anti-angiogenic agents useful against tumors has been studied (Wang, H.K., "The therapeutic potential of flavonoids. " Expert Opin. Investig. Drugs, 2000. 9(9): pp. 2103-19; Zhao, L., et al., "Mechanisms of tumor vascular shutdown induced by 5,6-dimethylxanthenone-4-acetic acid (DMXAA): Increased tumor vascular permeability." Int. J. Cancer, 2005. 116(2): pp. 322-6; and Gallo, D., et al., "Antitumour activity of the silybin-phosphatidylcholine complex, IdB 1016, against human ovarian cancer." Eur. J. Cancer, 2003. 39(16): pp. 2403-10).

Flavonoids, however, comprise twelve recognized chemical classes: flavones, isoflavones, flavans, flavanones, flavanols, flavanolols, anthocyanidins, catechins (including proanthocyanidins), leukoanthocyanidins, chalcones, dihydrochalcones, and aurones. Flavonoids therefore provide a wide array of biologically active chemical structures from which researchers can choose to investigate. One type of flavonoid, chalcones, can be intuitively considered to be an open form of several fused ring flavonoids. For example, 2-hydroxychalcone ((*E*)-3-(2-hydroxyphenyl)-1-phenylprop-2-en-1-one) can be considered to be the open ring form, or non-conformationally restricted form, of flavone, 2-phenyl-1,4-benzopyrone. Chalcones, like other flavonoids, have been investigated for their bioactive properties (Dimmock, J.R., et al., "Bioactivities of chalcones." Curr. Med. Chem., 1999, 6(12): pp. 1125-49).

Flavopiridol, derived from a medicinal plant from India and used for centuries in many indigenous medicines, is presently under investigation for a variety of solid tumors, *inter alia,* breast and lung cancer, as well as hematological cancers. Researchers have discovered that flavopiridol blocks cells *in vitro* from progressing from stages G1/S to G2/M in certain cell lines, thus affecting the entire cellular cycle. Moreover, flavopiridol has been shown to enhance the cytotoxic effect of conventional chemotherapeutic agents in gastric and breast cancer cell lines.

US 2004/0106581 A1 discloses compounds for the inhibition of cyclin-dependent kinases, and more particularly, to chromenone derivatives of the general formula:

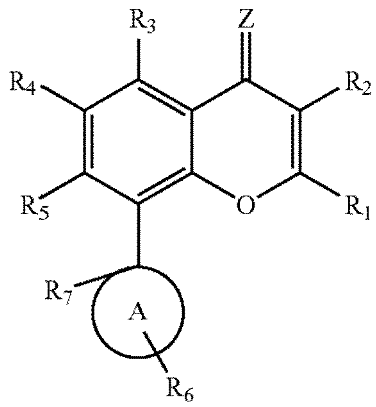

,

It is also disclosed that the compounds are suitable for use in the treatment and prophylaxis of diseases, which can be treated or prevented by the inhibition of cyclin-dependent kinases, such as cancer.

Although current research has indicated that both naturally occurring and synthetic flavonoids have a potential for use in cancer therapy, no single candidate or category of flavonoid compounds has been shown to serve as a definitive antitumor agent. Therefore, there is a long felt need for antitumor agents, especially agents demonstrating cell dependent kinase (CDK) inhibition and enhanced cytotoxic activity.

## BRIEF DESCRIPTION OF THE FIGURES

**Figure 1** depicts the results of initial cytotoxity testing for selected compounds using the human colon cancer cell line HCT116. Each compound was tested at various concentrations and the absorbance value of the resulting test solutions was measured at 490 nm. In these results, increased absorbance correlates with decreased cytotoxicity as further described herein below. For each example, reading from left to right, the concentrations were 100 μM, 50 μM, 25 μM, 12.5 μM, 6.25 μM and 1 μM, 0μM (control), and blank.

**Figure 2** depicts the results of initial cytotoxity testing for further selected compounds using the human colon cancer cell line HCT116 as described. Each compound was tested at various concentrations and the absorbance value of the resulting test solutions was measured at 490 nm. In these results, increased absorbance correlates with decreased cytotoxicity as further described herein below. For each example, reading from left to right, the concentrations were 100 μM, 50 μM, 25 μM, 12.5 μM, 6.25 μM and 1 μM, μM (control), and blank.

**Figure 3** depicts the results of further cytotoxity testing for selected compounds using the human colon cancer cell line HCT116. Each compound was tested at various concentrations and the absorbance value of the resulting test solutions was measured at 490 nm. In these results, increased absorbance correlates with decreased cytotoxicity as further described herein below. For each example, reading from left to right, the concentrations were 10 μM, 1 μM, 500 nM, 250 nM, 125 nM, 62.5 nM, 0 μM (control), and blank.

**Figure 4** depicts the results of further cytotoxity testing for selected compounds using the human colon cancer cell line HCT116. Each compound was tested at various concentrations and the absorbance value of the resulting test solutions was measured at 490 nm. In these results, increased absorbance correlates with decreased cytotoxicity as further described herein below. For each example, reading from left to right, the concentrations were 10 μM, 1 μM, 500 nM, 250 nM, 125 nM, 62.5 nM, 0 μM (control), and blank.

**Figure 5** shows a graphical representation of the absorbance values for doxorubicin hydrochloride that was used as a positive control.

**Figure 6** depicts the CDK2 inhibitory curve for flavopiridol.

**Figure 7** depicts the effect of (*E*)-3-(2-chlorophenyl)-1-(2-hydroxy-4,6-dimethoxy-3-(1,2,3,6-tetrahydropyridine-4-yl)phenyl)-2-propylene-1-one (B2) on tumor weight in animals having a Human Colon Carcinoma Xenograph. Data points (♦) represent the control animals, data points (■) represent animals given 7.5 mg/kg/d of B2, data points (A) represent animals treated with 2.5 mg/kg/d of B2, and (✱) represent animals receiving doxorubicin.

**Figure 8** depicts the effect of (*E*)-3-(2-chlorophenyl)-1-(2-hydroxy-4,6-dimethoxy-3-(1,2,3,6-tetrahydropyridine-4-yl)phenyl)-2-propylene-1-one (B2) on body weight in animals having a Human Colon Carcinoma Xenograph. Data points (♦) represent the control animals, data points (■) represent animals given 2.5 mg/kg/d of B2, data points (A) represent animals treated with 7.5 mg/kg/d of B2, and (*) represent animals receiving doxorubicin.

**Figure 9** is a photograph of animals in the control group.
**Figure 10** is a photograph of animals receiving 2.5 mg/kg/d of B2.
**Figure 11** is a photograph of animals receiving 7.5 mg/kg/d of B2.
**Figure 12** is a photograph of tumors excised from animals from the groups depicted in **Figures 9, 10,** and **11.** The top row are tumors from animals in the control group, the middle row are tumors from animals receiving 2.5 mg/kg/d of B2, and the bottom row are tumors from animals receiving 7.5 mg/kg/d of B2.

*: The compounds, compositions, medicaments, and the compounds and compositions for medical use are defined in the claims.

## DETAILED DESCRIPTION

*The materials, compounds, compositions, articles, and methods described herein may be understood more readily by reference to the following detailed description of specific aspects of the disclosed subject matter and the Examples included therein.

Before the present materials, compounds, compositions, articles, devices, and methods are disclosed and described, it is to be understood that the aspects described below are not limited to specific synthetic methods or specific reagents, as such may, of course, vary. It is also to be understood that the terminology used herein is for the purpose of describing particular aspects only and is not intended to be limiting.

### General Definitions

In this specification and in the claims that follow, reference will be made to a number of terms, which shall be defined to have the following meanings:
All percentages, ratios and proportions herein are by weight, unless otherwise specified. All temperatures are in degrees Celsius (°C) unless otherwise specified.

By "pharmaceutically acceptable" is meant a material that is not biologically or otherwise undesirable, i.e., the material can be administered to an individual along with the relevant active compound without causing clinically unacceptable biological effects or interacting in a deleterious manner with any of the other components of the pharmaceutical composition in which it is contained.

Ranges may be expressed herein as from "about" one particular value, and/or to "about" another particular value. When such a range is expressed, another aspect includes from the one particular value and/or to the other particular value. Similarly, when values are expressed as approximations, by use of the antecedent "about," it will be understood that the particular value forms another aspect. It will be further understood that the endpoints of each of the ranges are significant both in relation to the other endpoint, and independently of the other endpoint.

A weight percent of a component, unless specifically stated to the contrary, is based on the total weight of the formulation or composition in which the component is included.

By "effective amount" as used herein means "an amount of one or more of the disclosed antitumor agents, effective at dosages and for periods of time necessary to achieve the desired or therapeutic result." An effective amount may vary according to factors known in the art, such as the disease state, age, sex, and weight of the human or animal being treated. Although particular dosage regimes may be described in examples herein, a person skilled in the art would appreciated that the dosage regime may be altered to provide optimum therapeutic response. For example, several divided doses may be administered daily or the dose may be proportionally reduced as indicated by the exigencies of the therapeutic situation. In addition, the compositions of this disclosure can be administered as frequently as necessary to achieve a therapeutic amount.

"Admixture" or "blend" is generally used herein means a physical combination of two or more different components

"Excipient" is used herein to include any other compound that may be contained in or combined with one or more of the disclosed inhibitors that is not a therapeutically or biologically active compound. As such, an excipient should be pharmaceutically or biologically acceptable or relevant (for example, an excipient should generally be non-toxic to the subject). "Excipient" includes a single such compound and is also intended to include a plurality of excipients.

As used herein, by a "subject" is meant an individual. Thus, the "subject" can include domesticated animals (*e.g.,* cats, dogs, etc.), livestock (*e.g.,* cattle, horses, pigs, sheep, goats, etc.), laboratory animals (*e.g.*, mouse, rabbit, rat, guinea pig, etc.), and birds. "Subject" can also include a mammal, such as a primate or a human.

By "reduce" or other forms of the word, such as "reducing" or "reduction," is meant lowering of an event or characteristic (*e.g.*, tumor size or tumor progression). It is understood that this is typically in relation to some standard or expected value, in other words it is relative, but that it is not always necessary for the standard or relative value to be referred to.

By "prevent" or other forms of the word, such as "preventing" or "prevention," is meant to stop a particular event or characteristic, to stabilize or delay the development or progression of a particular event or characteristic, or to minimize the chances that a particular event or characteristic will occur. Prevent does not require comparison to a control as it is typically more absolute than, for example, reduce. As used herein, something could be reduced but not prevented, but something that is reduced could also be prevented. Likewise, something could be prevented but not reduced, but something that is prevented could also be reduced. It is understood that where reduce or prevent are used, unless specifically indicated otherwise, the use of the other word is also expressly disclosed.

By "treat" or other forms of the word, such as "treated" or "treatment," is meant to administer a composition or to perform a method in order to reduce, prevent, inhibit, breakdown, or eliminate a particular characteristic or event (*e.g.*, tumor size or tumor progression). The disclosed compounds affect tumor growth by inhibiting CDK, for example, the transition from G1/S to G2/M.

By "chemotherapeutic agent" is meant any drug, pharmaceutical or otherwise, that can be given to a subject as part of a combination therapy. Non-limiting examples of chemotherapeutic agents include anticancer drugs, for example, IL-2, taxol, and the like, antimicrobials, anti-virals, anti-fungicides, and the like.

Throughout the description and claims of this specification the word "comprise" and other forms of the word, such as "comprising" and "comprises," means including but not limited to, and is not intended to exclude, for example, other additives, components, integers, or steps.

As used in the description and the appended claims, the singular forms "a," "an," and "the" include plural referents unless the context clearly dictates otherwise. Thus, for example, reference to "a composition" includes mixtures of two or more such compositions, reference to "a phenylsulfamic acid" includes mixtures of two or more such phenylsulfamic acids, reference to "the compound" includes mixtures of two or more such compounds, and the like.

"Optional" or "optionally" means that the subsequently described event or circumstance can or cannot occur, and that the description includes instances where the event or circumstance occurs and instances where it does not.

Ranges can be expressed herein as from "about" one particular value, and/or to "about" another particular value. When such a range is expressed, another aspect includes from the one particular value and/or to the other particular value. Similarly, when values are expressed as approximations, by use of the antecedent "about," it will be understood that the particular value forms another aspect. It will be further understood that the endpoints of each of the ranges are significant both in relation to the other endpoint, and independently of the other endpoint. It is also understood that there are a number of values disclosed herein, and that each value is also herein disclosed as "about" that particular value in addition to the value itself. For example, if the value "10" is disclosed, then "about 10" is also disclosed. It is also understood that when a value is disclosed, then "less than or equal to" the value, "greater than or equal to the value," and possible ranges between values are also disclosed, as appropriately understood by the skilled artisan. For example, if the value "10" is disclosed, then "less than or equal to 10" as well as "greater than or equal to 10" is also disclosed. It is also understood that throughout the application data are provided in a number of different formats and that this data represent endpoints and starting points and ranges for any combination of the data points. For example, if a particular data point "10" and a particular data point "15" are disclosed, it is understood that greater than, greater than or equal to, less than, less than or equal to, and equal to 10 and 15 are considered disclosed as well as between 10 and 15. It is also understood that each unit between two particular units are also disclosed. For example, if 10 and 15 are disclosed, then 11, 12, 13, and 14 are also disclosed.

The following chemical hierarchy is used throughout the specification to describe and enable the scope of the present disclosure and to particularly point out and distinctly claim the units which comprise the compounds of the present disclosure, however, unless otherwise specifically defined, the terms used herein are the same as those of the artisan of ordinary skill. The term "hydrocarbyl" stands for any carbon atom-based unit (organic molecule), said units optionally containing one or more organic functional group, including inorganic atom comprising salts, *inter alia,* carboxylate salts, quaternary ammonium salts. Within the broad meaning of the term "hydrocarbyl" are the classes "acyclic hydrocarbyl" and "cyclic hydrocarbyl" which terms are used to divide hydrocarbyl units into cyclic and non-cyclic classes.

As it relates to the following definitions, "cyclic hydrocarbyl" units can comprise only carbon atoms in the ring (i.e., carbocyclic and aryl rings) or can comprise one or more heteroatoms in the ring (i.e., heterocyclic and heteroaryl rings). For "carbocyclic" rings the lowest number of carbon atoms in a ring are 3 carbon atoms; cyclopropyl. For "aryl" rings the lowest number of carbon atoms in a ring are 6 carbon atoms; phenyl. For "heterocyclic" rings the lowest number of carbon atoms in a ring is 1 carbon atom; diazirinyl. Ethylene oxide comprises 2 carbon atoms and is a $C_2$ heterocycle. For "heteroaryl" rings the lowest number of carbon atoms in a ring is 1 carbon atom; 1,2,3,4-tetrazolyl. The following is a non-limiting description of the terms "acyclic hydrocarbyl" and "cyclic hydrocarbyl" as used herein.

A. Substituted and unsubstituted acyclic hydrocarbyl:

For the purposes of the present disclosure the term "substituted and unsubstituted acyclic hydrocarbyl" encompasses 3 categories of units:

1) linear or branched alkyl, non-limiting examples of which include, methyl ($C_1$), ethyl ($C_2$), n-propyl ($C_3$), *iso*-propyl ($C_3$), n-butyl ($C_4$), *sec*-butyl ($C_4$), *iso*-butyl ($C_4$), *tert*-butyl ($C_4$), and the like; substituted linear or branched alkyl, non-limiting examples of which includes, hydroxymethyl ($C_1$), chloromethyl ($C_1$), trifluoromethyl ($C_1$), aminomethyl ($C_1$), 1-chloroethyl ($C_2$), 2-hydroxyethyl ($C_2$), 1,2-difluoroethyl ($C_2$), 3-carboxypropyl ($C_3$), and the like.
2) linear or branched alkenyl, non-limiting examples of which include, ethenyl ($C_2$), 3-propenyl ($C_3$), 1-propenyl *(also* 2-methylethenyl) ($C_3$), isopropenyl (*also* 2-methylethen-2-yl) ($C_3$), buten-4-yl ($C_4$), and the like; substituted linear or branched alkenyl, non-limiting examples of which include, 2-chloroethenyl (*also* 2-chlorovinyl) ($C_2$), 4-hydroxybuten-1-yl ($C_4$), 7-hydroxy-7-methyloct-4-en-2-yl ($C_9$), 7-hydroxy-7-methyloct-3,5-dien-2-yl ($C_9$), and the like.
3) linear or branched alkynyl, non-limiting examples of which include, ethynyl ($C_2$), prop-2-ynyl *(also* propargyl) ($C_3$), propyn-1-yl ($C_3$), and 2-methyl-hex-4-yn-1-yl ($C_7$); substituted linear or branched alkynyl, non-limiting examples of which include, 5-hydroxy-5-methylhex-3-ynyl ($C_7$), 6-hydroxy-6-methylhept-3-yn-2-yl ($C_8$), 5-hydroxy-5-ethylhept-3-ynyl ($C_9$), and the like.

B. Substituted and unsubstituted cyclic hydrocarbyl:

For the purposes of the present disclosure the term "substituted and unsubstituted cyclic hydrocarbyl" encompasses 5 categories of units:

1) The term "carbocyclic" is defined herein as "encompassing rings comprising from 3 to 20 carbon atoms, wherein the atoms which comprise said rings are limited to carbon atoms, and further each ring can be independently substituted with one or more moieties capable of replacing one or more hydrogen atoms." The following are non-limiting examples of "substituted and unsubstituted carbocyclic rings" which encompass the following categories of units:

i) carbocyclic rings having a single substituted or unsubstituted hydrocarbon ring, non-limiting examples of which include, cyclopropyl ($C_3$), 2-methylcyclopropyl ($C_3$), cyclopropenyl ($C_3$), cyclobutyl ($C_4$), 2,3-dihydroxycyclobutyl ($C_4$), cyclobutenyl ($C_4$), cyclopentyl ($C_5$), cyclopentenyl ($C_5$), cyclopentadienyl ($C_5$), cyclohexyl ($C_6$), cyclohexenyl ($C_6$), cycloheptyl ($C_7$), cyclooctanyl ($C_8$), 2,5-dimethylcyclopentyl ($C_5$), 3,5-dichlorocyclohexyl ($C_6$), 4-hydroxycyclohexyl ($C_6$), and 3,3,5-trimethylcyclohex-1-yl ($C_6$).
ii) carbocyclic rings having two or more substituted or unsubstituted fused hydrocarbon rings, non-limiting examples of which include, octahydropentalenyl ($C_8$), octahydro-1*H*-indenyl ($C_9$), 3a,4,5,6,7,7a-hexahydro-3*H*-inden-4-yl ($C_9$), decahydroazulenyl ($C_{10}$).
iii) carbocyclic rings which are substituted or unsubstituted bicyclic hydrocarbon rings, non-limiting examples of which include, bicyclo-[2.1.1]hexanyl, bicyclo[2.2.1]heptanyl,bicyclo[3.1.1]heptanyl, 1,3-dimethyl[2.2.1]heptan-2-yl, bicyclo[2.2.2]octanyl, and bicyclo[3.3.3]undecanyl.

2) The term "aryl" is defined herein as "units encompassing at least one phenyl or naphthyl ring and wherein there are no heteroaryl or heterocyclic rings fused to the phenyl or naphthyl ring and further each ring can be independently substituted with one or more moieties capable of replacing one or more hydrogen atoms." The following are non-limiting examples of "substituted and unsubstituted aryl rings" which encompass the following categories of units:

i) $C_6$ or $C_{10}$ substituted or unsubstituted aryl rings; phenyl and naphthyl rings whether substituted or unsubstituted, non-limiting examples of which include, phenyl ($C_6$), naphthylen-1-yl ($C_{10}$), naphthylen-2-yl ($C_{10}$), 4-fluorophenyl ($C_6$), 2-hydroxyphenyl ($C_6$), 3-methylphenyl ($C_6$), 2-amino-4-fluorophenyl ($C_6$), 2-(*N,N*-diethylamino)phenyl ($C_6$), 2-cyanophenyl ($C_6$), 2,6-di-*tert*-butylphenyl ($C_6$), 3-methoxyphenyl ($C_6$), 8-hydroxynaphthylen-2-yl ($C_{10}$), 4,5-dimethoxynaphthylen-1-yl ($C_{10}$), and 6-cyano-naphthylen-1-yl ($C_{10}$).
ii) $C_6$ or $C_{10}$ aryl rings fused with 1 or 2 saturated rings to afford $C_8$-$C_{20}$ ring systems, non-limiting examples of which include, bicyclo[4.2.0]octa-1,3,5-trienyl ($C_8$), and indanyl ($C_9$).

3) The terms "heterocyclic" and/or "heterocycle" are defined herein as "units comprising one or more rings having from 3 to 20 atoms wherein at least one atom in at least one ring is a heteroatom chosen from nitrogen (N), oxygen (O), or sulfur (S), or mixtures of N, O, and S, and wherein further the ring which contains the heteroatom is also not an aromatic ring." The following are non-limiting examples of "substituted and unsubstituted heterocyclic rings" which encompass the following categories of units:

i) heterocyclic units having a single ring containing one or more heteroatoms, non-limiting examples of which include, diazirinyl ($C_1$), aziridinyl ($C_2$), urazolyl ($C_2$), azetidinyl ($C_3$), pyrazolidinyl ($C_3$), imidazolidinyl ($C_3$), oxazolidinyl ($C_3$), isoxazolinyl ($C_3$), thiazolidinyl ($C_3$), isothiazolinyl ($C_3$), oxathiazolidinonyl ($C_3$), oxazolidinonyl ($C_3$),

hydantoinyl ($C_3$), tetrahydrofuranyl ($C_4$), pyrrolidinyl ($C_4$), morpholinyl ($C_4$), piperazinyl ($C_4$), piperidinyl ($C_4$), dihydropyranyl ($C_5$), tetrahydropyranyl ($C_5$), piperidin-2-onyl (valerolactam) ($C_5$), 2,3,4,5-tetrahydro-1*H*-azepinyl ($C_6$), 2,3-dihydro-1*H*-indole ($C_8$), and 1,2,3,4-tetrahydroquinoline ($C_9$).

ii) heterocyclic units having 2 or more rings one of which is a heterocyclic ring, non-limiting examples of which include hexahydro-1*H*-pyrrolizinyl ($C_7$), 3a,4,5,6,7,7a-hexahydro-1*H*-benzo[d]imidazolyl ($C_7$), 3a,4,5,6,7,7a-hexahydro-1*H*-indolyl ($C_8$), 1,2,3,4-tetrahydroquinolinyl ($C_9$), and decahydro-1*H*-cycloocta[b]pyrrolyl ($C_{10}$).

4) The term "heteroaryl" is defined herein as "encompassing one or more rings comprising from 5 to 20 atoms wherein at least one atom in at least one ring is a heteroatom chosen from nitrogen (N), oxygen (O), or sulfur (S), or mixtures of N, O, and S, and wherein further at least one of the rings which comprises a heteroatom is an aromatic ring." The following are non-limiting examples of "substituted and unsubstituted heterocyclic rings" which encompass the following categories of units:

i) heteroaryl rings containing a single ring, non-limiting examples of which include, 1,2,3,4-tetrazolyl ($C_1$), [1,2,3]triazolyl ($C_2$), [1,2,4]triazolyl ($C_2$), triazinyl ($C_3$), thiazolyl ($C_3$), 1*H*-imidazolyl ($C_3$), oxazolyl ($C_3$), isoxazolyl ($C_3$), isothiazolyl ($C_3$), furanyl ($C_4$), thiophenyl ($C_4$), pyrimidinyl ($C_4$), 2-phenylpyrimidinyl ($C_4$), pyridinyl ($C_5$), 3-methylpyridinyl ($C_5$), and 4-dimethylaminopyridinyl ($C_5$)

ii) heteroaryl rings containing 2 or more fused rings one of which is a heteroaryl ring, non-limiting examples of which include: 7*H*-purinyl ($C_5$), 9*H*-purinyl ($C_5$), 6-amino-9*H*-purinyl ($C_5$), 5*H*-pyrrolo[3,2-*d*]pyrimidinyl ($C_6$), 7*H*-pyrrolo[2,3-*d*]pyrimidinyl ($C_6$), pyrido[2,3-*d*]pyrimidinyl ($C_7$), 2-phenylbenzo[d]thiazolyl ($C_7$), 1*H*-indolyl ($C_8$), 4,5,6,7-tetrahydro-1-*H*-indolyl ($C_8$), quinoxalinyl ($C_8$), 5-methylquinoxalinyl ($C_8$), quinazolinyl ($C_8$), quinolinyl ($C_9$), 8-hydroxy-quinolinyl ($C_9$), and isoquinolinyl ($C_9$).

5) $C_1$-$C_6$ tethered cyclic hydrocarbyl units (whether carbocyclic units, $C_6$ or $C_{10}$ aryl units, heterocyclic units, or heteroaryl units) which connected to another moiety, unit, or core of the molecule by way of a $C_1$-$C_6$ alkylene unit. Non-limiting examples of tethered cyclic hydrocarbyl units include benzyl $C_1$-($C_6$) having the formula:

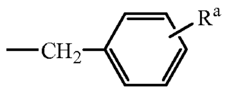

wherein $R^a$ is optionally one or more independently chosen substitutions for hydrogen. Further examples include other aryl units, *inter alia,* (2-hydroxyphenyl)hexyl $C_6$-($C_6$); naphthalen-2-ylmethyl $C_1$-($C_{10}$), 4-fluorobenzyl $C_1$-($C_6$), 2-(3-hydroxyphenyl)ethyl $C_2$-($C_6$), as well as substituted and unsubstituted $C_3$-$C_{10}$ alkylenecarbocyclic units, for example, cyclopropylmethyl $C_1$-($C_3$), cyclopentylethyl $C_2$-($C_5$), cyclohexylmethyl $C_1$-($C_6$);. Included within this category are substituted and unsubstituted $C_1$-$C_{10}$ alkylene-heteroaryl units, for example a 2-picolyl $C_1$-($C_6$) unit having the formula:

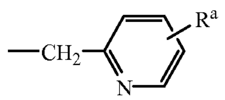

wherein $R^a$ is the same as defined above. In addition, $C_1$-$C_{12}$ tethered cyclic hydrocarbyl units include $C_1$-$C_{10}$ alkyleneheterocyclic units and alkylene-heteroaryl units, non-limiting examples of which include, aziridinylmethyl $C_1$-($C_2$) and oxazol-2-ylmethyl $C_1$-($C_3$).

For the purposes of the present disclosure carbocyclic rings are from $C_3$ to $C_{20}$; aryl rings are $C_6$ or $C_{10}$; heterocyclic rings are from $C_1$ to $C_9$; and heteroaryl rings are from $C_1$ to $C_9$.

For the purposes of the present disclosure, and to provide consistency in defining the present disclosure, fused ring units, as well as spirocyclic rings, bicyclic rings and the like, which comprise a single heteroatom will be characterized and referred to herein as being encompassed by the cyclic family corresponding to the heteroatom containing ring, although the artisan may have alternative characterizations. For example, 1,2,3,4-tetrahydroquinoline having the formula:

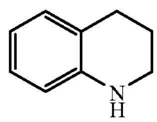

is, for the purposes of the present disclosure, considered a heterocyclic unit. 6,7-Dihydro-5*H*-cyclopentapyrimidine having the formula:

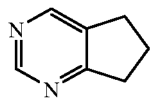

is, for the purposes of the present disclosure, considered a heteroaryl unit. When a fused ring unit contains heteroatoms in both a saturated ring (heterocyclic ring) and an aryl ring (heteroaryl ring), the aryl ring will predominate and determine the type of category to which the ring is assigned herein for the purposes of describing the invention. For example, 1,2,3,4-tetrahydro-[1,8]naphthpyridine having the formula:

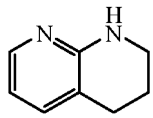

is, for the purposes of the present disclosure, considered a heteroaryl unit.

The term "substituted" is used throughout the specification. The term "substituted" is applied to the units described herein as "substituted unit or moiety is a hydrocarbyl unit or moiety, whether acyclic or cyclic, which has one or more hydrogen atoms replaced by a substituent or several substituents as defined herein below." The units, when substituting for hydrogen atoms are capable of replacing one hydrogen atom, two hydrogen atoms, or three hydrogen atoms of a hydrocarbyl moiety at a time. In addition, these substituents can replace two hydrogen atoms on two adjacent carbons to form said substituent, new moiety, or unit. For example, a substituted unit that requires a single hydrogen atom replacement includes halogen, hydroxyl, and the like. A two hydrogen atom replacement includes carbonyl, oximino, and the like. A two hydrogen atom replacement from adjacent carbon atoms includes epoxy, and the like. Three hydrogen replacement includes cyano, and the like. The term substituted is used throughout the present specification to indicate that a hydrocarbyl moiety, *inter alia,* aromatic ring, alkyl chain; can have one or more of the hydrogen atoms replaced by a substituent. When a moiety is described as "substituted" any number of the hydrogen atoms may be replaced. For example, 4-hydroxyphenyl is a "substituted aromatic carbocyclic ring (aryl ring)", (N,N-dimethyl-5-amino)octanyl is a " substituted $C_8$ linear alkyl unit, 3-guanidinopropyl is a "substituted $C_3$ linear alkyl unit," and 2-carboxypyridinyl is a "substituted heteroaryl unit."

The following are non-limiting examples of units which can substitute for hydrogen atoms on a carbocyclic, aryl, heterocyclic, or heteroaryl unit:

i) $C_1$-$C_{12}$ linear, branched, or cyclic alkyl, alkenyl, and alkynyl; methyl ($C_1$), ethyl ($C_2$), ethenyl ($C_2$), ethynyl ($C_2$), n-propyl ($C_3$), *iso*-propyl ($C_3$), cyclopropyl ($C_3$), 3-propenyl ($C_3$), 1-propenyl *(also* 2-methylethenyl) ($C_3$), isopropenyl *(also* 2-methylethen-2-yl) ($C_3$), prop-2-ynyl *(also* propargyl) ($C_3$), propyn-1-yl ($C_3$), n-butyl ($C_4$), *sec*-butyl ($C_4$), *iso*-butyl ($C_4$), *tert*-butyl ($C_4$), cyclobutyl ($C_4$), buten-4-yl ($C_4$), cyclopentyl ($C_5$), cyclohexyl ($C_6$);
ii) substituted or unsubstituted $C_6$ or $C_{10}$ aryl; for example, phenyl, naphthyl (also referred to herein as naphthylen-1-yl ($C_{10}$) or naphthylen-2-yl ($C_{10}$));
iii) substituted or unsubstituted $C_6$ or $C_{10}$ alkylenearyl; for example, benzyl, 2-phenylethyl, naphthylen-2-ylmethyl;
iv) substituted or unsubstituted $C_1$-$C_9$ heterocyclic rings; as described herein below;
v) substituted or unsubstituted $C_1$-$C_9$ heteroaryl rings; as described herein below;
vi) $-(CR^{102a}R^{102b})_aOR^{101}$; for example, -OH, $-CH_2OH$, $-OCH_3$, $-CH_2OCH_3$, $-OCH_2CH_3$, $-CH_2OCH_2CH_3$, $-OCH_2CH_2CH_3$, and $CH_2OCH_2CH_2CH_3$;
vii) $-(CR^{102a}R^{102b})_aC(O)R^{101}$; for example, $-COCH_3$, $-CH_2COCH_3$,$-COCH_2CH_3$, $-CH_2COCH_2CH_3$, $-COCH_2CH_2CH_3$, and$-CH_2COCH_2CH_2CH_3$;
viii) $-(CR^{102a}R^{102b})_aC(O)OR^{101}$; for example, $-CO_2CH_3$, $-CH_2CO_2CH_3$, $-CO_2CH_2CH_3$, $-CH_2CO_2CH_2CH_3$, $-CO_2CH_2CH_2CH_3$, and $-CH_2CO_2CH_2CH_2CH_3$;
ix) $-(CR^{102a}R^{102b})_aC(O)N(R^{101})_2$; for example, $-CONH_2$, $-CH_2CONH_2$, $-CONHCH_3$, $-CH_2CONHCH_3$, $-CON(CH_3)_2$, and $-CH_2CON(CH_3)_2$;
x) $-(CR^{102a}R^{102b})_aN(R^{101})_2$; for example, $-NH_2$, $-CH_2NH_2$, $-NHCH_3$,$-CH_2NHCH_3$, $-N(CH_3)_2$, and $-CH_2N(CH_3)_2$;
xi) halogen; -F, -Cl, -Br, and -I;
xii) $-(CR^{102a}R^{102b})_aCN$;
xiii) $-(CR^{102a}R^{102b})_aNO_2$;
xiv) $-CH_jX_k$; wherein X is halogen, the index j is an integer from 0 to 2, j + k = 3; for example, $-CH_2F$, $-CHF_2$, $-CF_3$,

$-CCl_3$, or $-CBr_3$;
xv) $-(CR^{102a}R^{102b})_aSR^{101}$; -SH, $-CH_2SH$, $-SCH_3$, $-CH_2SCH_3$, $-SC_6H_5$, and $-CH_2SC_6H_5$;
xvi) $-(CR^{102a}R^{102b})_aSO_2R^{101}$; for example, $-SO_2H$, $-CH_2SO_2H$, $-SO_2CH_3$, $-CH_2SO_2CH_3$, $-SO_2C_6H_5$, and $-CH_2SO_2C_6H_5$; and
xvii) $-(CR^{102a}R^{102b})_aSO_3R^{101}$; for example, $-SO_3H$, $-CH_2SO_3H$, $-SO_3CH_3$, $-CH_2SO_3CH_3$, $-SO_3C_6H_5$, and $-CH_2SO_3C_6H_5$;

wherein each $R^{101}$ is independently hydrogen, substituted or unsubstituted $C_1$-$C_6$ linear, branched, or cyclic alkyl, phenyl, benzyl, heterocyclic, or heteroaryl; or two $R^{101}$ units can be taken together to form a ring comprising 3-7 atoms; $R^{102a}$ and $R^{102b}$ are each independently hydrogen or $C_1$-$C_4$ linear or branched alkyl; the index "a" is from 0 to 4.

For the purposes of the present disclosure the terms "compound," "analog," and "composition of matter" stand equally well for each other and are used interchangeably throughout the specification. The disclosed compounds include all enantiomeric forms, diastereomeric forms, salts, and the like.

The compounds disclosed herein include all salt forms, for example, salts of both basic groups, *inter alia,* amines, as well as salts of acidic groups, *inter alia,* carboxylic acids. The following are non-limiting examples of anions that can form salts with protonated basic groups: chloride, bromide, iodide, sulfate, bisulfate, carbonate, bicarbonate, phosphate, formate, acetate, propionate, butyrate, pyruvate, lactate, oxalate, malonate, maleate, succinate, tartrate, fumarate, citrate, and the like. The following are non-limiting examples of cations that can form salts of acidic groups: ammonium, sodium, lithium, potassium, calcium, magnesium, bismuth, lysine, and the like.

Disclosed herein are antitumor agents as defined in the claims having the formula:

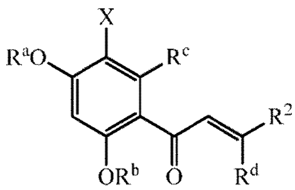

wherein $R^a$, $R^b$, $R^c$, $R^d$, $R^2$ and X are further described herein.

The disclosed agents can be in a non-conformationally restricted form (open form), for example, (*E*)-1-(2-hydroxy-4,6-hydroxy/alkoxy-3-X-substituted-phenyl)-3-$R^2$-substituted-prop-2-en-1-ones (chalcone form) having the formula:

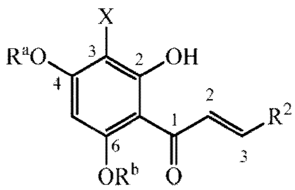

wherein the conventional numbering system, which is used herein, is indicated.

The following is a non-limiting description of the compounds encompassed within the present disclosure of anti-tumor agents.

$R^2$ is chosen from:

i) hydrogen;
ii) substituted or unsubstituted phenyl; or
iii) substituted or unsubstituted $C_1$-$C_5$ heteroaryl.

In one aspect of $R^2$ units, $R^2$ is hydrogen thereby providing antitumor agents having the formula:

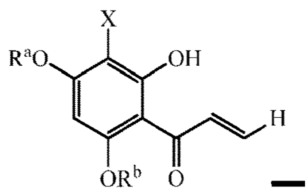

In another aspect of $R^2$ units, $R^2$ is substituted or unsubstituted phenyl. In one embodiment, $R^2$ is unsubstituted phenyl thereby providing antitumor agents having the formula:

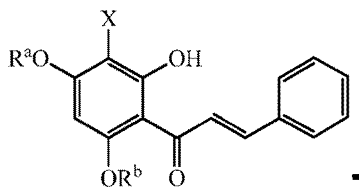

As further disclosed herein, $R^3$ units are absent from the antitumor agents encompassing this embodiment, and the index n, as described herein below, is equal to 0.

In a further aspect of $R^2$ units, $R^2$ is substituted phenyl thereby providing antitumor agents having the formula:

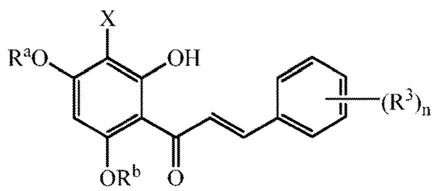

wherein $R^3$ represents from 1 to 5 independently chosen substitutes for hydrogen, the index n is an integer from 1 to 5. $R^3$ units that when taken together with the phenyl unit to which they are bonded, form the $R^2$ units of this aspect, are each independently chosen from:

$C_1$-$C_4$ substituted or unsubstituted linear or branched alkyl;
halogen; $OR^{10}$;
$R^{10}$ is chosen from:

a) hydrogen; or
b) $C_1$-$C_4$ substituted or unsubstituted linear or branched alkyl;

$N(R^{11a})(R^{11b})$;
$R^{11a}$ and $R^{11b}$ are each independently chosen from:

a) -H;
b) $C_1$-$C_4$ substituted or unsubstituted linear or branched alkyl; CN; $NO_2$.

In the $R^2$ units, $R^3$ represents substitutions independently chosen from:

i) $C_1$-$C_4$ substituted or unsubstituted linear or branched alkyl;
ii) halogen;
iii) $-OR^{10}$; wherein $R^{10}$ is hydrogen or methyl;
iv) $-N(R^{11a})(R^{11b})$; wherein $R^{11a}$ and $R^{11b}$ are each independently chosen from hydrogen or methyl;
v) $-C(O)R^{12}$; wherein $R^{12}$ is hydrogen or methyl
vi) -CN;
vii) $-NO_2$;
viii) $C_1$-$C_4$ linear or branched alkyl substituted by from 1 to 9 halogen atoms chosen from F, Cl, Br, or I; or

ix) $-SO_2NH_2$.

In one embodiment of $R^2$ units $R^3$ is $C_1$-$C_4$ substituted or unsubstituted linear or branched alkyl. In one iteration of this embodiment, $R^3$ is methyl ($C_1$). Non-limiting examples of $R^2$ units according to this iteration include 2-methylphenyl, 3-methylphenyl, and 4-methylphenyl. Further non-limiting examples include 2,3-dimethylphenyl, 2,4-dimethylphenyl, 2,5-dimethylphenyl, 2,6-dimethylphenyl, 3,4-dimethylphenyl, and 3,5-dimethylphenyl. Still further non-limiting examples include 2,3,4-trimethylphenyl, 2,3,5-trimethylphenyl, 2,3,6-trimethylphenyl, and 3,4,5-trimethylphenyl. Yet further non-limiting examples include 2,3,4,5-tetramethylphenyl, 2,3,4,6-tetramethylphenyl, and 2,3,4,5,6-pentamethylphenyl.

In another iteration of this embodiment, each $R^3$ is independently chosen from ethyl ($C_2$), n-propyl ($C_3$), *iso*-propyl ($C_3$), n-butyl ($C_4$), *sec*-butyl ($C_4$), *iso*-butyl ($C_4$), and *tert*-butyl ($C_4$). Non-limiting examples of $R^2$ units according to this iteration include 2-ethylphenyl, 3-ethylphenyl, 4-ethylphenyl, 2-n-propylphenyl, 3-n-propylphenyl, 4-n-propylphenyl, 2-*iso*-propylphenyl, 3-*iso*-propylphenyl, and 4-*iso*-propylphenyl. Further non-limiting examples include 2,3-diethylphenyl, 2,4-diethylphenyl, 2,5-diethylphenyl, 2,6-diethylphenyl, 3,4-diethylphenyl, and 3,5-diethylphenyl. Still further non-limiting examples include 2,3,4-triethylphenyl, 2,3,5-triethylphenyl, 2,3,6-triethylphenyl, and 3,4,5-triethylphenyl. Yet further non-limiting examples include 2,3,4,5-tetraethylphenyl, 2,3,4,6-tetraethylphenyl, and 2,3,4,5,6-pentaethylphenyl.

In a further embodiment, combinations of methyl ($C_1$) units and $C_2$-$C_4$ units can provide $R^2$ units. Non-limiting examples include 2-methyl-3-ethylphenyl, 2,6-dimethyl-3-ethylphenyl, and the like.

In a still further embodiment of this aspect, $R^3$ is $-OR^{10}$ (the index x is equal to 0) wherein $R^{10}$ is hydrogen or methyl ($C_1$). Non-limiting examples of $R^2$ units according to this iteration include 2-hydroxyphenyl, 3-hydroxyphenyl, 4-hydroxyphenyl, 2-methoxyphenyl, 3-methoxyphenyl, and 4-methoxyphenyl. Further non-limiting examples include 2,3-dihydroxyphenyl, 2,4-dihydroxyphenyl, 2,5-dihydroxyphenyl, 2,6-dihydroxyphenyl, 3,4-dihydroxyphenyl, and 3,5-dihydroxyphenyl. Still further non-limiting examples include 2,3-dimethoxyphenyl, 2,4-dimethoxyphenyl, 2,5-dimethoxyphenyl, 2,6-dimethoxyphenyl, 3,4-dimethoxyphenyl, and 3,5-dimethoxyphenyl. Yet further non-limiting examples include 2,3,4-trimethoxyphenyl, 2,3,5-trimethoxyphenyl, 2,3,6-trimethoxyphenyl, and 3,4,5-trimethoxyphenyl. Yet still further non-limiting examples include 2,3,4,5-tetramethoxyphenyl, 2,3,4,6-tetramethoxyphenyl, and 2,3,4,5,6-pentamethoxyphenyl.

In a still another further embodiment of this aspect, $R^3$ is halogen. In one iteration of this embodiment, $R^3$ is chloro. Non-limiting examples of $R^2$ units according to this iteration include 2-chlorophenyl, 3-chlorophenyl, and 4-chlorophenyl. Further non-limiting examples include 2,3-dichlorophenyl, 2,4-dichlorophenyl, 2,5-dichlorophenyl, 2,6-dichlorophenyl, 3,4-dichlorophenyl, and 3,5-dichlorophenyl. Still further non-limiting examples include 2,3,4-trichlorophenyl, 2,3,5-trichlorophenyl, 2,3,6-trichlorophenyl, and 3,4,5-trichlorophenyl. Yet further non-limiting examples include 2,3,4,5-tetrachlorophenyl, 2,3,4,6-tetrachlorophenyl, and 2,3,4,5,6-pentachlorophenyl.

In another iteration of this embodiment, $R^3$ is bromo. Non-limiting examples of $R^2$ units according to this iteration include 2-bromophenyl, 3-bromophenyl, and 4-bromophenyl. Further non-limiting examples include 2,3-dibromophenyl, 2,4-dibromophenyl, 2,5-dibromophenyl, 2,6-dibromophenyl, 3,4-dibromophenyl, and 3,5-dibromophenyl. Still further non-limiting examples include 2,3,4-tribromophenyl, 2,3,5-tribromophenyl, 2,3,6-tribromophenyl, and 3,4,5-tribromophenyl. Yet further non-limiting examples include 2,3,4,5-tetrabromophenyl, 2,3,4,6-tetrabromophenyl, and 2,3,4,5,6-pentabromophenyl.

In one iteration of this embodiment, $R^3$ is fluoro. Non-limiting examples of $R^2$ units according to this iteration include 2-chlorophenyl, 3-chlorophenyl, and 4-chlorophenyl. Further non-limiting examples include 2,3-dichlorophenyl, 2,4-dichlorophenyl, 2,5-dichlorophenyl, 2,6-dichlorophenyl, 3,4-dichlorophenyl, and 3,5-dichlorophenyl. Still further non-limiting examples include 2,3,4-trichlorophenyl, 2,3,5-trichlorophenyl, 2,3,6-trichlorophenyl, and 3,4,5-trichlorophenyl. Yet further non-limiting examples include 2,3,4,5-tetrachlorophenyl, 2,3,4,6-tetrachlorophenyl, and 2,3,4,5,6-pentachlorophenyl.

In a yet further aspect of $R^2$ units, $R^2$ is substituted or unsubstituted $C_1$-$C_5$ heteroaryl. In one embodiment of this aspect, $R^2$ is a substituted or unsubstituted $C_1$-$C_4$ heteroaryl chosen from:

i) pyrrol-2-yl and pyrrol-3-yl having the respective formulae:

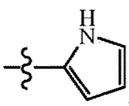
;
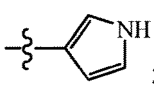
;

ii) imidazol-2-yl and imidazol-4-yl having the respective formulae:

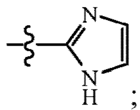; 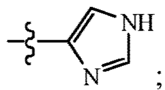;

iii) pyrazol-3-yl and pyrazol-4-yl having the respective formulae:

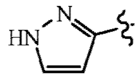; 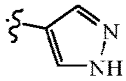;

iv) [1,2,3]triazol-4-yl, [1,2,3]triazol-5-yl, [1,2,4]triazol-4-yl, and [1,2,4]triazol-5-yl having the respective formulae:

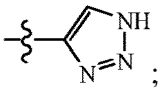; 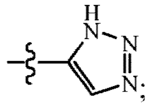; 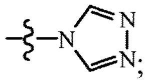; 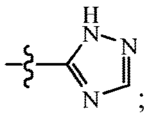;

v) 1,2,3,4-tetrazol-1-yl and 1,2,3,4-tetrazol-5-yl having the respective formulae:

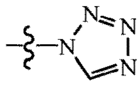; 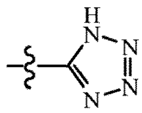;

vi) oxazol-2-yl, oxazol-4-yl, and oxazol-5-yl having the respective formulae:

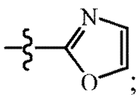; 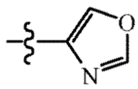; 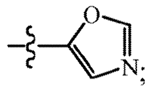;

vii) isoxazol-3-yl, isoxazol-4-yl, and isoxazol-5-yl having the respective formulae:

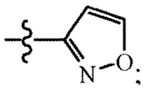; 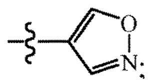; 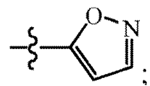;

viii) [1,2,4]oxadiazol-3-yl and [1,2,4]oxadiazol-5-yl having the respective formulae:

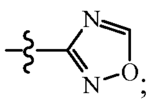; 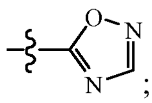;

ix) [1,3,4]oxadiazol-2-yl having the formula:

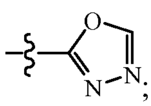;

x) furan-2-yl and furan-3-yl having the respective formulae:

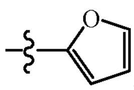; 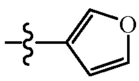;

xi) thiophen-2-yl and thiophen-3-yl having the respective formulae:

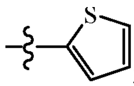 ; 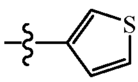 ;

xii) isothiazol-3-yl, isothiazol-4-yl and isothiazol-5-yl having the respective formulae:

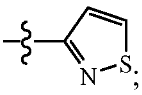 ; 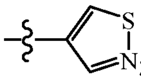 ; 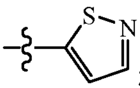 ;

xiii) thiazol-2-yl, thiazol-4-yl and thiazol-5-yl having the respective formulae:

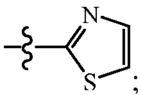 ; 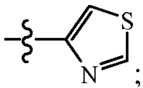 ; 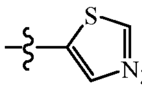 ;

xiv) [1,2,4]thiadiazol-3-yl and [1,2,4]thiadiazol-5-yl having the respective formulae:

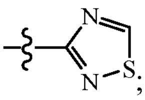 ; 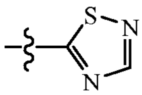 ;

and

xv) [1,3,4]thiadiazol-2-yl having the formula:

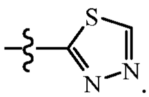 .

In one example of this embodiment $R^2$ is furan-2-yl. In another example, $R^2$ is furan-3-yl. In a further example, $R^2$ is pyrrol-2-yl. In a still further example, $R^2$ is imidazol-2-yl. In a yet still further example, $R^2$ is [1,2,3]triazol-4-yl. In a yet another further example, $R^2$ is oxazol-2-yl. In a yet still another further example, $R^2$ is thiophen-2-yl.

In another embodiment of this aspect, $R^2$ is a substituted or unsubstituted $C_5$ heteroaryl chosen from:

i) pyridin-2-yl, pyridin-3-yl and pyridin-4-yl having the respective formulae:

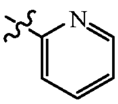 ; 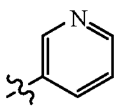 ; 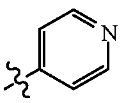 ;

ii) pyrimidin-2-yl, pyrimidin-4-yl and pyrimidin-5-yl having the respective formulae:

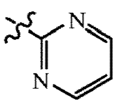 ; 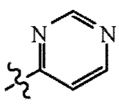 ; 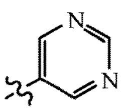 ;

iii) pyrazin-2-yl having the formula:

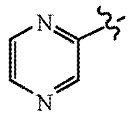

;

and

iv) triazin-2-yl having the formula:

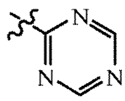

.

$R^a$ and $R^b$ are each independently hydrogen or methyl. In one aspect $R^a$ is hydrogen. An embodiment of this aspect is $R^a$ equal to hydrogen and $R^b$ equal to hydrogen. Another embodiment of this aspect is $R^a$ equal to hydrogen and $R^b$ equal to methyl.

In another aspect $R^a$ is methyl. An embodiment of this aspect is $R^a$ equal to methyl and $R^b$ equal to hydrogen. Another embodiment of this aspect is $R^a$ equal to methyl and $R^b$ equal to methyl.

In a further aspect $R^b$ is hydrogen. An embodiment of this aspect is $R^b$ equal to hydrogen and $R^a$ equal to hydrogen. Another embodiment of this aspect is $R^b$ equal to hydrogen and $R^a$ equal to methyl.

In a still further aspect $R^b$ is methyl. An embodiment of this aspect is $R^b$ equal to methyl and $R^a$ equal to hydrogen. Another embodiment of this aspect is $R^b$ equal to methyl and $R^a$ equal to methyl.

$R^d$ is hydrogen wherein the compounds disclosed herein have the formula:

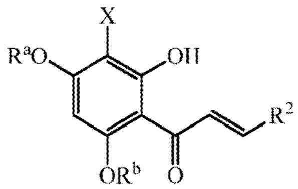

.

$R^c$ is hydroxyl X is having the formula $-[CH_2]_yR^{23}$; wherein $R^{23}$ is an N-substituted heterocyclic ring; and the index y is equal to 1. One embodiment of this aspect relates to $R^{23}$ units chosen from morphonlin-4-yl having the formula:

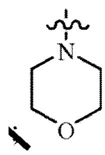

;

or piperidin-1-yl, having the formula:

One embodiment of $R^{23}$ units comprising $C_4$-$C_5$ heterocyclic rings relates to $R^{23}$ units comprising heterocyclic rings having the formula:

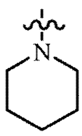

or

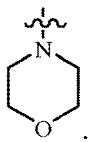

.

Non-limiting examples of antitumor agents according to this embodiment comprising piperidin-1-yl units include:

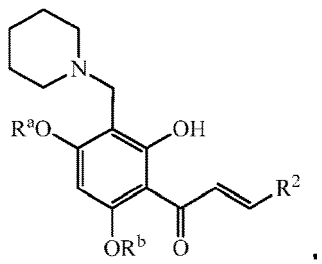

Non-limiting examples of antitumor agents according to this embodiment comprising morpholin-4-yl units include:

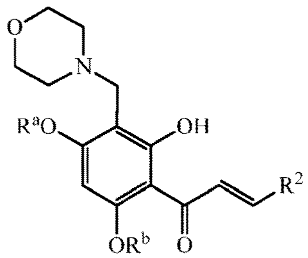

Another aspect of X units relates to X units having the formula $-[CH_2]_yR^{23}$; wherein $R^{23}$ is an N-substituted heterocyclic ring; and the index y is equal to 0. One embodiment of this aspect relates to $R^{23}$ units chosen from a substituted or unsubstituted $C_5$ heterocyclic ring having the formula:

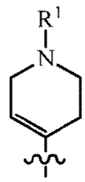

wherein $R^1$ is hydrogen or methyl.

A further iteration of this embodiment relates to $R^{23}$ units wherein $R^1$ is hydrogen thereby providing a 1,2,3,6-tetrahydropyridin-4-yl X unit.

One example of this iteration relates to $R^1$ equal to methyl ($C_1$) thereby providing a 1-methyl-1,2,3,6-tetrahydropyridin-4-yl X unit.

The antitumor agents (compounds) of the present disclosure are arranged into several Categories to assist the formulator in applying a rational synthetic strategy for the preparation of analogs which are not expressly exampled herein. The arrangement into categories does not imply increased or decreased efficacy for any of the compositions of matter described herein.

A first category of antitumor agents disclosed herein have the formula:

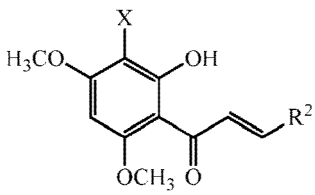

wherein X has the formula $-[CH_2]_yR^{23}$; $R^{23}$ is $C_1$-$C_5$ substituted or unsubstituted heterocyclic ring; and the index y is an integer from 0 to 5.

A first embodiment of the first category of the disclosed antitumor agents has the formula:

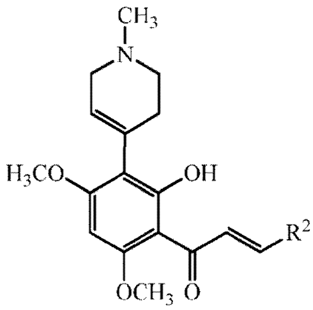

wherein $R^{23}$ is 1-methyl-1,2,3,6-tetrahydropyridin-4-yl and the index y is equal to 0. For this embodiment, $R^2$ units comprise substituted or unsubstituted phenyl, non-limiting examples of which are further disclosed herein below in Table I.

TABLE I

| No. | $R^2$ | No. | $R^2$ |
|---|---|---|---|
| A1 | phenyl | A30 | 2-fluorophenyl |
| A2 | 2-chlorophenyl | A31 | 2-methylphenyl |
| A3 | 2-nitrophenyl | A32 | 2-hydroxyphenyl |
| A4 | 2,3-dimethoxyphenyl | A33 | 2-bromophenyl |
| A5 | 3-methylphenyl | A34 | 2-cyanophenyl |
| A6 | 3-methoxyphenyl | A35 | 2-methoxyphenyl |
| A7 | 3-chlorophenyl | A36 | 2-dimethylaminophenyl |
| A8 | 3-nitrophenyl | A37 | 3-fluorophenyl |
| A9 | 4-methylphenyl | A38 | 3-bromophenyl |
| A10 | 4-methoxyphenyl | A39 | 3-cyanophenyl |
| A11 | 4-hydroxyphenyl | A40 | 3-hydroxyphenyl |
| A12 | 4-chlorophenyl | A41 | 3-dimethylaminophenyl |
| A13 | 4-bromophenyl | A42 | 3,4-dimethoxyphenyl |
| A14 | 4-dimethylaminophenyl | A43 | 4-nitrophenyl |
| A15 | 4-cyanophenyl | A44 | 4-fluorophenyl |
| A16 | 2,3,4-trimethoxyphenyl | A45 | 4-sulfonylamino |
| A17 | 3,4,5-trimethoxyphenyl | A46 | 4-formylamino |

The compounds that comprise the first embodiment of the first category of the disclosed antitumor agents can be prepared by the procedure outlined below in Scheme I and disclosed in Example 1.

Scheme I

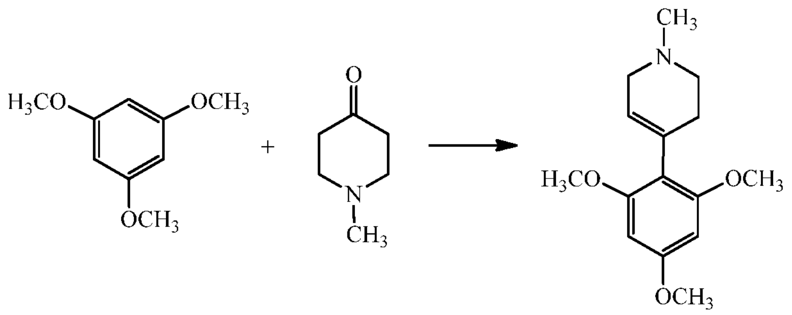

1

Reaction and conditions: (a) HOAc, HCl (g); heat, 3 hr.

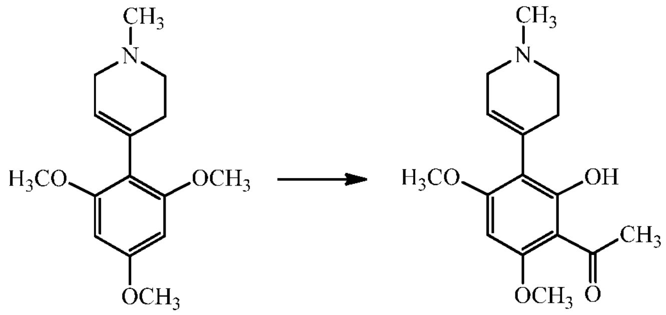

1 2

Reaction and conditions: (b) $Ac_2O$, $BF_3$:$Et_2O$, $CH_2Cl_2$; rt., 24 hr. 1-(2-hydroxy-4,6-dimethoxy-3-(1-methyl-1,2,3,6-tetrahydropyridin-4-yl)phenyl)ethanone

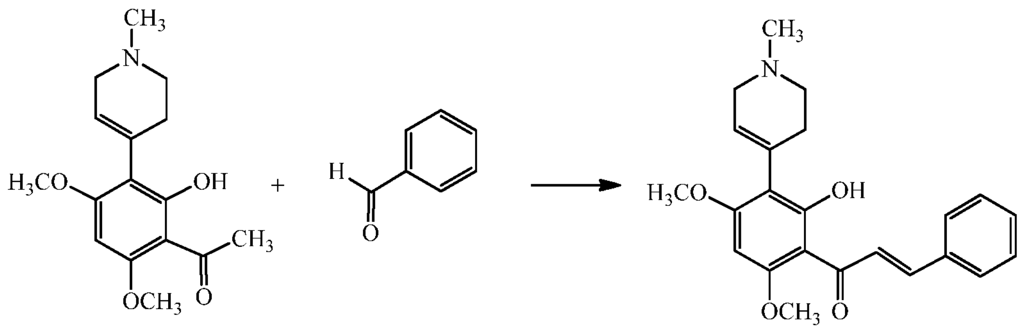

2 3

Reaction and conditions: (c) KOH, EtOH; rt., 3 hr.

EXAMPLE 1

(E)-1-(2-Hydroxy-4,6-dimethoxy-3-(1-methyl-1,2,3,6-tetrahydropyridine-4-yl)phenyl)-3-phenylprop-2-en-1-one (3)

Preparation of 1-methyl-4-(2,4,6-trimethoxy)-1,2,3,6-tetrahydropyridine (1): To a three-necked flask was added 1,3,5-trimethoxy benzene (26.7 g, 0.16 mol) followed by the addition of acetic acid (50 mL). Stirring was continued until the solid was completely dissolved. *N*-Methylpiperidin-4-one (21.2 g, 0.19 mol) was added dropwise at or below 25 °C after which anhydrous HCl was passed in to the reaction solution for 1 hour. The reaction solution was heat to 95 °C - 100 °C for 3 hours. The acetic acid was then removed under reduced pressure to afford a viscous purple crude product.

This product was dissolved in water (50 mL) and the aqueous solution extracted with diethyl ether (20 mL). The pH of the aqueous solution was adjusted to 8-9 using a 40% NaOH aqueous solution, which resulted in the formation of a precipitate that was collected by filtration. The white powder (30 g) that was obtained was recrystalized from petroleum ether: ethyl acetate (10:1) to afford the desired product. M.p.: 118-120 °C (literature value 118-122 °C after recrystalization from petroleum ether). $^1$H-NMR ($CDCl_3$): δ, 6.1 (s, 2H), 5.5 (m, 1H), 3.7-3.8 (3s, 9H), 3.1 (m, 2H), 2.6 (m, 2H), 2.4 (s, 3H), 2.3 (m, 2H).

Preparation of 1-(2-hydroxy-4,6-dimethoxy-3-(1-methyl-1,2,3,6-tetrahydropyridin-4-yl)phenyl)ethanone (2): 1-methyl-4-(2,4,6-trimethoxy)-1,2,3,6-tetrahydropyridine, **1**, (3.2 g, 0.012 mol) is dissolved in methylene chloride (50 mL) and cooled in an ice bath. Boron trifluoride etherate, $BF_3 \cdot Et_2O$ (9.6 mL, 0.072 mol) was added after which was added acetic anhydride (6.4 mL, 0.048 mol). The reaction was stirred at room temperature for 24 hours. The reaction solution was then diluted with water (100 mL). The pH of the resulting aqueous solution was adjusted to 8 with sodium carbonate and the aqueous solution was extracted with methylene chloride (50 mL × 3). The combined organic layer was dried with $Na_2SO_4$. The solvent was removed to afford a yellow powder product (3.7 g), which was dissolved in methanol (20 mL). KOH (50 mL of a 5% aq. sol.) was added and the mixture stirred for 2 hours. A 10% solution of NaOH was added and the insoluble material was removed by filtration. The pH of the resulting filtrate was adjusted to 8-9 with HCl and the resulting light yellow precipitate was collected to afford 2.6 g of the desired product (76% yield). ESI-MS: m/z $[M+1]^+$ 292.3; $^1$HNMR ($CDCl_3$): δ 6.0 (1H, s), 5.6(1H, s), 3.9 (6H, s), 3.1 (2H, m), 2.7 (2H, m), 2.6 (3H, s), 2.4 (3H, s).

Preparation of (E)-1-(2-Hydroxy-4,6-dimethoxy-3-(1-methyl-1,2,3,6-tetrahydropyridine-4-yl)phenyl)-3-phenylprop-2-en-1-one (3): KOH (10.0 g, 0.18 mol) was dissolved in the mixture of ethanol (80 mL) and water (20 mL), followed by the addition of 1-(2-hydroxy-4,6-dimethoxy-3 -(1-methyl-1,2,3,6-tetrahydropyridin-4-yl)phenyl)ethanone, 2, (6.2 g, 0.02 mol) and phenyl aldehyde (0.3 mL, 0.03mol). The reaction was stirred at room temperature for 3 hours. After acidification with 2 N HCl to pH 8-9, the resulting precipitate was collected by filtration and washed with water until the pH of the washings tested neutral. The desired product was obtained as a yellow solid (6.7 g, yield 89%). m.p 173-174 °C. ESI-MS: m/z$[M+1]^+$ 380.6; $^1$H-NMR ($CD_3OD$): δ 7.8-7.9 (2H, dd), 7.7 (2H, m), 7.4-7.5 (3H, m), 6.3 (1H, s), 5.5 (1H, br s), 3.9-4.0 (6H, s), 3.1-3.2 (2H, m), 2.7 (2H, m), 2.4 (3H, s), 2.3 (2H, m).

The following are non-limiting examples of compounds encompassed within the first embodiment of the first category of the disclosed antitumor agents. The artisan will understand that phenyl aldehyde in step (c) of the above disclosed procedure can be replaced by other reagents, *inter alia,* 2-chlorophenyl aldehyde to readily afford the compounds disclosed herein and to prepare other compounds that are not specifically exemplified herein.

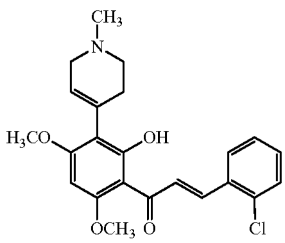

(E)-3-(2-chlorophenyl)-1-(2-hydroxy-4,6-dimethoxy-3-(1-methyl-1,2,3,6-tetrahydropyridine-4-yl)phenyl)-2-propylene-1-one (A2). m.p. 184-185 °C ESI-MS:m/z$[M+1]^+$ 414.3 $^1$HNMR ($CDCl_3$) δ 8.10-8.14 (1H, d, J=15.6Hz), 7.81-7.85 (1H, d, J=15.6Hz), 7.68-7.70 (2H, m), 7.42-7.45 (2H, m), 6.00 (1H, s), 5.59-5.60 (1H, br s), 3.96 (3H, s), 3.86 (3H,s), 3.15-3.16 (2H,m), 2.70-2.73 (2H, m), 2.44 (3H, s), 2.40-2.41 (2H, m); IR (KBr) 3114, 3056, 2943, 1739, 1631, 1563, 1331, 1213, 1125, 970, 759 $cm^{-1}$.

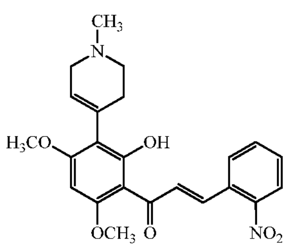

(E)-1-(2-hydroxy-4,6-dimethoxy-3-(1-methyl-1,2,3,6-tetrahydropyridine-4-yl)phenyl)-3-(2-nitrophenyl)-2-propylene-1-one (A3). m.p. 149-151 °C, ESI-MS:m/z$[M+1]^+$ 425.6; $^1$HNMR ($CD_3OD$) δ 7.99-8.03 (1H, d, J=15.6Hz), 7.94-7.98 (1H, d,J =15.6Hz), 7.82-7.90 (2H, m), 7.62-7.71 (2H, m), 6.34 (1H, s), 5.66-5.67 (1H, s), 3.88-3.99 (6H, s), 3.19-3.24 (2H, m), 2.83-2.85 (2H, m), 2.70 (3H, s), 2.64-2.68 (2H, m); IR (KBr) 3424, 2976, 1709, 1614, 1527, 1397,

1339, 1210, 1117, 805 $cm^{-1}$.

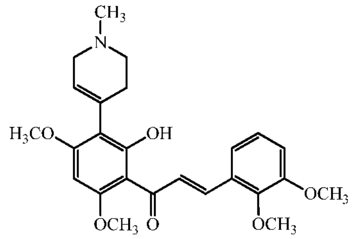

(E)-3-(2,3-dimethoxyphenyl)-1-(2-hydroxy-4,6-dimethoxy-3-(1-methyl-1,2,3,6-tetrahydropyridine-4-yl)phenyl)-2-propylene-1-one (A4). m.p. 171-172 °C; ESI-MS:m/z$[M+1]^+$ 440.6; $^1$HNMR ($CD_3OD$) δ 7.99-8.03 (1H, d, J=15.8Hz), 7.91-7.95 (1H, d, J=15.8Hz), 7.28-7.30 (1H, dd, $J_1$=7.60Hz, $J_2$=1.60Hz), 7.13-7.15 (1H, dd, $J_1$=8.04Hz, $J_2$=7.60Hz), 7.08-7.11 (1H, dd, $J_1$=8.04Hz, $J_2$=1.60Hz), 6.24 (1H, s), 5.52-5.53 (1H, s), 4.01 (3H, s), 3.88-3.90 (9H, s), 3.12-3.14 (2H, m), 2.70-2.73 (2H, t, J=5.84Hz), 2.40 (3H,s), 2.38-2.40(2H, m); IR (KBr) 3423, 2937, 2836, 1735, 1623, 1562, 1419, 1264, 1119, 970, 795 $cm^{-1}$.

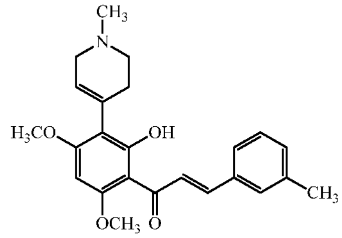

(E)-1-(2-hydroxy-4,6-dimethoxy-3-(1-methyl-1,2,3,6-tetrahydropyridine-4-yl)phenyl)-3-*meta*-methylphenyl-2-propylene-1-one (A5). m.p. 165-166 °C; ESI-MS:m/z$[M+1]^+$ 395.8; $^1$HNMR ($CD_3OD$) δ 7.88-7.91 (1H, d, J=19.6Hz), 7.69-7.73 (1H, d, J=19.6Hz), 7.47-7.48 (2H, m), 7.29-7.35 (2H,m), 6.34 (1H, s), 5.53-5.57 (1H, brs), 3.93 (3H, s), 3.89-3.90 (3H, s), 3.12-3.14 (2H, m), 2.74-2.76 (2H, m), 2.65 (3H, s), 2.41 (3H, s), 2.35-2.37 (2H, m); IR (KBr) 3052, 2930, 2837, 1736, 1681, 1563, 1335, 1244, 1128, 970, 794 $cm^{-1}$.

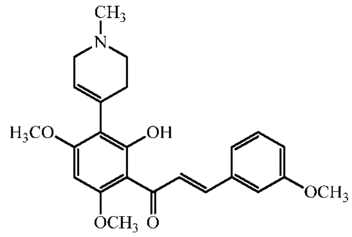

(*E*)-1-(2-hydroxy-4,6-dimethoxy-3-(1-methyl-1,2,3,6-tetrahydropyridine-4-yl)phenyl)-3-(3-methoxyphenyl)-2-propylene-1-one (A6). m.p. 140-141 °C; ESI-MS:m/z$[M+1]^+$ 410.5; $^1$HNMR ($CD_3OD$) δ 7.79-7.83 (1H, d, J=15.6Hz), 7.67-7.71 (1H, d, J=15.6Hz), 7.31-7.38 (1H, m), 7.25-7.29 (1H,m), 7.01-7.05 (1H, m), 6.90-6.92 (1H, m), 6.36 (1H, s), 5.52-5.57 (1H, br s), 3.87-3.93 (9H, s), 3.10-3.12 (2H, m), 2.72-2.75 (2H, m), 2.65-2.68 (3H,s), 2.41 (2H, m); IR (KBr) 3007, 2932, 2888, 1681, 1593, 1564, 1339, 1207, 1121,967, 796 $cm^{-1}$.

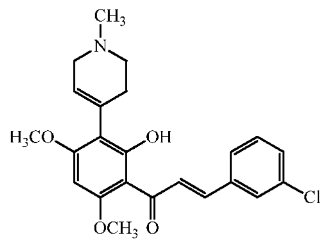

(E)-3-(3-chlorophenyl)-1-(2-hydroxy-4,6-dimethoxy-3-(1-methyl-1,2,3,6-tetrahydropyridine-4-yl)phenyl)-2-propylene-1-one (A7). m.p. 182-184 °C; ESI-MS:m/z[M+1]$^+$ 414.4; $^1$HNMR ($CD_3OD$) δ7.54 (1H, d, J=15.6Hz), 7.50 (1H, d, J=15.6Hz), 7.38-7.41 (2H, m), 7.36-7.38 (2H, m), 6.35 (1H, s), 5.44-5.48 (1H, brs), 3.93 (3H, s), 3.90 (3H, s), 3.13-3.14 (2H,m), 2.67-2.70 (2H, m), 2.40 (3H, s), 2.36-2.39 (2H, m); IR (KBr) 3006, 2932, 1681, 1593, 1567, 1340, 1279, 112, 966, 799 $cm^{-1}$.

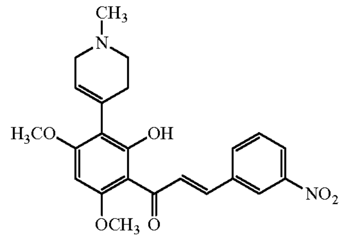

(E)-1-(2-hydroxy-4,6-dimethoxy-3-(1-methyl-1,2,3,6-tetrahydropyridine-4-yl)phenyl)-3-(3-nitrophenyl)-2-propylene-1-one (A8). m.p. 128-130 °C; $^1$HNMR ($CD_3OD$) δ 8.51 (1H, d, J=15.6Hz), 8.46 (1H, d, J=15.6Hz), 8.23-8.28 (1H, m), 8.05-8.08 (1H, m), 7.71-7.74 (1H, m), 7.67-7.71(1H, m), 6.38 (1H, s), 5.60-5.63 (1H, s), 3.90-3.94 (6H, s), 3.16-3.18 (2H, m), 2.98-3.00 (2H, m), 2.42 (3H, s), 2.39-2.40 (2H, m); IR (KBr) 3429, 3082, 2970, 1612, 1588, 1346, 1209, 1120, 967, 804 $cm^{-1}$.

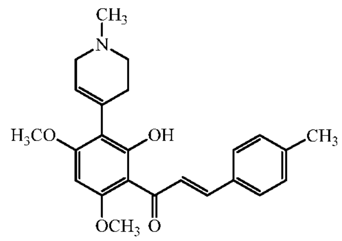

(E)-1-(2-hydroxy-4,6-dimethoxy-3-(1-methyl-1,2,3,6-tetrahydropyridine-4-yl)phenyl)-3-(4-methylphenyl)-2-propylene-1-one (A9). m.p. 150-151 °C; ESI-MS:m/z[M+1]$^+$ 395.2; $^1$HNMR ($CDCl_3$) δ 7.81-7.86 (1H, d, J=15.6Hz), 7.73-7.78 (1H, d, J=15.6Hz), 7.49-7.52 (2H, m), 7.20-7.22 (2H,m), 6.11 (1H, s), 5.59 (1H, br s), 3.95-3.97 (3H, s), 3.85-3.86 (3H, s), 3.12-3.16 (2H, m), 2.90-2.93 (2H, m), 2.44 (3H, s), 2.40 (3H, s), 2.36-2.39 (2H, m); IR (KBr) 2971, 2939, 2883, 1737, 1673,1562, 1510,1327, 1210, 1124, 970, 813 $cm^{-1}$.

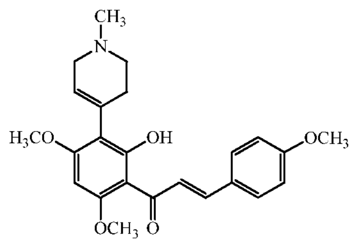

(E)-1-(2-hydroxy-4,6-dimethoxy-3-(1-methyl-1,2,3,6-tetrahydropyridine-4-yl)phenyl)-3-(4-methoxyphenyl)-2-propylene-1-one (A10). m.p. 177-178 °C; ESI-MS:m/z[M+1]$^+$ 410.5; $^1$HNMR ($CD_3OD$) δ 7.80-7.83 (1H, d, J=15.5Hz), 7.71-7.75 (1H, d, J=15.5Hz), 7.61-7.64 (2H, m), 6.99-7.01 (2H, m), 6.25 (1H, s), 5.51-5.53 (1H, br s), 4.03(3H, s), 3.87-3.9 (6H, s), 3.15-3.16 (2H, m), 2.73-2.75 (2H, t, J=5.80Hz), 2.41 (3H, s), 2.39-2.41 (2H, m); IR (KBr) 3120, 3036, 2977, 2936, 2888, 1630, 1604, 1556, 1292, 1124, 975, 828 $cm^{-1}$.

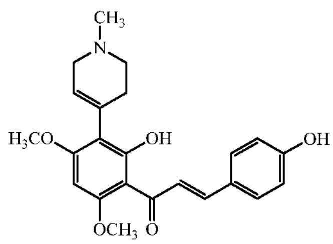

(E)-1-(2-hydroxy-4,6-dimethoxy-3-(1-methyl-1,2,3,6-tetrahydropyridine-4-yl)phenyl)-3-(4-hydroxyphenyl)-2-propylene-1-one (A11). m.p. 98-100 °C; ESI-MS:m/z$[M+1]^+$ 396.4; $^1$HNMR ($CD_3OD$) δ 9.7 (1H, s), 7.77 (1H, d, J=18.4Hz), 7.74 (1H, d, J=18.4Hz), 7.25-7.31 (2H, m), 6.86-6.8 (2H, m), 6.23 (1H, s), 5.56 (1H, br s), 3.97-3.99 (3H, s), 3.87-3.89 (3H, s), 3.57-3.58 (2H, m), 3.16-3.19 (2H, m), 2.61 (3H, s), 2.36-2.37 (2H, m); IR (KBr) 3438, 3005, 2973, 1675, 1615, 1273, 1127, 837 $cm^{-1}$.

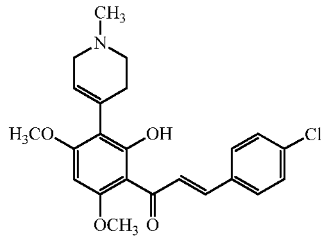

(E)-3-(4-chlorophenyl)-1-(2-hydroxy-4,6-dimethoxy-3-(1-methyl-1,2,3,6-tetrahydropyridine-4-yl)phenyl)-2-propylene-1-one (A12). m.p. 169-170 °C; ESI-MS:m/z$[M+1]^+$ 414.4; $^1$HNMR ($CD_3OD$) δ 7.89-7.93 (1H, d, J=15.5Hz), 7.67-7.71 (1H, d, J=15.5Hz), 7.65-7.66 (2H, m), 7.41-7.49 (2H, m), 6.26 (1H, s), 5.52-5.56 (1H, br s), 3.93 (3H, s), 3.89-3.90 (3H, s), 3.13-3.15 (2H, m), 2.72-2.75 (2H, m), 2.41 (3H, s), 2.35-2.37 (2H, m); IR (KBr) 3067, 3001, 2936, 1677, 1631, 1592, 1328, 1209, 969, 822 $cm^{-1}$.

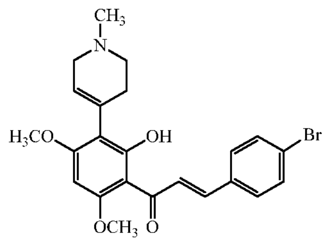

(E)-3-(4-bromophenyl)-1-(2-hydroxy-4,6-dimethoxy-3-(1-methyl-1,2,3,6-tetrahydropyridine-4-yl)phenyl)-2-propylene-1-one (A13). m.p. 164-165 °C; ESI-MS:m/z$[M+1]^+$ 460.4; $^1$HNMR ($CD_3OD$) 87.90-7.94 (1H, d, J=15.6 Hz), 7.66-6.70 (1H, d, J=15.6 Hz), 7.56-7.60 (2H, m), 7.40-7.43 (2H, m), 6.26 (1H, s), 5.53 (1H, br s), 3.93 (3H, s), 3.89 (3H, s), 3.15-3.16 (2H, m), 2.74 (2H, m), 2.41 (3H, s), 2.36 (2H, m); IR (KBr) 3008, 2934, 1678, 1623, 1593, 1322, 1209, 1123, 965, 819 $cm^{-1}$.

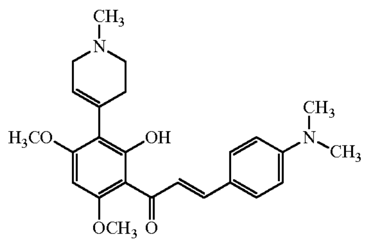

(E)-3-(4-dimethylaminophenyl)-1-(2-hydroxy-4,6-dimethoxy-3-(1-methyl-1,2,3,6-tetrahydropyridine-4-yl)phenyl)-2-propylene-1-one (A14). m.p. 176-177 °C; ESI-MS:m/z$[M+1]^+$ 423.5; $^1$HNMR (DMSO-$d_6$) δ7.70-7.74 (1H, d, J=15.4Hz), 7.64-7.68 (1H, d, J=15.4Hz), 7.55-7.58 (2H, d, J=8.92 Hz), 6.75-6.77 (2H, d, J=8.92 Hz), 6.26 (1H, s), 5.40-5.41(1H, br s), 3.99 (3H, s), 3.85(3H, s), 3.30 (2H, m), 3.00 (6H, s), 2.92-2.93 (2H, m), 2.26 (3H, s), 2.20 (2H, m); IR (KBr) 2966, 2931, 2840, 1742, 1600, 1547, 1331, 1208, 1158, 971, 816 $cm^{-1}$.

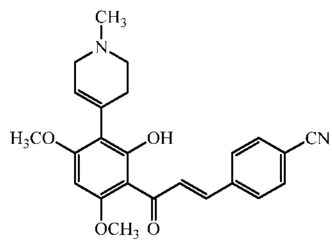

(E)-3-(4-cyanophenyl)-1-(2-hydroxy-4,6-dimethoxy-3-(1-methyl-1,2,3,6-tetrahydropyridine-4-yl)phenyl)-2-propylene-1-one (A15). m.p. 125-126 °C; ESI-MS:m/z[M+1]$^+$ 405.6; $^1$HNMR (DMSO-$d_6$) δ 7.85-7.88 (1H, d, J=15.6Hz), 7.79-7.82 (1H, d, J=15.6Hz), 7.55-7.59 (2H, m), 7.45-7.48 (2H, m), 6.14 (1H, s), 5.62-5.63 (1H, br s), 3.90 (6H, s), 3.12-3.13 (2H, m), 2.63-2.67 (2H, m), 2.38 (3H, s), 2.22-2.23 (2H, m); IR (KBr) 3403,3185, 2976,2941, 2226, 1674, 1615, 1507, 1394, 1211, 1118, 964, 805 cm$^{-1}$.

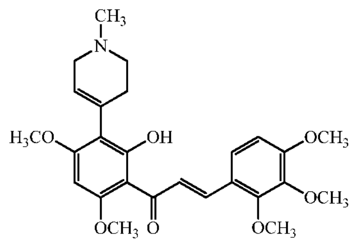

(E)-1-(2-hydroxy-4,6-dimethoxy-3-(1-methyl-1,2,3,6-tetrahydropyridine-4-yl)phenyl)-3-(2,3-4-trimethoxyphenyl)-2-propylene-1-one (A16). m.p. 145-147 °C; ESI-MS:m/z[M+1]$^+$ 470.5; $^1$HNMR ($CD_3OD$) δ 7.81-7.85 (1H, d, J=15.6Hz), 7.64-7.68 (1H, d, J=15.6Hz), 6.97 (1H, d), 6.78 (1H, d), 6.25 (1H, s), 5.53-5.58 (1H, br s), 3.78-3.93 (15H, s), 3.09-3.14 (2H, m), 2.65-2.67 (1H, m), 2.71-2.74 (1H, m), 2.40 (3H, s), 2.34 (2H, m); IR (KBr) 3116, 2997, 2936, 2836, 1735, 1680, 1568, 1346, 1278, 1244, 1209, 112, 970, 802 cm$^{-1}$.

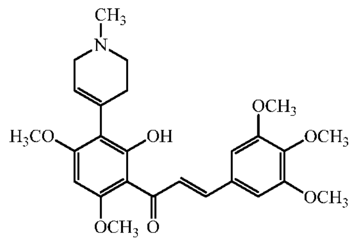

(E)-1-(2-hydroxy-4,6-dimethoxy-3-(1-methyl-1,2,3,6-tetrahydropyridine-4-yl)phenyl)-3-(3,4,5-trimethoxyphenyl)-2-propylene-1-one (A17). m.p. 156-158 °C; ESI-MS:m/z[M+1]$^+$ 470.5; $^1$HNMR ($CD_3OD$) δ 7.84-7.88 (1H, d, J=15.6Hz), 7.69-7.73 (1H, d, J=15.6Hz), 6.96-6.99 (2H, m), 6.31(1H, s), 5.61(1H, br s), 3.91-3.93 (9H, s), 3.83-3.84 (6H, s), 3.21(2H, m), 2.91(2H, m), 2.64 (3H, s); IR (KBr) 3116, 3004, 2936, 1674, 2837, 1623, 1565, 1506, 1320, 1278, 1209, 1126, 970, 887, 825 cm$^{-1}$.

A second embodiment of the first category of the disclosed antitumor agents has the formula:

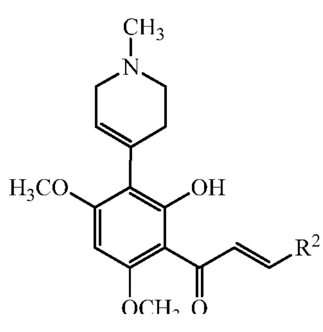

wherein $R^{23}$ is 1,2,3,6-tetrahydropyridin-4-yl and the index y is equal to 0. For this embodiment, $R^2$ units comprise

substituted or unsubstituted phenyl, non-limiting examples of which are further disclosed herein below in Table II.

TABLE II

| No. | $R^2$ | No. | $R^2$ |
|---|---|---|---|
| B1 | phenyl | B30 | 2-fluorophenyl |
| B2 | 2-chlorophenyl | B31 | 2-methylphenyl |
| B3 | 2-nitrophenyl | B32 | 2-hydroxyphenyl |
| B4 | 2,3-dimethoxyphenyl | B33 | 2-bromophenyl |
| B5 | 3-methylphenyl | B34 | 2-cyanophenyl |
| B6 | 3-methoxyphenyl | B35 | 2-methoxyphenyl |
| B7 | 3-chlorophenyl | B36 | 2-dimethylaminophenyl |
| B8 | 3-nitrophenyl | B37 | 3-fluorophenyl |
| B9 | 4-methylphenyl | B38 | 3-bromophenyl |
| B10 | 4-methoxyphenyl | B39 | 3-cyanophenyl |
| B11 | 4-hydroxyphenyl | B40 | 3-hydroxyphenyl |
| B12 | 4-chlorophenyl | B41 | 3-dimethylaminophenyl |
| B13 | 4-bromophenyl | B42 | 3,4-dimethoxyphenyl |
| B14 | 4-dimethylaminophenyl | B43 | 4-nitrophenyl |
| B15 | 4-cyanophenyl | B44 | 4-fluorophenyl |
| B16 | 2,3,4-trimethoxyphenyl | B45 | 4-sulfonylamino |
| B17 | 3,4,5-trimethoxyphenyl | B46 | 4-formylamino |

The compounds encompassed within the second embodiment of the first category of the disclosed antitumor agents can be prepared by substituting piperidin-4-one for N-methylpiperidin-4-one in the procedure outlined in Scheme I and disclosed in Example 1 herein above. The following are non-limiting examples of compounds encompassed within the second embodiment of the first category of the disclosed antitumor agents.

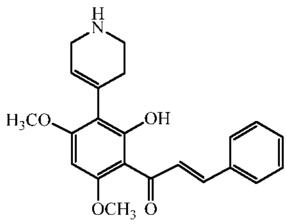

1-(2-hydroxy-4,6-dimethoxy-3-(1,2,3,6-tetrahydropyridine-4-yl)phenyl)-3-phenyl-2-propylene-1-one (B1). m.p. 118-120 °C; ESI-MS:m/z$[M+1]^+$ 366.3; $^1H$ NMR ($CD_3OD$) $\delta$ 7.91-7.95 (1H, d, J=15.6Hz), 7.72-7.76 (1H, d, J=15.6Hz), 7.66-7.69 (2H, m), 7.43-7.47 (3H, m), 6.27 (1H, s), 5.58 (1H, s), 3.94 (3H, s), 3.91 (3H, s), 3.50-3.52 (2H, m), 3.10-3.14 (2H, m), 2.33-2.34 (2H, m); IR (KBr) 3440, 3057, 2938, 1668, 1611, 1209, 1120, 970, 700 $cm^{-1}$.

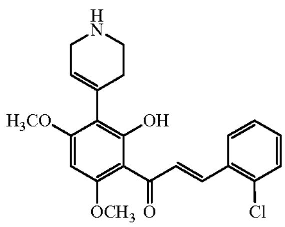

(E)-3-(2-chlorophenyl)-1-(2-hydroxy-4,6-dimethoxy-3-(1,2,3,6-tetrahydropyridine-4 -yl)phenyl)-2-propylene-1-one (B2). m.p. 105-106 °C; ESI-MS:m/z[M+1]$^+$ 400.7; $^1$H NMR ($CD_3OD$) δ8.07-8.11 (1H, d, J=17.9Hz), 7.89-7.93 (1H, d, J=17.9Hz), 7.82-7.85 (2H, m), 7.49-7.51 (2H, m), 6.26 (1H, s), 5.57 (1H, s), 4.03 (3H, s), 3.95 (3H, s), 3.47 (2H, m), 3.06-3.08 (2H, m), 2.29-2.30 (2H, m); IR (KBr) 3442, 3061, 2965, 1673, 1161, 1569, 1209, 1121,971, 753 cm$^{-1}$.

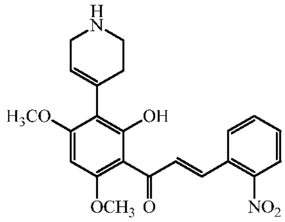

(E)-1-(2-hydroxy-4,6-dimethoxy-3-(1,2,3,6-tetrahydropyridine-4-yl)phenyl)-3-(2-nitrophenyl)-2-propylene-1-one (B3). m.p. 128-130 °C; ESI-MS:m/z[M+1]$^+$ 411.5; $^1$H NMR ($CD_3OD$) δ 8.11 (1H, d, J=15.6Hz), 7.85 (1H, m), 7.75 (H, d, J=15.6Hz), 7.42-7.43 (1H, m),7.36-7.39 (2H, m), 6.26 (1H, s), 3.96 (6H, s), 3.32 (2H, m), 3.15-3.17 (2H, m), 2.62 (2H, m); IR (KBr) 3423, 2979, 1613, 1500, 1462, 1276, 1211, 1123, 973, 765 cm$^{-1}$.

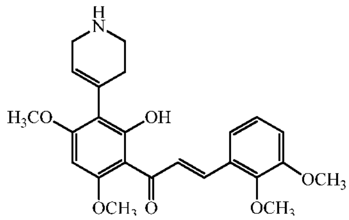

(E)-3-(2,3-dimethoxyphenyl)-1-(2-hydroxy-4,6-dimethoxy-3-(1,2,3,6-tetrahydropyridine-4-yl)phenyl)-2-propylene-1-one (B4). m.p. 109-110 °C; ESI-MS:m/z[M+1]$^+$ 426.5; $^1$H NMR ($CD_3OD$) δ 7.99-8.03 (1H, d, J=15.8Hz), 7.92-7.96 (1H, d, J =15.8Hz), 7.28-7.30 (1H, dd, $J_1$=7.6Hz, $J_2$=1.1Hz), 7.11-7.16 (2H, m), 6.27 (1H, s), 5.60 (1H, s), 4.02 (3H, s), 3.90 (9H, s), 3.64 (2H, m), 3.32-3.34 (2H, t, J=5.80Hz), 2.43-2.44 (2H, m); IR (KBr) 3427, 2969, 2835, 1622, 1534, 1331, 1268, 1121, 970, 799, 747 cm$^{-1}$.

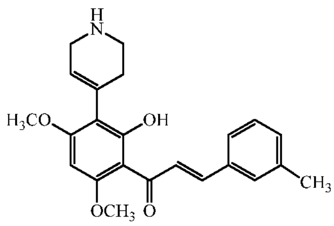

(E)-1-(2-hydroxy-4,6-dimethoxy-3-(1,2,3,6-tetrahydropyridine-4-yl)phenyl)-3-(3-methylphenyl)-2-propylene-1-one (B5). m.p. 95-96 °C; ESI-MS:m/z[M+1]$^+$ 380.5; $^1$H NMR ($CD_3OD$) δ 7.87-7.91 (1H, d, J=15.6 Hz), 7.69-7.73 (1H, d, J=15.6 Hz), 7.46-7.48 (2H, m), 7.27-7.34 (2H, m), 6.27 (1H, s), 5.57-5.59 (1H, s), 4.03 (3H, s), 3.93 (3H, s), 3.50-3.52 (2H, m), 3.10-3.13 (2H, t, J=5.80Hz), 2.40 (3H, s), 2.32-2.34 (2H, m); IR (KBr) 3441, 3004, 2939, 2841, 1673, 1610, 1569, 1324, 1209, 1121, 971, 797 cm$^{-1}$.

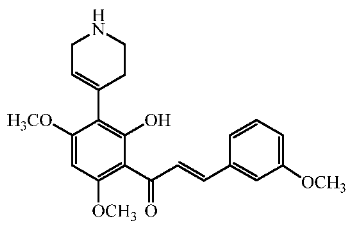

(E)-1-(2-hydroxy-4,6-dimethoxy-3-(1,2,3,6-tetrahydropyridine-4-yl)phenyl)-3-(3-methoxyphenyl)-2-propylene-1-one (B6). m.p. 99-100 °C; ESI-MS:m/z$[M+1]^+$ 396.4; $^1H$ NMR ($CD_3OD$) $\delta$7.88-7.92 (1H, d, J=15.6Hz), 7.68-7.72 (1H, d, J=15.6Hz), 7.38 (1H, dd, $J_1$=8.00Hz, $J_2$=7.84Hz), 7.34 (1H, m), 7.25-7.27 (1H, m), 6.99-7.02 (1H, dd, $J_1$=2.40Hz, $J_2$=1.92Hz), 6.27 (1H, s), 5.57 (1H, s), 4.03 (3H, s), 3.93 (3H, s), 3.89 (3H, s), 3.48 (2H, m), 3.06-3.09 (2H, m, J=5.80Hz), 2.30-2.32 (2H, m); IR (KBr) 3441, 2937, 2835, 1674, 1596, 1260, 1209, 1159, 1120, 970, 795 $cm^{-1}$.

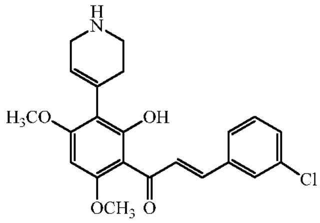

(E)-3 -(3 -chlorophenyl)-1-(2-hydroxy-4,6-dimethoxy-3-(1,2,3,6-tetrahydropyridine-4-yl)phenyl)-2-propylene-1-one (B7). m.p. 102-103 °C; ESI-MS:m/z$[M+1]^+$ 400.4; $^1H$ NMR ($CD_3OD$) $\delta$ 7.90-7.94 (1H, d, J=15.6Hz), 7.65-7.69 (1H, d, J=15.6Hz), 7.61-7.62 (1H, m), 7.44-7.45 (1H, m), 7.43-7.44 (2H, m), 6.27 (1H, s), 5.59 (1H, s), 4.04 (3H, s), 3.92 (3H, s), 3.60 (2H, m), 3.14-3.17 (2H, m), 2.36-2.38 (2H, m); IR (KBr) 3441, 3002, 2938, 1611, 1571, 1323, 1293, 1209, 1121, 971, 795 $cm^{-1}$.

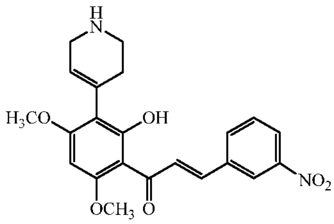

(E)-1-(2-hydroxy-4,6-dimethoxy-3-(1,2,3,6-tetrahydropyridine-4-yl)phenyl)-3-(3-nitrophenyl)-2-propylene-1-one (B8). m.p. 199-200 °C; ESI-MS:m/z$[M+1]^+$ 411.4; $^1H$ NMR ($CD_3OD$) $\delta$ 8.52 (1H, d, J=15.6Hz), 8.46 (1H, d, J=15.6Hz), 8.28-8.33 (1H, m), 8.03-8.09 (1H, m), 7.72-7.87 (2H, m), 6.33 (1H, s), 5.65 (1H, s), 3.99 (6H, s), 3.51 (2H, m), 3.12 (2H, m), 2.71 (2H, m); IR (KBr) 3449, 2978, 1703, 1613, 1590, 1529, 1477, 1396, 1171,870, 807 $cm^{-1}$.

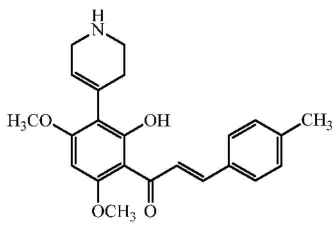

(E)-1-(2-hydroxy-4,6-dimethoxy-3-(1,2,3,6-tetrahydropyridine-4-yl)phenyl)-3-(4-methylphenyl)-2-propylene-1-one (B9). m.p. 98-100 °C; ESI-MS:m/z$[M+1]^+$ 380.4; $^1H$ NMR ($CD_3OD$) $\delta$ 7.85-7.89 (1H, d, J=15.6Hz), 7.69-7.73 (1H, d, J=15.6Hz), 7.54-7.56 (2H, d, J=8.08Hz), 7.26-7.28 (2H, d, J=7.96HZ), 6.26 (1H, s), 5.58 (1H, s), 4.03 (3H, s), 3.90 (3H, s), 3.51-3.52 (2H, m), 3.12-3.14 (2H, t, J=5.84Hz), 2.39 (3H, s), 2.33-2.36 (2H, m); IR (KBr) 3425, 3003, 2920, 2842, 1611, 1567, 1208, 1160, 1121, 972, 814 $cm^{-1}$.

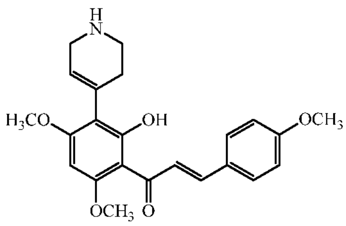

(E)-1-(2-hydroxy-4,6-dimethoxy-3-(1,2,3,6-tetrahydropyridine-4-yl)phenyl)-3-(4-methoxyphenyl)-2-propylene-1-one (B10). m.p. 90-91 °C; ESI-MS:m/z$[M+1]^+$ 396.3; $^1H$ NMR ($CD_3OD$) δ 7.81-7.85 (1H, d, J=14.7Hz), 7.73-7.77 (1H, d, J=14.7Hz), 7.62-7.64 (2H, d, J=8.76Hz), 6.96-7.01 (2H, d, J=8.72Hz), 6.29 (1H, s), 5.63 (1H, s), 4.05 (3H, s), 3.94 (3H, s), 3.87 (3H, s), 3.47-3.52 (2H, m), 3.39-3.41 (2H, m), 2.57 (2H, m); IR (KBr) 3441, 3000, 2936, 2836, 1672, 1606, 1518, 1250, 1209, 1160, 1119, 970, 828 $cm^{-1}$.

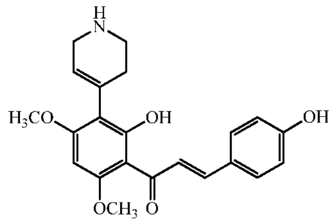

(E)-1-(2-hydroxy-4,6-dimethoxy-3-(1,2,3,6-tetrahydropyridine-4-yl)phenyl)-3-(4-hydroxyphenyl)-2-propylene-1-one (B11). m.p. 109-110 °C; ESI-MS:m/z$[M+1]^+$ 382.5; $^1H$ NMR ($CD_3OD$) δ 7.91-7.95 (2H, d, J=15.6Hz), 7.70-7.74 (1H, d, J=15.6Hz), 7.66-7.6 (2H, m), 7.45-7.47 (2H, m), 6.30 (1H, s), 5.64 (1H, s), 4.03 (3H, s), 3.91 (3H, s), 3.74 (2H, m), 3.40-3.41 (2H, m), 2.56-2.59 (2H, m); IR (KBr) 3430, 2976, 1611, 1513, 1275, 1124, 803 $cm^{-1}$.

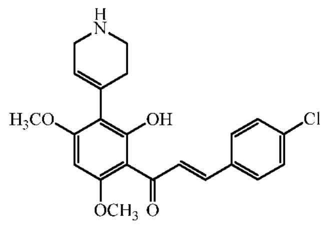

(E)-3-(4-chlorophenyl)-1-(2-hydroxy-4,6-dimethoxy-3-(1,2,3,6-tetrahydropyridine-4-yl)phenyl)-2-propylene-1-one (B12). m.p. 93-94 °C; ESI-MS:m/z$[M+1]^+$ 400.0; $^1H$ NMR ($CD_3OD$) δ 7.90-7.94 (2H, d, J=15.6Hz), 7.69-7.73 (1H, d, J=15.6Hz), 7.66-7.68 (2H, d, J=8.52Hz), 7.44-7.46 (2H, d, J=8.60Hz), 6.31 (1H, s), 5.64 (1H, s), 4.03 (3H, s), 3.94 (3H, s), 3.52-3.54 (2H, m), 3.22-3.25 (2H, m), 2.43 (2H, m); IR (KBr) 3422, 2941, 1611, 1571, 1325, 1209, 1121, 970, 821 $cm^{-1}$.

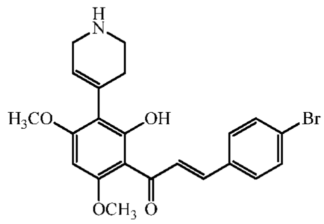

(E)-3-(4-bromophenyl)-1-(2-hydroxy-4,6-dimethoxy-3-(1,2,3,6-tetrahydropyridine-4-yl)phenyl)-2-propylene-1-one (B13). m.p. 120-121 °C; ESI-MS:m/z$[M+1]^+$ 445.4; $^1H$ NMR ($CD_3OD$) δ7 .91-7.95 (1H, d, J=15.6Hz), 7.66-7.70 (1H, d, J=15.6Hz), 7.60-7.62 (2H, dd, $J_1$=8.52Hz, $J_2$=3.16Hz), 7.40-7.42 (2H, dd, $J_1$=8.48Hz, $J_2$=3.52Hz), 6.28 (1H, s), 5.60 (1H, s), 4.04 (3H, s), 3.91 (3H, s), 3.50-3.51 (2H, m), 3.10-3.11 (2H, m), 2.32-2.33 (2H, m); IR (KBr) 3444, 3002, 2936, 1673, 1610, 1599, 1329, 1208, 1120, 971, 818 $cm^{-1}$.

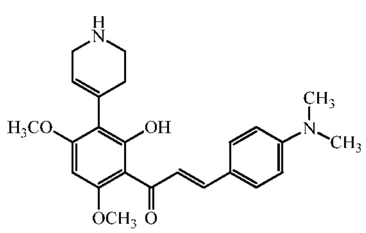

(E)-3-(4-dimethylaminophenyl)-1-(2-hydroxy-4,6-dimethoxy-3-(1,2,3,6-tetrahydropyridine-4-yl)phenyl)-2-propylene-1-one (B14). m.p. 127-128 °C; ESI-MS:m/z[M+1]+ 409.6; $^1$HNMR ($CD_3OD$) δ7.76-7.78 (2H, d, J=15.6Hz), 7.53-7.55 (2H, d, J=8.88Hz), 6.78-6.80 (2H, d, J=8.92Hz), 6.28 (1H, s), 5.63 (1H, s), 4.05 (3H, s), 3.91 (3H, s), 3.77 (2H, m), 3.39 (2H, m), 3.06 (6H, s), 2.56 (2H, m); IR (KBr) 3423, 2940, 2806, 1604, 1550, 1333, 1208, 1158, 1121, 969, 815 $cm^{-1}$.

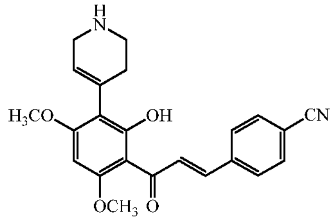

(E)-3-(4-cyanophenyl)-1-(2-hydroxy-4,6-dimethoxy-3-(1,2,3,6-tetrahydropyridine-4-yl)phenyl)-2-propylene-1-one (B15). m.p. 135-136 °C; ESI-MS:m/z[M+1]+ 391.3; $^1$H NMR ($CD_3OD$) δ 7.98-8.02 (1H, d, J=15.5Hz), 7.81-7.85 (1H, d, J=15.5Hz), 7.69-7.73 (2H, m), 7.59-7.61 (2H, m), 6.28 (1H, s), 5.55 (1H, s), 3.92 (6H, s), 3.47-3.53 (2H, m), 3.10-3.13 (2H, t, J=5.80Hz), 2.66-2.68 (2H, m); IR (KBr) 3430, 2969, 2227, 1676, 1594, 1505, 1343, 1210, 1121, 829, 801 $cm^{-1}$.

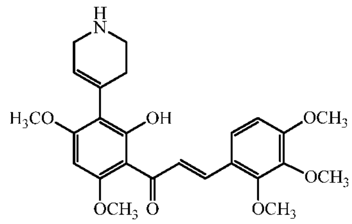

(E)-1-(2-hydroxy-4,6-dimethoxy-3-(1,2,3,6-tetrahydropyridine-4-yl)phenyl)-3-(2,3,4-trimethoxyphenyl)-2-propylene-1-one (B16). m.p. 98-100 °C; ESI-MS:m/z[M+1]+ 456.4; $^1$HNMR ($CD_3OD$) δ 7.82-7.6 (1H, d, J=15.5Hz), 7.66-7.70 (1H, d, J=15.5Hz), 6.98 (2H, m), 6.26 (1H, s), 5.58 (1H, s), 4.03 (3H, s), 3.93 (12H, s), 3.50-3.52 (2H, m), 3.10-3.13 (2H, t, J=5.80Hz), 2.33 (2H, m); IR (KBr) 3441, 2938, 2836, 1673, 1612, 1592, 1504, 1318, 1279, 1209, 1152, 1122, 970, 797 $cm^{-1}$.

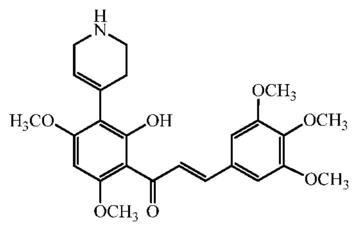

(E)-1-(2-hydroxy-4,6-dimethoxy-3-(l,2,3,6-tetrahydropyridine-4-yl)phenyl)-3-(3, 4,5-trimethoxyphenyl)-2-propylene-1-one (B17). m.p. 124-125 °C; ESI-MS:m/z[M+1]+ 456.4; $^1$HNMR ($CD_3OD$) δ 7.83-7.87 (1H, d, J=15.5Hz), 7.67-7.71 (1H, d, J=15.5Hz), 6.99 (2H, m), 6.28 (1H, s), 5.55 (1H, s), 3.92 (15H, s), 3.52 (2H, m), 3.15-3.17 (2H, t, J=5.80Hz), 2.72-2.74 (2H, m); IR (KBr) 3442, 2938, 2836, 1674, 1593, 1504, 1320, 1279, 1124, 800 $cm^{-1}$.

A third embodiment of the first category of the disclosed antitumor agents has the formula:

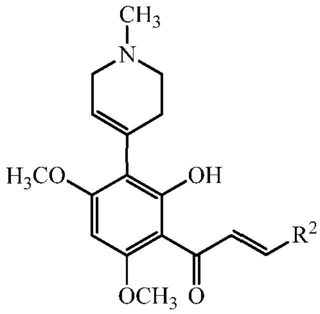

wherein $R^{23}$ is 1,2,3,6-tetrahydropyridin-4-yl or 1-methyl-1,2,3,6-tetrahydropyridin-4-yl and the index y is equal to 0. For this embodiment, $R^2$ units comprise substituted or unsubstituted $C_1$-$C_5$ heteroaryl units, non-limiting examples of which are further disclosed herein below in Table III.

TABLE III

| No. | $R^{23}$ | $R^2$ |
|---|---|---|
| E1 | 1-methyl-1,2,3,6-tetrahydropyridin-4-yl | furan-2-yl |
| E2 | 1,2,3,6-tetrahydropyridin-4-yl | furan-2-yl |
| E3 | 1-methyl-1,2,3,6-tetrahydropyridin-4-yl | furan-3-yl |
| E4 | 1,2,3,6-tetrahydropyridin-4-yl | furan-3-yl |
| E5 | 1-methyl-1,2,3,6-tetrahydropyridin-4-yl | [1,2,3]triazol-4-yl |
| E6 | 1,2,3,6-tetrahydropyridin-4-yl | [1,2,3]triazol-4-yl |
| E7 | 1-methyl-1,2,3,6-tetrahydropyridin-4-yl | [1,2,3]triazol-5-yl |
| E8 | 1,2,3,6-tetrahydropyridin-4-yl | [1,2,3]triazol-5-yl |
| E9 | 1-methyl-1,2,3,6-tetrahydropyridin-4-yl | [1,2,4]triazol-5-yl |
| E10 | 1,2,3,6-tetrahydropyridin-4-yl | [1,2,4]triazol-5-yl |
| E11 | 1-methyl-1,2,3,6-tetrahydropyridin-4-yl | imidazol-2-yl |
| E12 | 1,2,3,6-tetrahydropyridin-4-yl | imidazol-2-yl |
| E13 | 1-methyl-1,2,3,6-tetrahydropyridin-4-yl | imidazol-4-yl |
| E14 | 1,2,3,6-tetrahydropyridin-4-yl | imidazol-4-yl |
| E15 | 1-methyl-1,2,3,6-tetrahydropyridin-4-yl | pyrrol-2-yl |
| E16 | 1,2,3,6-tetrahydropyridin-4-yl | pyrrol-2-yl |
| E17 | 1-methyl-1,2,3,6-tetrahydropyridin-4-yl | pyrrol-3-yl |
| E18 | 1,2,3,6-tetrahydropyridin-4-yl | pyrrol-3-yl |
| E19 | 1-methyl-1,2,3,6-tetrahydropyridin-4-yl | oxazol-2-yl |
| E20 | 1,2,3,6-tetrahydropyridin-4-yl | oxazol-2-yl |
| E21 | 1-methyl-1,2,3,6-tetrahydropyridin-4-yl | oxazol-4-yl |
| E22 | 1,2,3,6-tetrahydropyridin-4-yl | oxazol-4-yl |
| E23 | 1-methyl-1,2,3,6-tetrahydropyridin-4-yl | oxazol-5-yl |
| E24 | 1,2,3,6-tetrahydropyridin-4-yl | oxazol-5-yl |
| E25 | 1-methyl-1,2,3,6-tetrahydropyridin-4-yl | thiazol-2-yl |
| E26 | 1,2,3,6-tetrahydropyridin-4-yl | thiazol-2-yl |
| E27 | 1-methyl-1,2,3,6-tetrahydropyridin-4-yl | thiazol-4-yl |

(continued)

| No. | $R^{23}$ | $R^2$ |
|---|---|---|
| E28 | 1,2,3,6-tetrahydropyridin-4-yl | thiazol-4-yl |
| E29 | 1-methyl-1,2,3,6-tetrahydropyridin-4-yl | thiazol-5-yl |
| E30 | 1,2,3,6-tetrahydropyridin-4-yl | thiazol-5-yl |

The compounds encompassed within the third embodiment of the first category of the disclosed antitumor agents can be prepared by substituting $C_1$-$C_5$ heteroaryl carboxaldehydes for the substituted or unsubstituted phenyl aldehydes in the procedure outlined in Scheme I and disclosed in Example 1 herein above. Non-limiting examples, include pyroll-2-carbaldehyde [CAS No. 1003-29-8], imidazol-2-carbaldehyde [CAS No. 10111-08-7], oxazol-2-carbaldehyde [CAS No. 65373-52-6], [1,2,3]triazol-4-carbaldehyde [CAS No. 32829-25-7], thiazol-4-carbaldehyde [CAS No. 3364-80-5] and thiophen-2-carbaldehyde [CAS No. 98-03-3]. The following are non-limiting examples of compounds encompassed within the second embodiment of the first category of the disclosed antitumor agents.

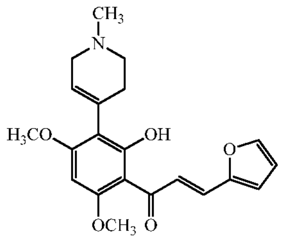

(E)-3-(furan-2-yl)-1-(2-hydroxy-4,6-dimethoxy-3-(1-methyl-1,2,3,6-tetrahydro-pyridine-4-yl)phenyl)-2-propylene-1-one (E1). m.p. 145-146 °C; ESI-MS:m/z[M+1]$^+$ 370.4; $^1$H NMR ($CDCl_3$) $\delta$ 7.74-7.78 (1H, d, J=15.8Hz), 7.67 (1H, m), 7.55-7.57 (1H, d, J=15.8Hz), 6.80 (1H, m), 6.58-6.60 (1H, m), 6.23 (1H, s), 5.52 (1H, s), 4.00 (3H, s), 3.88 (3H, s), 3.11-3.13 (2H, m), 2.69-2.72 (2H, m), 2.39 (3H, s), 2.38 (2H. m); IR (KBr) 3107, 2938, 1629, 1551, 1429, 1321, 1220, 1126, 973, 920, 878, 796 cm$^{-1}$.

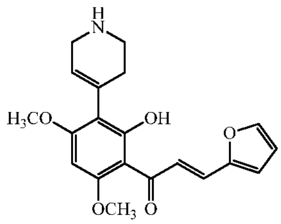

(E)-3-(furan-2-yl)-1-(2-hydroxy-4,6-dimethoxy-3-(1,2,3,6-tetrahydropyridin-4-yl)phenyl)-2-propylene-1-one (E2). m.p. 119-120 °C; ESI-MS:m/z[M+1]$^+$ 356.6; $^1$H NMR ($CDCl_3$) $\delta$ 9.06-9.10 (1H, d, J=15.6Hz), 8.85-8.89 (1H, d, J=15.6Hz), 8.18-8.21 (1H, m), 7.90-7.92 (1H, m), 7.55-7.64 (1H, m), 5.28 (6H, s), 4.23-4.26 (2H, m), 3.31-3.36 (2H, m); IR (KBr) 3429, 3118, 2969, 1703, 1611, 1571, 1415, 1210, 1161, 1121, 969, 82, 798, 746 cm$^{-1}$.

A second category of antitumor agents disclosed herein have the formula:

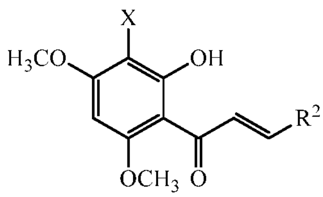

wherein X has the formula -$[CH_2]_yR^{23}$; $R^{23}$ is $C_1$-$C_5$ substituted or unsubstituted heterocyclic ring; and the index y is equal to 1 to 5.

A first embodiment of the second category of the disclosed antitumor agents has the formula:

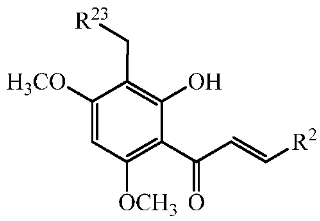

wherein $R^{23}$ is a $C_1$-$C_5$ heterocyclic ring and the index y is equal to 1. For this embodiment, $R^2$ units comprise substituted or unsubstituted phenyl, non-limiting examples of which are further disclosed herein below in Table IV.

TABLE IV

| No. | $R^2$ | $R^{23}$ | No. | $R^2$ | $R^{23}$ |
|---|---|---|---|---|---|
| C1 | 4-methylphenyl | piperidin1-yl | D1 | 4-methylphenyl | morpholin-4-yl |
| C2 | 4-methoxyphenyl | piperidin1-yl | D2 | 4-methoxyphenyl | morpholin-4-yl |
| C3 | 3 -methoxyphenyl | piperidin1-yl | D3 | 4-chlorophenyl | morpholin-4-yl |
| C4 | 3-chlorophenyl | piperidin1-yl | D4 | 4-bromophenyl | morpholin-4-yl |
| C5 | 3-methylphenyl | piperidin1-yl | D5 | 3-chlorophenyl | morpholin-4-yl |
| C6 | 4-bromophenyl | piperidin1-yl | D6 | 3-methoxyphenyl | morpholin-4-yl |
| C7 | 4-cyanophenyl | piperidin1-yl | D7 | 3-methylphenyl | morpholin-4-yl |
| C8 | 3-fluorophenyl | piperidin1-yl | D8 | 3,4,5-trimethoxyphenyl | morpholin-4-yl |
| C9 | 4-fluorophenyl | piperidin1-yl | D9 | 4-fluorophenyl | morpholin-4-yl |
| C10 | 3-cyanophenyl | piperidin1-yl | D10 | 3-cyanophenyl | morpholin-4-yl |
| C11 | 3-nitrophenyl | piperidin1-yl | D11 | 3-nitrophenyl | morpholin-4-yl |
| C12 | 4-nitrophenyl | piperidin1-yl | D12 | 4-nitrophenyl | morpholin-4-yl |
| C13 | 4-sulfonylamino | piperidin1-yl | D13 | 4-sulfonylamino | morpholin-4-yl |
| C14 | 4-formylamino | piperidin1-yl | D14 | 4-formylamino | morpholin-4-yl |
| C15 | 4-cyanophenyl | piperidin1-yl | D15 | 4-cyanophenyl | morpholin-4-yl |

The compounds that comprise this embodiment of the second category of the disclosed antitumor agents can be prepared by the procedure outlined below in Scheme II and disclosed in Example 2.

Scheme II

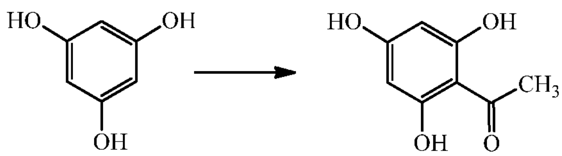

4

Reagents and conditions: (a) HCl (g), $CH_3CN$; -20 °C, 2 hr.

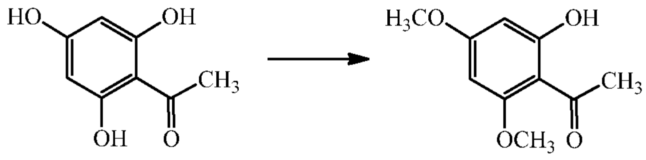

4 5

Reagents and conditions: (b) $(CH_3)_2SO_4$, $K_2CO_3$, acetone; rt. 1 hr.

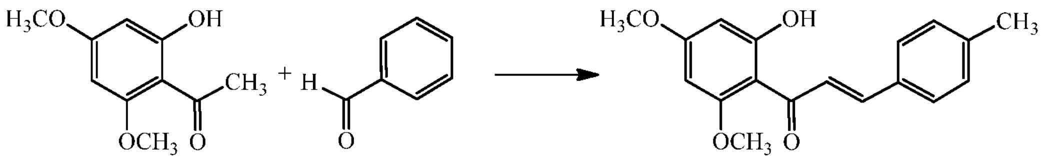

5 6

Reagents and conditions: (c) KOH, EtOH/$H_2O$; rt. 3 hr.

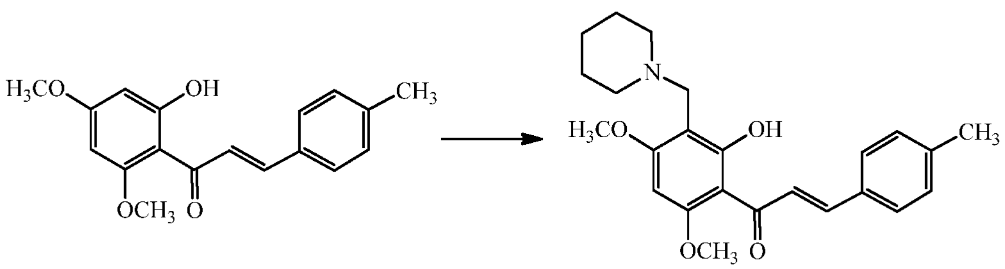

6 7

Reagents and conditions: (d) HCHO/$HCO_2H$, piperidine; reflux, 2 hr.

EXAMPLE 2

(E)-1-(2-hydroxy-4,6-dimethoxy-3-(piperidin-1-ylmethyl)phenyl)-3-(4-methylphenyl)-2-propylene-1-one (7)

Preparation of 1-(2,4,6-trihydroxyphenyl)ethanone (4): To a 3-neck flask was added 1,3,5-trihydroxybenzene (10.1 g, 0.10 mol), anhydrous zinc chloride (2.5 g, 0.018 mol), and anhydrous ethyl ether (50 mL). After the solids had completely dissolved, anhydrous $CH_3CN$ (11 mL, 0.20 mol) was added. Dry HCl gas was introduced to the reaction solution for 2 hours at a temperature below -20 °C. The resulting white solid was collected by filtration and washed with ethyl ether. The crude product was dissolved in water (20 mL) and the solution was brought to reflux for 2 hours. The reaction was cooled and the resulting solid was collected by filtration to afford 13.5 g (80% yield) of the desired product as a light yellow solid. M.p. 218-219 °C.

Preparation of 1-(2-hydroxy-4,6-dimethoxyphenyl)ethanone (5): 1-(2,4,6-trihydroxyphenyl)ethanone, **4,** (16.8 g, 0.10 mol), anhydrous potassium carbonate (41.50 g, 0.30 mol) and anhydrous acetone (168 mL) were combined and stirred at room temperature after which dimethyl sulfate (26.9 g, 0.21 mol) was slowly added. Once the addition was complete, the reaction was stirred for 1 hour. The pH of the solution was adjusted to neutral using 2N HCl. The resulting yellow solid was collected by filtration and washed with water then dried to afford 16 g (82% yield) of the desired product as a white solid. M.p. 76-78 °C.

Preparation of (E)-1-(2-hydroxy-4,6-dimethoxyphenyl)-3-(4-methylphenyl)prop-2-en-1-one (6): KOH (10.0 g, 0.18 mol) was dissolved in the mixture of ethanol (80 mL) and water (20 mL) followed by the addition of 1-(2-hydroxy-4,6-dimethoxyphenyl)ethanone, **5,** (4.0 g, 0.02 mol) and 4-methylphenyl aldehyde (3.6 mL, 0.03 mol). The solution was stirred at room temperature for 3 hours. The pH was then adjusted to 8-9 with 2N HCl and the resulting solid was collected by filtration and washed with water until the pH of the washings tested neutral. The solid was dried to afford 5.5 g (92% yield) of the desired product as a yellow powder that was used for the next step without further characterization.

Preparation of (E)-1-(2-hydroxy-4,6-dimethoxy-3-(piperidin-1-ylmethyl)phenyl)-3-(4-methylphenyl)-2-propylene-1-one (7): To a reaction flack was added (E)-1-(2-hydroxy-4,6-dimethoxyphenyl)-3-(4-methylphenyl)prop-2-en-1-one, **6,** (1.6 g, 5.0 mmol), paraformaldehyde (0.5 g, 15.0 mmol), piperidine (1.1 mL, 11 mmol) and formic acid (20 mL) Several drops of HCl were added. The solution was then brought to reflux for 2 hours after which the solvent was removed under reduced pressure. Water was then added followed by 5% NaOH. The resulting aqueous solution was extracted with methylene chloride (40 mL × 3). The organic layers were combined and washed with saturated sodium chloride (30 mL × 3). The organic layer was dried with anhydrous sodium sulfate and filtered. After evaporation of the solvent, anhydrous ethyl ether was and the resulting solid was collected to afford 1.2 g (63% yield) of the desired product as a yellow powder. M.p. 127-128 °C; ESI-MS: m/z[M+1]$^+$ 396.4; $^1$H NMR ($CDCl_3$): δ 7.6-7.9 (2H, dd), 7.4 (2H, m), 7.2 (2H, m), 6.0 (1H, s), 3.9 (6H, s), 3.7 (2H, s), 2.6 (4H, m), 2.4 (3H, s), 1.6 (4H, m), 1.5 (2H, m); IR (KBr) 3118, 3010, 2927, 2844, 1627, 1585, 1322, 1223, 1123, 983, 894, 814, 790, 730 cm$^{-1}$.

The compounds encompassed within the second category of the disclosed antitumor agents can be prepared by substituting morpholine, substituted morpholines, pyrrolidine, substituted pyrrolidines, piperazine, substituted piperazines, and the like for piperidine in the procedure outlined in Scheme II and disclosed in Example 2 herein above. The following are non-limiting examples of compounds encompassed within the second category of the disclosed antitumor agents.

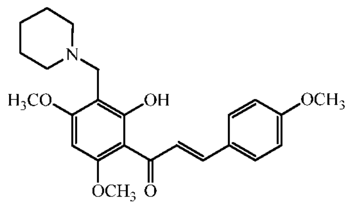

(E)-1-(2-hydroxy-4,6-dimethoxy-3-(piperidin-1-ylmethyl)phenyl)-3-(4-methoxyphenyl)-2-propylene-1-one (C2). M. p. 129-130 °C; ESI-MS:m/z[M+1]$^+$ 413.2; $^1$H NMR ($CDCl_3$) δ 7.80-7.84 (1H, d, J=15.6Hz), 7.76-7.80 (1H, d, J=15.6Hz), 7.51-7.53 (2H, m), 6.89-6.91 (2H, m), 6.02 (1H, s), 3.88 (9H, s), 3.68 (2H, s), 2.52 (4H, m), 1.59 (4H, m), 1.44 (2H, m); IR (KBr) 3115, 3003, 2932, 2840, 1623, 1555, 1509, 1325, 1220, 1124, 986, 830, 790 cm$^{-1}$.

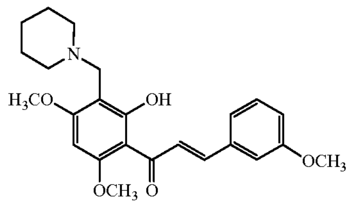

(E)-1-(2-hydroxy-4,6-dimethoxy-3-(piperidin-1-ylmethyl)phenyl)-3-(3-methoxyphenyl)-2-propylene-1-one (C3). M. p. 130-131 °C; ESI-MS:m/z[M+1]$^+$ 417.9; $^1$H NMR ($CDCl_3$) δ 7.83-7.86 (1H, d, J=15.5Hz), 7.79-7.82 (1H, d, J=15.5Hz), 7.42 (1H, m), 7.52 (1H, m), 7.32 (1H, m), 7.39 (1H, m), 6.00 (1H, s), 3.91 (6H,s),3.69 (2H, s), 2.42-2.61 (4H, m), 1.56-1.61 (4H, m), 1.37-1.45 (2H, m); IR (KBr) 3003, 2934, 1674, 1599, 1321, 1225, 1125, 1097, 986, 821, 784 cm$^{-1}$.

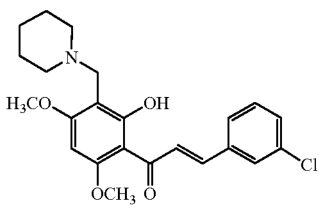

(E)-3 -(3 -chlorophenyl)-1-(2-hydroxy-4,6-dimethoxy-3-(piperidin-1-ylmethyl)phenyl-2-propylene-1-one (C4). M. p. 134-135 °C; ESI-MS: m/z[M+1]$^+$ 416.6; $^1$H NMR($CDCl_3$) δ 7.85-7.89 (1H, d, J=15.2Hz), 7.66-7.70 (1H, d, J=15.2Hz), 7.50 (1H, m), 7.31-7.35 (3H, m), 6.10 (1H, s), 5.40 (1H, s), 3.91 (3H, s), 3.84 (3H, s), 2.78-2.83 (4H, m), 1.55 (6H, m); IR (KBr) 3117, 3055, 2961, 1658, 1617, 1570, 1342, 1215, 1108, 811, 793 cm$^{-1}$.

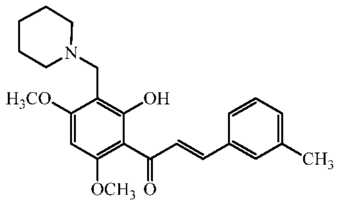

(E)-3-(3-methylphenyl)-1-(2-hydroxy-4,6-dimethoxy-3-(piperidin-1-ylmethyl)-phenyl-2-propylene-1-one (C5). M.p. 113-114 °C; ESI-MS:m/z[M+1]$^+$ 397.7; $^1$H NMR ($CDCl_3$) δ 7.51-7.55 (1H, d, J=15.9Hz), 7.36-7.40 (1H, d, J=15.9Hz), 7.35 (2H, m), 7.29 (1H, m), 7.17 (1H, m), 6.01 (1H, s), 5.24 (1H, s), 3.91 (6H, s), 3.68 (2H, s), 2.52 (4H, m), 2.36 (3H, s), 1.57-1.61 (4H, m), 1.44 (2H, m); IR (KBr) 3017, 2930, 1629, 1558, 1323, 1241, 1221, 1125, 981, 796 $cm^{-1}$.

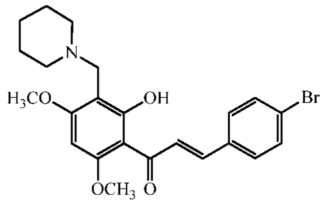

(E)-3-(4-bromophenyl)-1-(2-hydroxy-4,6-dimethoxy-3-(piperidin-1-ylmethyl)-phenyl-2-propylene-1-one (C6). M.p. 99-100 °C; ESI-MS:m/z[M+1]$^+$ 462.4; $^1$H NMR($CDCl_3$) δ 7.70-7.74 (1H, d, J=15.5Hz), 7.50-7.54 (1H, d, J=15.5Hz), 7.47 (1H, m), 7.52 (1H, m), 7.38 (1H, m), 7.40 (1H, m), 6.00 (1H, s), 3.91 (6H, s), 3.72 (2H, s), 2.42-2.56 (4H, m), 1.56-1.63 (6H, m); IR (KBr) 2933, 1674, 1600, 1618, 1321, 1223, 1125, 986, 818 $cm^{-1}$.

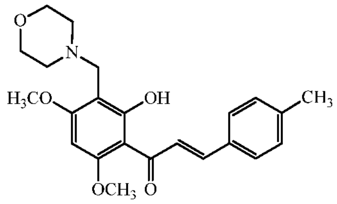

(E)-1-(2-hydroxy-4,6-dimethoxy-3-(morpholin-4-ylmethyl)phenyl)-3-(4-methylphenyl)-2-propylene-1-one (D1). M. p. 126-127 °C; ESI-MS:m/z[M+1]$^+$ 398.2; $^1$H NMR ($CDCl_3$) δ 7.66-7.70 (1H, d, J=15.7Hz), 7.56-7.60 (1H, d, J=15.7Hz), 7.47-7.49 (2H, d, J=8.04Hz), 7.19-7.21 (2H, d, J=7.92Hz), 6.02 (1H, s), 3.92 (3H, s), 3.89 (3H, s), 3.70-3.72 (4H, m), 3.68 (2H, s), 2.56 (4H, m), 2.38 (3H, s); IR (KBr) 3105, 2974, 2851, 1626, 1556, 1511, 1321, 1114, 977, 865, 814, 792 $cm^{-1}$.

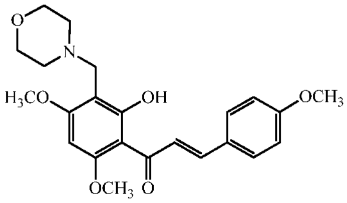

(E)-1-(2-hydroxy-4,6-dimethoxy-3-(morpholin-4-ylmethyl)phenyl)-3-(4-methoxyphenyl)-2-propylene-1-one (D2). M. p. 159-160 °C; ESI-MS:m/z[M+1]$^+$ 414.4; $^1$H NMR ($CDCl_3$) δ 7.66-7.70 (1H, d, J=15.6Hz), 7.54-7.55 (2H, d, J=6.88Hz), 7.50-7.53 (1H, d, J=15.6Hz), 6.91-6.93 (2H, d, J=8.76Hz), 6.02 (1H, s), 3.92 (3H, s), 3.88 (3H, s), 3.84 (3H, s), 3.70 (4H, m), 3.66 (2H, s), 2.56 (4H, m); IR (KBr) 3121, 2967, 2838, 1737, 1624, 1557, 1509, 1325, 1257, 1114, 99, 829, 794 $cm^{-1}$.

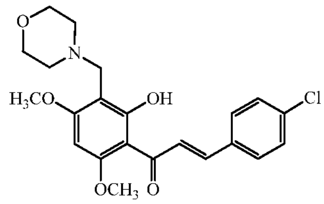

(E)-3-(4-chlorophenyl)-1-(2-hydroxy-4,6-dimethoxy-3-(morpholin-4-ylmethyl)phenyl)-2-propylene-1-one (D3). M. p. 140-141 °C; ESI-MS:m/z[M+1]$^+$ 420.0; $^1$H NMR ($CDCl_3$) δ 7.65 (1H, d, J=15.6Hz), 7.63 (1H, d, J=15.6Hz), 7.52-7.54 (2H, d, J=8.44Hz), 7.38-7.40 (2H, d, J=8.44Hz), 6.04 (1H, s), 3.93 (6H, s), 3.75 (4H, m), 3.73 (2H, s), 2.60 (4H, m); IR (KBr) 3123, 3011, 2914, 1736, 1675, 1604, 1399, 1322, 1223, 1109, 912, 822 cm$^{-1}$.

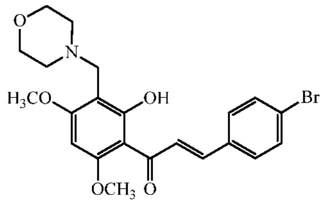

(E)-3-(4-bromophenyl)-1-(2-hydroxy-4,6-dimethoxy-3-(morpholin-4-ylmethyl)phenyl)-2-propylene-1-one (D4). M. p. 144-145 °C; ESI-MS:m/z[M+1]$^+$ 462.4; $^1$H NMR ($CDCl_3$) δ 7.61 (2H,d,J=15.6Hz),7.51-7.54 (2H,d,J=8.44Hz),7.43-7.45 (2H, d, J=8.52Hz), 6.02 (1H, s), 3.92 (3H, s), 3.90 (3H, s), 3.71 (4H, m), 3.66-3.70 (2H, s), 2.56-2.57 (4H, m); IR (KBr) 2949, 2917, 1679, 1604, 1576, 1399, 1323, 1224, 1110, 864, 819, 772 cm$^{-1}$.

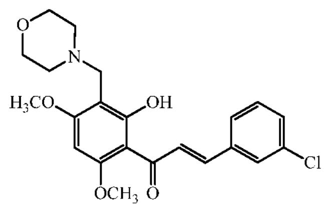

(E)-3-(3-chlorophenyl)-1-(2-hydroxy-4,6-dimethoxy-3-(morpholinemethyl)phenyl)-2-propylene-1-one (D5). M. p. 135-136 °C; ESI-MS:m/z[M+1]$^+$ 418.4; $^1$H NMR ($CDCl_3$) δ 7.56-7.59 (2H, d, J=15.6Hz), 7.56 (1H, m), 7.43 (1H, m), 7.33 (2H, m), 6.02 (1H, s), 3.92 (3H, s), 3.89 (3H, s), 3.70-3.72 (4H, m), 3.67 (2H, s), 2.56 (4H, m); IR (KBr) 3114, 2943, 1625, 1561, 1319, 1229, 1139, 981, 864, 788, 684 cm$^{-1}$.

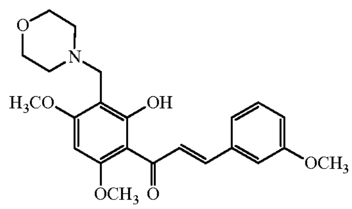

(E)-3-(3 -methoxyphenyl)-1-(2-hydroxy-4,6-dimethoxy-3-(morpholinemethyl)-phenyl)-2-propylene-1-one (D6). M. p. 169-170 °C; ESI-MS:m/z[M+1]$^+$ 414.4; $^1$H NMR ($CDCl_3$) δ 7.63-7.67 (1H, d, J=15.7Hz), 7.56-7.60 (1H, d, J=15.7Hz), 7.32 (1H, dd, $J_1$=7.84Hz, $J_2$=7.96Hz), 7.18 (1H, d, J=7.64Hz), 7.10 (1H, m), 6,93 (1H, ddd, $J_1$=8.04Hz, $J_2$=2.24Hz, $J_3$=2.12Hz) 6.02 (1H, s), 3.91 (9H, s), 3.70-3.72 (4H, m), 3.66 (2H, s), 2.56 (4H, m); IR (KBr) 3006, 2936, 1660, 1618, 1570, 1266, 1214, 1157, 1109, 813, 793 cm$^{-1}$.

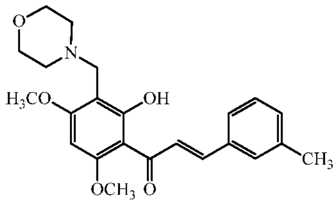

(E)-3-(3-methylphenyl)-1-(2-hydroxy-4,6-dimethoxy-3-(morpholinemethyl)-phenyl)-2-propylene-1-one (D7). M.p. 158-159 °C; $^1H$ NMR ($CDCl_3$) $\delta$ 7.90 (1H, d, J=15.7Hz), 7.78 (1H, d, J=15.7Hz), 7.31 (1H, m), 7.20 (1H, m), 6.91 (1H, m), 6,74 (1H, m), 6.00 (1H, s), 3.90 (6H, s), 3.67 (4H, m), 3.63 (2H, s), 2.60 (3H, s), 2.47 (4H, m); IR (KBr) 2959, 2943, 1612, 1559, 1345, 1209, 1113, 981, 796 $cm^{-1}$.

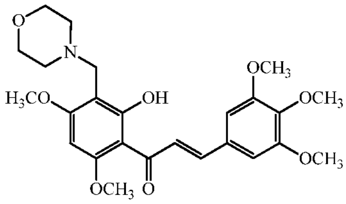

(E)-3-(3,4,5-trimethoxyphenyl)-1-(2-hydroxy-4,6-dimethoxy-3-(morpholinemethyl)-phenyl)-2-propylene-1-one (D8). M.p. 169-170 °C; ESI-MS:m/z[M+1]$^+$ 474.4; $^1H$ NMR ($CDCl_3$) $\delta$ 7.58-7.62 (1H, d, J =15.6Hz), 7.50-7.54 (1H, d, J=15.6Hz), 6.82 (2H, s), 6.03 (1H, s), 3.90 (15H, s), 3.72 (4H, m), 3.71 (2H, s), 2.58 (4H, m); IR (KBr) 3123, 2975, 2841, 1738, 1627, 1583, 1503, 1318, 1280, 1243, 1119, 861, 824, 767 $cm^{-1}$.

A second embodiment of the second category of the disclosed antitumor agents has the formula:

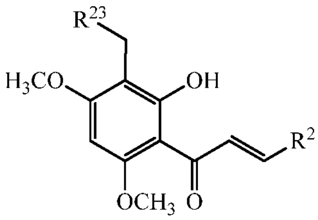

wherein $R^{23}$ is a $C_1$-$C_5$ heterocyclic ring and the index y is equal to 1. For this embodiment, $R^2$ units comprise substituted or unsubstituted $C_1$-$C_5$ heteroaryl rings, non-limiting examples of which are further disclosed herein below in Table V.

TABLE V

| No. | $R^2$ | $R^{23}$ | No. | $R^2$ | $R^{23}$ |
|---|---|---|---|---|---|
| E31 | furan-2-yl | piperidin1-yl | E45 | pyrrol-2-yl | piperidin1-yl |
| E32 | furan-2-yl | morpholin-4-yl | E46 | pyrrol-2-yl | morpholin-4-yl |
| E33 | furan-3-yl | piperidin1-yl | E47 | pyrrol-3-yl | piperidin1-yl |
| E34 | furan-3-yl | morpholin-4-yl | E48 | pyrrol-3-yl | morpholin-4-yl |
| E35 | [1,2,3]triazol-4-yl | piperidin1-yl | E49 | oxazol-2-yl | piperidin1-yl |
| E36 | [1,2,3]triazol-4-yl | morpholin-4-yl | E50 | oxazol-2-yl | morpholin-4-yl |
| E37 | [1,2,3]triazol-5-yl | piperidin1-yl | E51 | oxazol-4-yl | piperidin1-yl |
| E38 | [1,2,3]triazol-5-yl | morpholin-4-yl | E52 | oxazol-4-yl | morpholin-4-yl |
| E39 | [1,2,4]triazol-5-yl | piperidin1-yl | E53 | oxazol-5-yl | piperidin1-yl |
| E40 | [1,2,4]triazol-5-yl | morpholin-4-yl | E54 | oxazol-5-yl | morpholin-4-yl |
| E41 | imidazol-2-yl | piperidin1-yl | E55 | thiazol-2-yl | piperidin1-yl |

(continued)

| No. | $R^2$ | $R^{23}$ | No. | $R^2$ | $R^{23}$ |
|---|---|---|---|---|---|
| E42 | imidazol-2-yl | morpholin-4-yl | E56 | thiazol-2-yl | morpholin-4-yl |
| E43 | imidazol-4-yl | piperidin1-yl | E57 | thiazol-4-yl | piperidin1-yl |
| E44 | imidazol-4-yl | morpholin-4-yl | E58 | thiazol-4-yl | morpholin-4-yl |

The compounds encompassed within the second embodiment of the second category of the disclosed antitumor agents can be prepared by substituting $C_1$-$C_5$ heteroaryl carboxaldehydes for the substituted or unsubstituted phenyl aldehydes in the procedure outlined in Scheme I and disclosed in Example 1 herein above. Non-limiting examples, include pyroll-2-carbaldehyde [CAS No. 1003-29-8], imidazol-2-carbaldehyde [CAS No. 10111-08-7], oxazol-2-carbaldehyde [CAS No. 65373-52-6], [1,2,3]triazol-4-carbaldehyde [CAS No. 32829-25-7], thiazol-4-carbaldehyde [CAS No. 3364-80-5] and thiophen-2-carbaldehyde [CAS No. 98-03-3]. The following are non-limiting examples of compounds encompassed within the second embodiment of the first category of the disclosed antitumor agents.

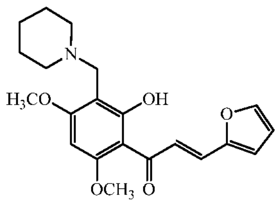

(E)-3-(furan-2-yl)-1-(2-hydroxy-4,6-dimethoxy-3-(piperidine-1-ylmethyl)phenyl)-2-propylene-1-one (E31). M. p. 95-96 °C; ESI-MS:m/z[M+1]$^+$ 370.4; $^1$H NMR ($CDCl_3$) $\delta$ 8.98 (1H, s), 8.59-8.63 (1H, d, J=15.6Hz), 8.49-8.53 (1H, d, J=15.6Hz), 8.12 (1H, m), 7.87 (1H, m), 7.54 (1H, m), 5.18 (6H, s), 4.88 (2H, s), 3.75 (4H, m), 2.80 (4H, m), 2.72 (2H. m); IR (KBr) 3119, 2933, 1617, 1548, 1416, 1314, 1276, 1231, 1123, 969, 880, 860, 783, 745 cm$^{-1}$.

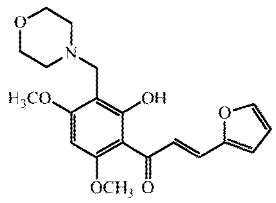

(E)-3-(furan-2-yl)-1-(2-hydroxy-4,6-dimethoxy-3-(morpholin-4-ylmethyl)phenyl)-2-propylene-1-one (E32). M. p. 147-148 °C; ESI-MS:m/z[M+1]$^+$ 374.4; $^1$H NMR ($CDCl_3$) 7.68 (1H, m), 7.64-7.68 (1H,d, J=15.5Hz), 7.50-7.54 (1H, d, J=15.5Hz), 6.81 (1H, m), 6.59 (1H, m), 6.27 (1H, s), 3.99 (6H, s), 3.67-3.70 (4H, m), 3.61 (2H, s), 2.56-2.62 (4H, m); IR (KBr) 3092, 2969, 1737, 1613, 1414, 1316, 1224, 1110, 885, 744 cm$^{-1}$.

**PROCEDURES**

**Preliminary Cytotoxicity Screening**

Human colon cancer cell line HCT116 can be used for the preliminary cytotoxicity screening of the disclosed compounds as disclosed by Mosmann, T., "Rapid colorimetric assay for cellular growth and survival: application to proliferation and cytotoxicity assays." J. Immunol. Methods, (1983) 65(1-2): p. 55-63 referred to herein further as the "MTT test".

**MTT Test**

The disclosure of Mosmann recites the following. "MTT 93-(4,5-dimethylthirazol-2-yl)-2,5-diphenyl tetrazolium bromide; Sigman catalog no. M2128) was dissolved in PBS at 5 mg/ml and filtered to sterilize and remove a small amount of insoluble residue present in some batches of MTT. At the times indicated below, stock MTT solution (10ml per 100 ml) was added to all wells of an assay, and plates were incubated at 37°C for 4 h. Acid-siopropanol (100 ml of 0.04 N

HCl in isopropanol) was added to all wells and mixed thoroughly to dissolve the dark blue crystals. After a few minutes at room temperature to ensure that all crystals were dissolved, the plates were read o a Dynatech MR580 Microelisa reader, using a test wavelength of 570 nm, a reference wavelength of 630 nm, and a calibration setting of 1.99 (or 1.0 if the samples were strongly colored). Plates were normally read within 1 h of adding the isopropanol.

"Our final procedure was to add 0.01 ml MTT (5 mg/ml in phosphate-buffered saline) to 0.1 ml cells in growth medium. After 4 h at 37°C for MTT cleavage, the formazan product was solublilized by the addition of 0.1 ml 0.04 N HCl in isopropanol. Optical density was measured on a Dynatech MR 580 plate reader, using a reference wavelength of 630 nm and a test wavelength of 570 nm."

3-(4,5-Dimethylthiazol-2-yl)-2,5-diphenyltetrazolium bromide, a tetrazole, can pass through cellular membrane to enter into cells. This tetrazole may be reduced to the purple formazan in the mitochondria of living cells, which can diffuse out to form crystals. This crystal can be dissolved in the dimethyl sulfoxide to form a colored solution. The absorbance of this colored solution can be quantified by measuring at certain wavelength (490 nm) using a spectrophotometer. The quantity of the formazan crystal is proportional to the number of living cells.

As used herein, cells were cultured in McCoy's 5A medium supplemented with 10% heat-inactivated Fetal Bovine Serum (FBS) and 5% Penicillin/Streptomycin solution. McCoy's 5A, 1X (Iwakata & Grace modified) medium with L-Glutamine, DPBS, 1X w/o Ca and Mg, Penicillin/Streptomycin solution, Trypsin EDTA, 1X and PBS, 1X w/o Ca and Mg were purchased from Mediatech, Inc. (USA). FBS was purchased from Hyclone (USA).

The cytotoxicity of the compounds was estimated using the MTT assay. A 96-well plate was seeded with 100 μl medium containing HCT116 cells in suspension at a density of $2.5x10^4$ cells/ml [10]. After 24 hours of incubation, the cells were treated with the compounds, which were dissolved in DMSO (dimethyl sulfoxide) as stock solutions and diluted to final concentrations of 100 μM, 50 μM, 25 μM, 12.5 μM, 6.25 μM & 1 μM (for compounds tested in the micromolar concentrations) and 10 μM, 1 μM, 500 nM, 250 nM, 125 nM & 62.5 nM (for compounds tested in the nanomolar concentration range) using medium containing 0.1% DMSO. The final concentration of DMSO in the culture medium was maintained at 0.1% - 0.001% (v/v). After 48 hrs incubation in the presence of test compounds, the spent medium was removed and the wells were washed twice with 100 μL of PBS solution. 100 μL of fresh medium and 10 μL of MTT (5 mg/ml in PBS) was added to the wells and incubated at 37°C in dark for 4 hrs. The formazan product was dissolved by adding 100 μL of 100% DMSO after removing the medium from each well and was stabilized by adding 12.5 μL of Sorensen's glycine buffer to each well. After completely dissolving the formazan crystals, the absorbance was measured at 490nm using a Wallace 1420 multi plate reader (PerkinElmer Inc., Massachusetts, USA). The level of absorbance relates to the number of viable cells. Doxorubicin hydrochloride was used as the standard.

**Primary screening**

Compounds can be tested for their level of cytotoxicity as part of an initial screening. For example, samples of the disclosed compound were applied to the cells at 100 μM and 10 μM concentrations in two 96 well plates. The cytotoxicity of the compounds was estimated based on the absorbance values measured at 490nm.

**TABL IX**

| No. | Compound | $IC_{50}$ (μmol/L) |
|---|---|---|
| A1 | 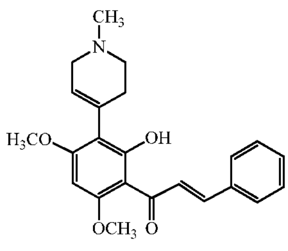 (E)-1-(2-Hydroxy-4,6-dimethoxy-3-(1-methyl-1,2,3,6-tetrahydropyridine-4-yl)phenyl)-3 -phenylprop-2-en-1-one | 10.07 |

(continued)

| No. | Compound | $IC_{50}$ (μmol/L) |
|---|---|---|
| A2 | 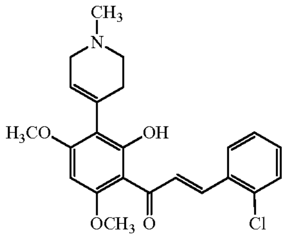 (E)-3-(2-chlorophenyl)-1-(2-hydroxy-4,6-dimethoxy-3-(1-methyl-1,2,3,6-tetrahydropyridine-4-yl)phenyl)-2-propylene-1-one | 28.28 |
| A3 | 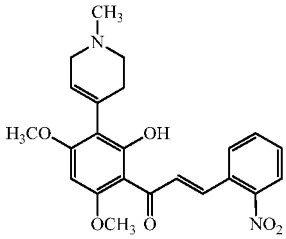 (E)-1-(2-hydroxy-4,6-dimethoxy-3-(1-methyl-1,2,3,6-tetrahydropyridine-4-yl)phenyl)-3-(2-nitrophenyl)-2-propylene-1-one | 17.95 |
| A4 | 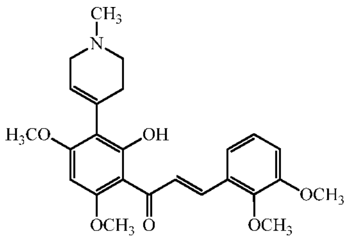 (E)-3-(2,3-dimethoxyphenyl)-1-(2-hydroxy-4,6-dimethoxy-3-(1-methyl-1,2,3,6-tetrahydropyridine-4-yl)phenyl)-2-propylene-1-one | 5.22 |
| A5 | 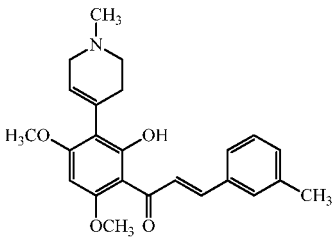 (E)-1-(2-hydroxy-4,6-dimethoxy-3-(1-methyl-1,2,3,6-tetrahydropyridine-4-yl)phenyl)-3-(3-methylphenyl)-2-propylene-1-one | 8.89 |
| A6 | 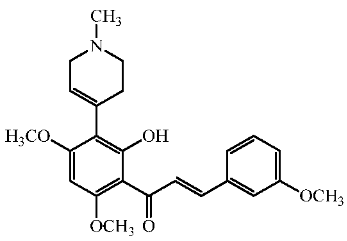 (E)-1-(2-hydroxy-4,6-dimethoxy-3-(1-methyl-1,2,3,6-tetrahydropyridine-4-yl)phenyl)-3-(3-methoxyphenyl)-2-propylene-1-one | 6.78 |

(continued)

| No. | Compound | $IC_{50}$ (μmol/L) |
|---|---|---|
| A7 | 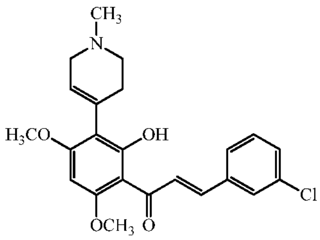 (E)-3-(3-chlorophenyl)-1-(2-hydroxy-4,6-dimethoxy-3-(1-methyl-1,2,3,6-tetrahydropyridine-4-yl)phenyl)-2-propylene-1-one | 3.24 |
| A8 | 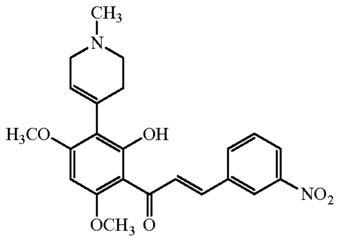 (E)-1-(2-hydroxy-4,6-dimethoxy-3-(1-methyl-1,2,3,6-tetrahydropyridine-4-yl)phenyl)-3-(3-nitrophenyl)-2-propylene-1-one | 10.47 |
| A9 | 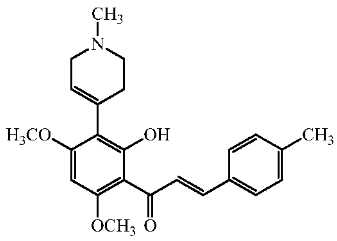 (E)-1-(2-hydroxy-4,6-dimethoxy-3-(1-methyl-1,2,3,6-tetrahydropyridine-4-yl)phenyl)-3-(4-methylphenyl)-2-propylene-1-one | 3.87 |
| A10 | 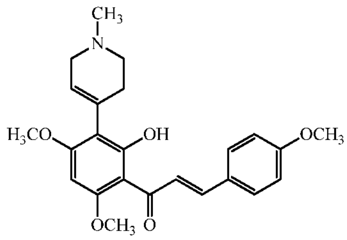 (E)-1-(2-hydroxy-4,6-dimethoxy-3-(1-methyl-1,2,3,6-tetrahydropyridine-4-yl)phenyl)-3-(4-methoxyphenyl)-2-propylene-1-one | 11.8 |
| A11 | 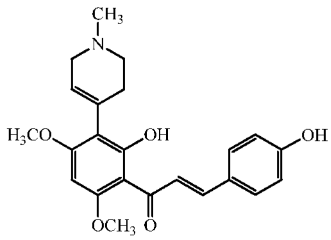 (E)-1-(2-hydroxy-4,6-dimethoxy-3-(1-methyl-1,2,3,6-tetrahydropyridine-4-yl)phenyl)-3-(4-hydroxyphenyl)-2-propylene-1-one | 88.15 |

(continued)

| No. | Compound | $IC_{50}$ (μmol/L) |
|---|---|---|
| A12 | 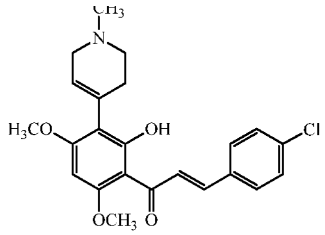 (E)-3-(4-chlorophenyl)-1-(2-hydroxy-4,6-dimethoxy-3-(1-methyl-1,2,3,6-tetrahydropyridine-4-yl)phenyl)-2-propylene-1-one | 8.3 |
| A13 | 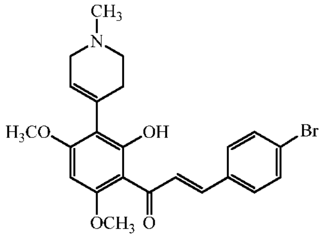 (E)-3-(3-bromophenyl)-1-(2-hydroxy-4,6-dimethoxy-3-(1-methyl-1,2,3,6-tetrahydropyridine-4-yl)phenyl)-2-propylene-1-one | 8.76 |
| A14 | 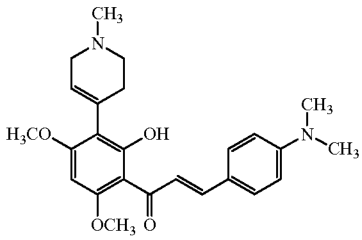 (E)-3-(4-dimethylaminophenyl)-1-(2-hydroxy-4,6-dimethoxy-3-(1-methyl-1,2,3,6-tetrahydropyridine-4-yl)phenyl)-2-propylene-1-one | 30.54 |
| A15 | 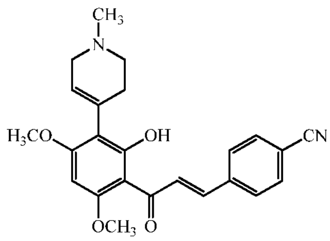 (E)-3-(4-cyanophenyl)-1-(2-hydroxy-4,6-dimethoxy-3-(1-methyl-1,2,3,6-tetrahydropyridine-4-yl)phenyl)-2-propylene-1-one | 13.27 |
| A16 | 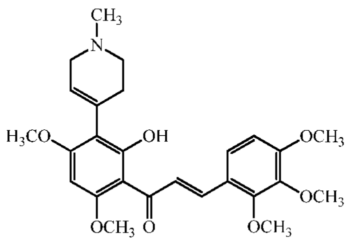 (E)-1-(2-hydroxy-4,6-dimethoxy-3-(1-methyl-1,2,3,6-tetrahydropyridine-4-yl)phenyl)-3-(2,3-4-trimethoxyphenyl)-2-propylene-1-one | 3.31 |

(continued)

| No. | Compound | $IC_{50}$ (μmol/L) |
|---|---|---|
| A17 | 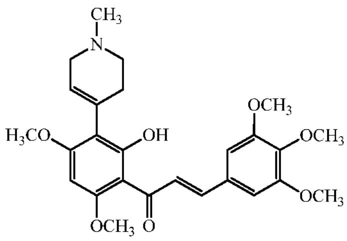 (E)-1-(2-hydroxy-4,6-dimethoxy-3-(1-methyl-1,2,3,6-tetrahydropyridine-4-yl)phenyl)-3-(3,4,5-trimethoxyphenyl)-2-propylene-1-one | 18.41 |
| B1 | 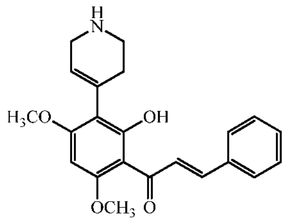 1-(2-hydroxy-4,6-dimethoxy-3-(1,2,3,6-tetrahydropyridine-4-yl)phenyl)-3-phenyl-2-propylene-1-one | 8.41 |
| B2 | 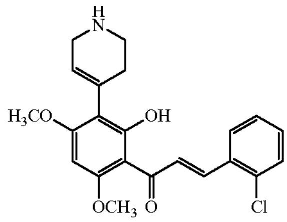 (E)-3-(2-chlorophenyl)-1-(2-hydroxy-4,6-dimethoxy-3-(1,2,3,6-tetrahydropyridine-4-yl)phenyl)-2-propylene-1-one | 0.1 |
| B3 | 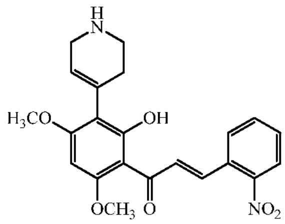 (E)-1-(2-hydroxy-4,6-dimethoxy-3-(1,2,3,6-tetrahydropyridine-4-yl)phenyl)-3-(2-nitrophenyl)-2-propylene-1-one | 5.68 |
| B4 | 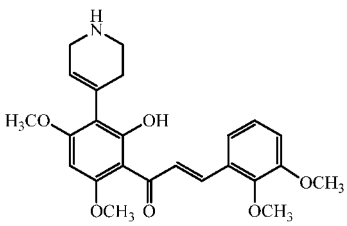 (E)-3-(2,3-dimethoxyphenyl)-1-(2-hydroxy-4,6-dimethoxy-3-(1,2,3,6-tetrahydropyridine-4-yl) phenyl)-2-propylene-1-one | 4.47 |

(continued)

| No. | Compound | $IC_{50}$ (μmol/L) |
|---|---|---|
| B5 | 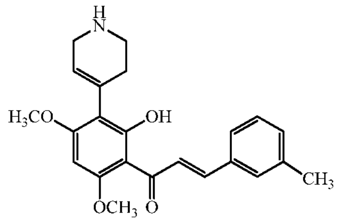 (E)-1-(2-hydroxy-4,6-dimethoxy-3-(1,2,3,6-tetrahydropyridine-4-yl)phenyl)-3-(3-methylphenyl)-2-propylene-1-one | 6.13 |
| B6 | 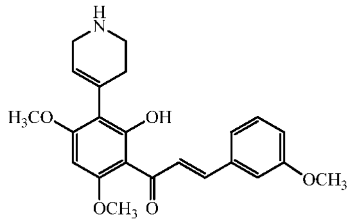 (E)-1-(2-hydroxy-4,6-dimethoxy-3-(1,2,3,6-tetrahydropyridine-4-yl)phenyl)-3-(3-methoxyphenyl)-2-propylene-1-one | 3.08 |
| B7 | 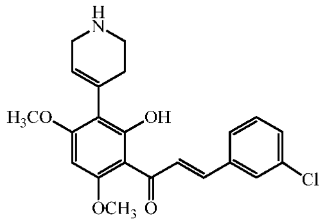 (E)-3-(3-chlorophenyl)-1-(2-hydroxy-4,6-dimethoxy-3-(1,2,3,6-tetrahydropyridine-4-yl)phenyl)-2-propylene-1-one | 9.97 |
| B8 | 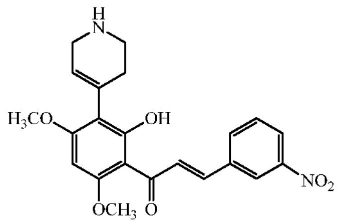 (E)-1-(2-hydroxy-4,6-dimethoxy-3-(1,2,3,6-tetrahydropyridine-4-yl)phenyl)-3-(3-nitrophenyl)-2-propylene-1-one | 4.1 |
| B9 | 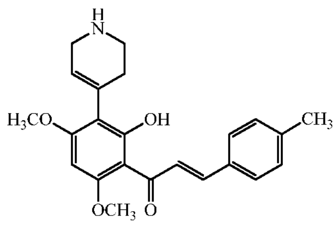 (E)-1-(2-hydroxy-4,6-dimethoxy-3-(1,2,3,6-tetrahydropyridine-4-yl)phenyl)-3-(4-methylphenyl)-2-propylene-1-one | 4.02 |

(continued)

| No. | Compound | $IC_{50}$ (μmol/L) |
|---|---|---|
| B10 | 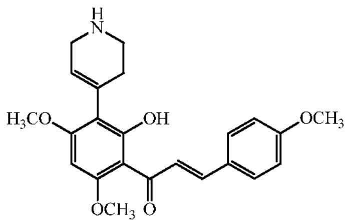 (E)-1-(2-hydroxy-4,6-dimethoxy-3-(1,2,3,6-tetrahydropyridine-4-yl)phenyl)-3-(4-methoxyphenyl)-2-propylene-1-one | 11.36 |
| B11 | 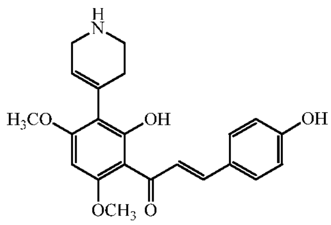 (E)-1-(2-hydroxy-4,6-dimethoxy-3-(1,2,3,6-tetrahydropyridine-4-yl)phenyl)-3-(4-hydroxyphenyl)-2-propylene-1-one | 4.03 |
| B12 | 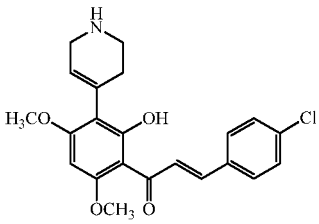 (E)-3-(4-chlorophenyl)-1-(2-hydroxy-4,6-dimethoxy-3-(1,2,3,6-tetrahydropyridine-4-yl)phenyl)-2-propylene-1-one | 4.82 |
| B13 | 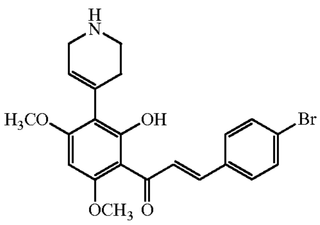 (E)-3-(4-bromophenyl)-1-(2-hydroxy-4,6-dimethoxy-3-(1,2,3,6-tetrahydropyridine-4-yl)phenyl)-2-propylene-1-one | 18.17 |
| B14 | 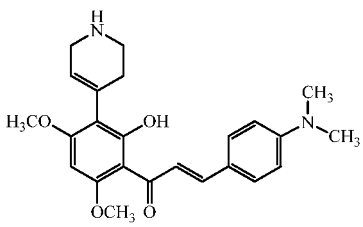 (E)-3-(4-dimethylaminophenyl)-1-(2-hydroxy-4,6-dimethoxy-3-(1,2,3,6-tetrahydropyridine-4-yl)phenyl)-2-propylene-1-one | 32.0 |

(continued)

| No. | Compound | $IC_{50}$ (μmol/L) |
|---|---|---|
| B15 | 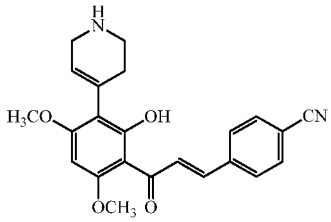 (E)-3-(4-cyanophenyl)-1-(2-hydroxy-4,6-dimethoxy-3-(1,2,3,6-tetrahydropyridine-4-yl)phenyl)-2-propylene-1-one | 7.3 |
| B16 | 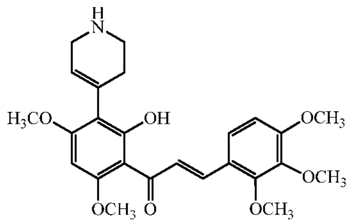 (E)-1-(2-hydroxy-4,6-dimethoxy-3-(1,2,3,6-tetrahydropyridine-4-yl)phenyl)-3-(2,3,4-trimethoxyphenyl)-2-propylene-1-one | 5.54 |
| B17 | 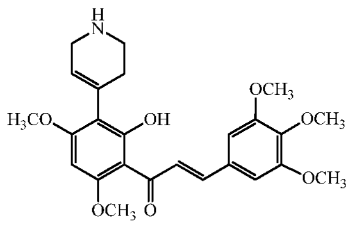 (E)-1-(2-hydroxy-4,6-dimethoxy-3-(1,2,3,6-tetrahydropyridine-4-yl)phenyl)-3-(3,4,5-trimethoxyphenyl)-2-propylene-1-one | 4.2 |
| E1 | 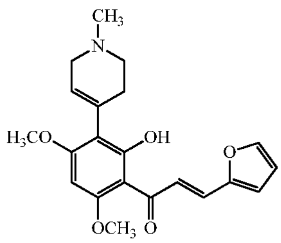 (E)-3-(furan-2-yl)-1-(2-hydroxy-4,6-dimethoxy-3-(1-methyl-1,2,3,6-tetrahydro-pyridine-4-yl) phenyl)-2-propylene-1-one | 10.17 |
| E2 | 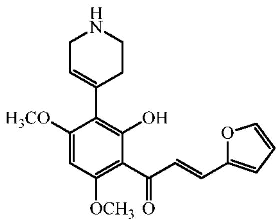 (E)-3-(furan-2-yl)-1-(2-hydroxy-4,6-dimethoxy-3-(1,2,3,6-tetrahydropyridine-4-yl)phenyl)-2-propylene-1-one | 11.05 |

(continued)

| No. | Compound | $IC_{50}$ (μmol/L) |
|---|---|---|
| C1 | 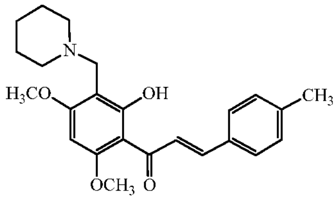 (E)-1-(2-hydroxy-4,6-dimethoxy-3-(piperidin-1-ylmethyl)phenyl)-3-(4-methylphenyl)-2-propylene-1-one | 5.57 |
| C2 | 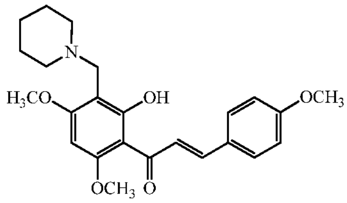 (E)-1-(2-hydroxy-4,6-dimethoxy-3-(piperidin-1-ylmethyl)phenyl)-3-(4-methoxyphenyl)-2-propylene-1-one | 5.46 |
| C3 | 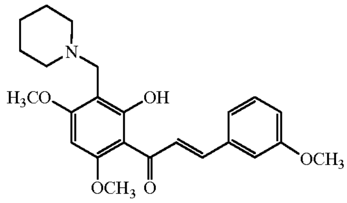 (E)-1-(2-hydroxy-4,6-dimethoxy-3-(piperidin-1-ylmethyl)phenyl)-3-(3-methoxyphenyl)-2-propylene-1-one | 2.85 |
| C4 | 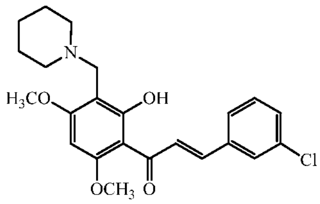 (E)-3-(3-chlorophenyl)-1-(2-hydroxy-4,6-dimethoxy-3-(piperidin-1-ylmethyl)phenyl-2-propylene-1-one | 3.87 |
| D1 | 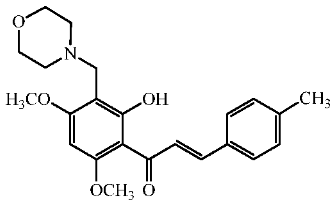 (E)-1-(2-hydroxy-4,6-dimethoxy-3-(morpholin-4-ylmethyl)phenyl)-3-(4-methylphenyl)-2-propylene-1-one | 14.82 |

(continued)

| No. | Compound | IC50 (μmol/L) |
|---|---|---|
| D2 | 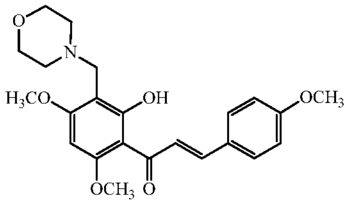<br>(E)-1-(2-hydroxy-4,6-dimethoxy-3-(morpholin-4-ylmethyl)phenyl)-3-(4-methoxyphenyl)-2-propylene-1-one | 21.42 |
| D3 | 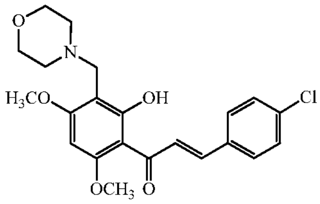<br>(E)-3-(4-chlorophenyl)-1-(2-hydroxy-4,6-dimethoxy-3-(morpholin-4-ylmethyl)phenyl)-2-propylene-1-one | 6.97 |
| D4 | 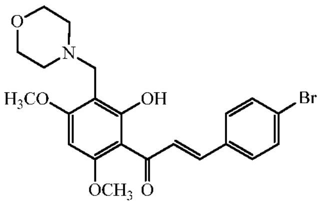<br>(E)-3-(4-bromophenyl)-1-(2-hydroxy-4,6-dimethoxy-3-(morpholin-4-ylmethyl)phenyl)-2-propylene-1-one | 5.52 |
| D5 | 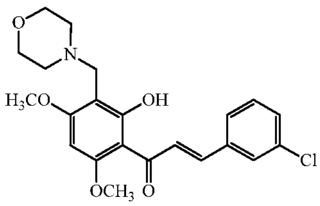<br>(E)-3-(3 -chlorophenyl)-1-(2-hydroxy-4,6-dimethoxy-3-(morpholinemethyl)phenyl)-2-propylene-1-one | 2.8 |
| D6 | 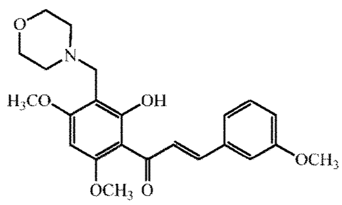<br>(E)-3-(3-methoxyphenyl)-1-(2-hydroxy-4,6-dimethoxy-3-(morpholinemethyl)-pheny)-2-propylene-1-one | 2.17 |

(continued)

| No. | Compound | $IC_{50}$ (μmol/L) |
|---|---|---|
| E31 | 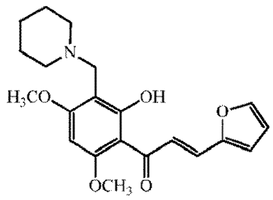 (E)-3-(furan-2-yl)-1-(2-hydroxy-4,6-dimethoxy-3-(piperidine-1-ylmetliyl)phenyl)-2-propylene-1-one | 8.52 |
| E32 | 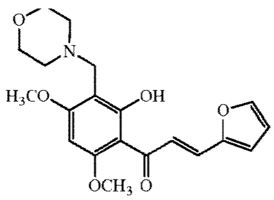 (E)-3-(furan-2-yl)-1-(2-hydroxy-4,6-dimethoxy-3-(morpholin-4-ylmethyl)phenyl)-2-propylene-1-one | 45.71 |

**Secondary screening**

After primary screenings, compounds which show inhibitory properties based upon one or more criteria, can be further tested in the same screen as used in the primary screening, for example, at different concentrations, or screened in one or more other test procedures, i.e., other cell lines and the like. As depicted in **Figure 1** and **Figure 2,** compounds C01, C02, C08, D03, D04, A01, A04, A09, A12, A24, B02, B03, B04, B05, B06, B08, B09, B11, B12, B15, B16 and B17 were tested at micro molar concentrations of 100 μM, 50 μM, 25 μM, 12.5 μM, 6.25 μM and 1 μM. Again, based upon the criteria of the formulator, compounds exhibiting desirable $IC_{50}$ values can be further evaluated for one or more desirable properties, i.e., cytotoxicity level, bioavailability, and the like.

As depicted in **Figure 3** and **Figure 4,** flavopiridol (control) and disclosed compounds B01, B06, C03, C05, C06, C07, D06, D07, D08, A07, A13, A16, A26, A28 and A29 were tested in the micro- and nanomolar concentration ranges (10 μM, 1 μM, 500 nM, 250 nM, 125 nM and 62.5 nM).

**Figure 5** shows a graphical representation of the absorbance values for the doxorubicin hydrochloride that was used as a positive control. The standard was not applied to the cells along with the flavonoid analogs, but was tested separately.

Compounds A04, A24, B01, D04 and flavopiridol were further evaluated and their $IC_{50}$ values are as shown in Table VIII below.

TABLE VIII

| Compound | $IC_{50}$ value |
|---|---|
| A04 | >1uM |
| A24 | >1uM |
| D04 | ~3uM |
| Control (flavopiridol) | ~130nM |
| B01 | ~100nM |

**Kinase Assay**

An invitrogen Z'-LYTE™ kinase assay kit (CDK2) can be used to test the inhibitory effects of compounds to CDK2 enzyme. Z'-LYTE kinase assays are suitable for screening inhibitors of tyrosine and serine/threonine protein kinase families. This test utilizes fluorescence resonance energy transfer (FRET) between courmarin and fluorescein

for the detection. Reaction progress is quantified by using a ratiometric approach (coumarin emission/fluorescein emission).

The kit utilizes a synthetic peptide substrate, which is labeled by a donor fluorophore (coumarin) and an acceptor fluorophore (fluorescein) that could make up a FRET pair. In the first reaction, the kinase transfers γ-phosphate of ATP to the substrate, while the inhibitors suppress the phosphorylation. After the first reaction, development reagent is added. It quenches the reaction and the protease cleaves non-phosphorylated peptide substrate at a higher rate comparable to that phosphorylated substrate. Cleavages disrupt FRET of the non-phosphorylated substrate, while phosphorylated substrates keep the FRET. Therefore, the reaction progress could be quantitated by calculating the emission ratio.

The reagent for the CDK2/CyclinA and Z'-LYTE™ kinase assay kit iss Ser/Thr 12 peptide. Determination of optimal CDK2 concentration and ATP concentration are carried out first. Following this initial testing, the kinase assay is conducted.

For tests conducted on the disclosed antitumor agents, kinase reaction buffer was diluted to the required concentration. Then the flavonoid compounds were diluted to 100 μM as highest concentration with 3-fold dilution to 1.7 nM by kinase buffer. The compounds were mixed with certain concentration of ATP (18 μM) and CDK2 (300 ng/mL) and were incubated at room temperature for 1hour. After that, the development solution was diluted to certain concentration, wherein the dilution is predicated on the kinase used for the assay. The samples were incubated for 1 hour at room temperature. The development reagent was added and the amount of substrate present was detected by using a Wallace 1420 multi plate reader (PerkinElmer Inc., Massachusetts, USA) at excited wavelength 355 nm and 460 nm. The percent inhibition and $IC_{50}$ value was then determined for each compound.

**Figure 6** shows the flavopiridol inhibitory curve for CDK2 $IC_{50}$= 19.5 nM.

### *In Vivo* Human Colon Xenograft Test

The disclosed compounds can be further evaluated by *in vivo* testing. Disclosed herein is a non-limiting example of an *in vivo* test wherein human colon carcinoma is injected into test animals.

### A. Test Protocol

#### 1. Drugs and treatment in animals

(*E*)-3-(2-Chlorophenyl)-1-(2-hydroxy-4,6-dimethoxy-3-(1,2,3,6-tetrahydropyridine-4-yl)phenyl)-2-propylene-1-one hydrochloride, B2, was dissolved in a pyrogen-free, sterile

PBS solution containing 1% dimethyl sulfoxide (DMSO). For daily injections, a freshly prepared solution was stored at 4 °C. Before injection the B2 solution was warmed up to 37°C and vigorously agitated in a vortex. B2 was administered through intraperitoneal injection (i.p.), 2.5, or 7.5 mg/kg/d, 20 days.

The positive control animals received 5 mg/kg/d doxorubicin intraperitoneally (i.p.), and the blank group mice received PBS solution containing 1% DMSO i.p., 5 mL/kg/d.

#### 2. Animals

Female BALB/cASlac-nu mice (SLAC LABORATORY ANIMAL, Shanghai), ages 4 to 5 weeks, were used in this procedure. Animals were maintained according to the guidelines established by the National Institutes of Health.

#### 3. Tumor cell lines and injection of tumor cells into animals

The Human Colon Carcinoma HCT116 were grown in RPMI-1640 medium with 10% fetal calf serum and L-glutamine, and the cells were maintained using standard tissue culture conditions.

For the production of s.c. tumors, $1\times10^7$ cells in 0.1 mL of medium without serum were inoculated in the right flank of mice. After the tumors were implanted and reached a palpable size, the mice were randomized into control and treatment groups (n =6)

#### 4. Evaluation of antitumor response in animals with localized s.c. tumors

Subcutaneous tumors were measured with caliper, and the weight of the animals recorded at least three times a week. Tumor volume was estimated from caliper measurements of two perpendicular dimensions of the tumor in millimeters using the formula:

$$\text{Tumor Volume (mm}^3\text{)} = \text{length (mm)} \times \text{width (mm)}^2/2$$

$$\text{Tumor Inhibition Ratio (\%)} = \text{(the blank group tumor weight average- the treatment group tumor weight average)/ the blank group tumor weight average} \times 100$$

Relative Tumor Volume (RTV) = $V_t$ / $V_0$. $V_0$: the tumor volume measured at the time of randomization; $V_t$: the tumor volume measured each time during the administration.

Relative Increment Ratio T/C (%)= the treatment group(T) RTV/ the blank control group(C) RTV×100. T/C(%)>40: inefficacy; T/C(%)≤40,and P<0.05: utility.

**B. Test Results**

During the administration, the weight of the mice in the two B2 treatment groups was similar to the blank control group, and no significant toxicity was found.

In HCT116 xenografts, we observed at the dosage of 2.5 mg/kg/day an optimal T/C of 19.0% (Table X) suggesting that at this dose level the compound was more effective at reducing tumor volume.

Tables IX and X show the *in vivo* antitumor activities of B2 against the human colon carcinoma xenograft.

TABLE IX

| Group | No. of Animals | | body wgt. (g) | | Tumor wgt. (g) | % inhibit. |
|---|---|---|---|---|---|---|
| | Initial | Final | Initial | Final | | |
| Control | 6 | 6 | 21.4±0.59 | 20.2±2.84 | 0.348±0.1626 | -- |
| B2 2.5 mg/kg/d×20d | 6 | 5 | 19.9±1.10 | 19.4±2.93 | 0.041±0.0093 | 88.2% |
| B2 7.5 mg/kg/d×20d | 7 | 6 | 18.0±1.83 | 20.0±1.75 | 0.437±0.3105 | -25.6% |
| Dox. 5.0 mg/kg/d×5d | 6 | 6 | 19.5±1.31 | 16.3±0.78 | | -- |

TABLE X

| Group | Tumor v ol. ($mm^3$) | | RTV | T/V (%) |
|---|---|---|---|---|
| | Initial | Final | | |
| Control | 57±26.3 | 601±248.6 | 15.08±4.906 | |
| B2 2.5 mg/kg/d×20d | 53±28.0 | 121±78.2 | 2.86±0.202 | 19.0 |
| B2 7.5 mg/kg/d×20d | 58±39.7 | 777±459.5 | 12.57±0.667 | 83.3 |
| Dox. 5.0 mg/kg/d×5d | | | | |

**METHODS**

The disclosed compounds can be used to prevent, abate, minimize, control, and/or lessen tumor metastasis in humans and animals. The disclosed compounds can also be used to slow the rate of primary tumor growth. The disclosed compounds when administered to a subject in need of treatment can be used to stop the spread of cancer cells. As such, the compounds disclosed herein can be administered as part of a combination therapy with one or more drugs or other pharmaceutical agents. When used as part of the combination therapy, the decrease in metastasis and reduction in primary tumor growth afforded by the disclosed compounds allows for a more effective and efficient use of any pharmaceutical or drug therapy being used to treat the patient. In addition, control of metastasis by the disclosed compound affords the subject a greater ability to concentrate the disease in one location.

Disclosed herein are methods for preventing metastasis of malignant tumors or other cancerous cells as well as to reduce the rate of tumor growth. The methods comprise administering an effective amount of one or more of the disclosed compounds to a subject diagnosed with a malignant tumor or cancerous cells or to a subject having a tumor

or cancerous cells.

Further disclosed herein is the use of the disclosed compounds for making a medicament for preventing metastasis of malignant tumors or other cancerous cells and for slowing tumor growth.

The following are non-limiting examples of cancers that can be treated by the disclosed methods and compositions: Acute Lymphoblastic; Acute Myeloid Leukemia; Adrenocortical Carcinoma; Adrenocortical Carcinoma, Childhood; Appendix Cancer; Basal Cell Carcinoma; Bile Duct Cancer, Extrahepatic; Bladder Cancer; Bone Cancer; Osteosarcoma and Malignant Fibrous Histiocytoma; Brain Stem Glioma, Childhood; Brain Tumor, Adult; Brain Tumor, Brain Stem Glioma, Childhood; Brain Tumor, Central Nervous System Atypical Teratoid/Rhabdoid Tumor, Childhood; Central Nervous System Embryonal Tumors; Cerebellar Astrocytoma; Cerebral Astrocytoma/Malignant Glioma; Craniopharyngioma; Ependymoblastoma; Ependymoma; Medulloblastoma; Medulloepithelioma; Pineal Parenchymal Tumors of Intermediate Differentiation; Supratentorial Primitive Neuroectodermal Tumors and Pineoblastoma; Visual Pathway and Hypothalamic Glioma; Brain and Spinal Cord Tumors; Breast Cancer; Bronchial Tumors; Burkitt Lymphoma; Carcinoid Tumor; Carcinoid Tumor, Gastrointestinal; Central Nervous System Atypical Teratoid/Rhabdoid Tumor; Central Nervous System Embryonal Tumors; Central Nervous System Lymphoma; Cerebellar Astrocytoma; Cerebral Astrocytoma/Malignant Glioma, Childhood; Cervical Cancer; Chordoma, Childhood; Chronic Lymphocytic Leukemia; Chronic Myelogenous Leukemia; Chronic Myeloproliferative Disorders; Colon Cancer; Colorectal Cancer; Craniopharyngioma; Cutaneous T-Cell Lymphoma; Esophageal Cancer; Ewing Family of Tumors; Extragonadal Germ Cell Tumor; Extrahepatic Bile Duct Cancer; Eye Cancer, Intraocular Melanoma; Eye Cancer, Retinoblastoma; Gallbladder Cancer; Gastric (Stomach) Cancer; Gastrointestinal Carcinoid Tumor; Gastrointestinal Stromal Tumor (GIST); Germ Cell Tumor, Extracranial; Germ Cell Tumor, Extragonadal; Germ Cell Tumor, Ovarian; Gestational Trophoblastic Tumor; Glioma; Glioma, Childhood Brain Stem; Glioma, Childhood Cerebral Astrocytoma; Glioma, Childhood Visual Pathway and Hypothalamic; Hairy Cell Leukemia; Head and Neck Cancer; ;Hepatocellular (Liver) Cancer; Histiocytosis, Langerhans Cell; Hodgkin Lymphoma; Hypopharyngeal Cancer; Hypothalamic and Visual Pathway Glioma; Intraocular Melanoma; Islet Cell Tumors; Kidney (Renal Cell) Cancer; Langerhans Cell Histiocytosis; Laryngeal Cancer; Leukemia, Acute Lymphoblastic; Leukemia, Acute Myeloid; Leukemia, Chronic Lymphocytic; Leukemia, Chronic Myelogenous; Leukemia, Hairy Cell; Lip and Oral Cavity Cancer; Liver Cancer; Lung Cancer, Non-Small Cell; Lung Cancer, Small Cell; Lymphoma, AIDS-Related; Lymphoma, Burkitt; Lymphoma, Cutaneous T-Cell; Lymphoma, Hodgkin; Lymphoma, Non-Hodgkin; Lymphoma, Primary Central Nervous System; Macroglobulinemia, Waldenström; Malignant Fibrous Histiocytoma of Bone and Osteosarcoma; Medulloblastoma; Melanoma; Melanoma, Intraocular (Eye); Merkel Cell Carcinoma; Mesothelioma; Metastatic Squamous Neck Cancer with Occult Primary; Mouth Cancer; Multiple Endocrine Neoplasia Syndrome, (Childhood); Multiple Myeloma/Plasma Cell Neoplasm; Mycosis Fungoides; Myelodysplastic Syndromes; Myelodysplastic/Myeloproliferative Diseases; Myelogenous Leukemia, Chronic; Myeloid Leukemia, Adult Acute; Myeloid Leukemia, Childhood Acute; Myeloma, Multiple; Myeloproliferative Disorders, Chronic; Nasal Cavity and Paranasal Sinus Cancer; Nasopharyngeal Cancer; Neuroblastoma; Non-Small Cell Lung Cancer; Oral Cancer; Oral Cavity Cancer; Oropharyngeal Cancer; Osteosarcoma and Malignant Fibrous Histiocytoma of Bone; Ovarian Cancer; Ovarian Epithelial Cancer; Ovarian Germ Cell Tumor; Ovarian Low Malignant Potential Tumor; Pancreatic Cancer; Pancreatic Cancer, Islet Cell Tumors; Papillomatosis; Parathyroid Cancer; Penile Cancer; Pharyngeal Cancer; Pheochromocytoma; Pineal Parenchymal Tumors of Intermediate Differentiation; Pineoblastoma and Supratentorial Primitive Neuroectodermal Tumors; Pituitary Tumor; Plasma Cell Neoplasm/Multiple Myeloma; Pleuropulmonary Blastoma; Primary Central Nervous System Lymphoma; Prostate Cancer; Rectal Cancer; Renal Cell (Kidney) Cancer; Renal Pelvis and Ureter, Transitional Cell Cancer; Respiratory Tract Carcinoma Involving the NUT Gene on Chromosome 15; Retinoblastoma; Rhabdomyosarcoma; Salivary Gland Cancer; Sarcoma, Ewing Family of Tumors; Sarcoma, Kaposi; Sarcoma, Soft Tissue; Sarcoma, Uterine; Sezary Syndrome; Skin Cancer (Nonmelanoma); Skin Cancer (Melanoma); Skin Carcinoma, Merkel Cell; Small Cell Lung Cancer; Small Intestine Cancer; Soft Tissue Sarcoma; Squamous Cell Carcinoma, Squamous Neck Cancer with Occult Primary, Metastatic; Stomach (Gastric) Cancer; Supratentorial Primitive Neuroectodermal Tumors; T-Cell Lymphoma, Cutaneous; Testicular Cancer; Throat Cancer; Thymoma and Thymic Carcinoma; Thyroid Cancer; Transitional Cell Cancer of the Renal Pelvis and Ureter; Trophoblastic Tumor, Gestational; Urethral Cancer; Uterine Cancer, Endometrial; Uterine Sarcoma; Vaginal Cancer; Vulvar Cancer; Waldenström Macroglobulinemia; and Wilms Tumor.

Disclosed herein is a method for treating carcinoma in a subject, comprising administering to the subject having a carcinoma an effective amount of one or more of the disclosed compounds. The methods include treating a subject with malignant tumors.

Also disclosed herein is a method for treating a subject diagnosed with cancer, comprising administering to the subject an effective amount of one or more of the disclosed compounds.

Further disclosed herein is a method for treating carcinoma in a subject, comprising administering to the subject having a carcinoma a composition comprising:

a) an effective amount of one or more of the disclosed compounds; and
b) an effective amount of an anticancer drug.

Still further disclosed herein is a method for treating carcinoma in a subject, comprising administering to the subject having a carcinoma a composition comprising:

a) an effective amount of one or more of the disclosed compounds; and
b) an effective amount of a compound that inhibits tumor growth.

Yet further disclosed herein is a method for treating a subject diagnosed with cancer, comprising administering to the subject diagnosed with cancer a composition comprising:

a) an effective amount of one or more of the disclosed compounds; and
b) an effective amount of an anticancer drug.

Still yrt further disclosed herein is a method for treating a subject diagnosed with cancer, comprising administering to the subject diagnosed with cancer a composition comprising:

a) an effective amount of one or more of the disclosed compounds; and
b) an effective amount of a compound that inhibits tumor growth.

Disclosed herein is the use of a compound disclosed herein for making a medicament for treating carcinoma.
Disclosed herein is the use of a compound disclosed herein for making a medicament for treating malignant tumors.
Disclosed herein is the use of a compound disclosed herein for making a medicament for reducing the volume of tumors in a subject having malignant tumors.

**COMPOSITONS**

Disclosed herein are compositions which can be used to prevent metastasis of cancer cells in a subject, the compositions comprising an effective amount of one or more of the compounds disclosed herein. Further disclosed herein are compositions that can be used to treat tumors in a human or other mammal.
One aspect relates to a composition comprising:

a) an effective amount of one or more compounds disclosed herein; and
b) one or more pharmaceutically acceptable ingredients.

Another aspect relates a composition comprising:

a) an effective amount of one or more compounds disclosed herein; and
b) an effective amount of one or chemotherapeutic agents;

wherein the disclosed compounds and the chemotherapeutic agents can be administered together or in any order.
One embodiment relates to a composition comprising:

a) an effective amount of one or more compounds disclosed herein; and
b) an effective amount of taxol;

wherein the disclosed compounds and taxol can be administered together or in any order.
Another embodiment relates to a composition comprising:

a) an effective amount of one or more compounds disclosed herein; and
b) an effective amount of gemcitabine;

wherein the disclosed compounds and gemcitabine can be administered together or in any order.
A further embodiment relate to a composition comprising:

a) an effective amount of one or more compounds disclosed herein; and
b) an effective amount of erlotinib;

wherein the disclosed compounds and erlotinib can be administered together or in any order.

A yet further embodiment relate to a composition comprising:

a) an effective amount of one or more compounds disclosed herein; and
b) an effective amount of doxil;

wherein the disclosed compounds and doxil can be administered together or in any order.

A still further embodiment relate to a composition comprising:

a) an effective amount of one or more compounds disclosed herein; and
b) an effective amount of irinortecan;

wherein the disclosed compounds and irinortecan can be administered together or in any order.

A still yet further embodiment relate to a composition comprising:

a) an effective amount of one or more compounds disclosed herein; and
b) an effective amount of bevacizumab;

wherein the disclosed compounds and bevacizumab can be administered together or in any order.

A still yet another further embodiment relate to a composition comprising:

a) an effective amount of one or more compounds disclosed herein; and
b) an effective amount of flavopiridol;

wherein the disclosed compounds and flavopiridol can be administered together or in any order.

A "chemotherapeutic agent" or "chemotherapeutic compound" is a chemical compound useful in the treatment of cancer. Chemotherapeutic cancer agents that can be used in combination with those disclosed herein include, but are not limited to, mitotic inhibitors (vinca alkaloids). These include vincristine, vinblastine, vindesine and Navelbine™ (vinorelbine-5'-noranhydroblastine). In yet other embodiments, chemotherapeutic cancer agents include topoisomerase I inhibitors, such as camptothecin compounds. As used herein, "camptothecin compounds" include Camptosar™ (irinotecan HCL), Hycamtin™ (topotecan HCL) and other compounds derived from camptothecin and its analogues. Another category of chemotherapeutic cancer agents that may be used in the methods and compositions of the present disclosure are podophyllotoxin derivatives, such as etoposide, teniposide and mitopodozide. The present disclosure further encompasses other chemotherapeutic cancer agents known as alkylating agents, which alkylate the genetic material in tumor cells. These include without limitation cisplatin, cyclophosphamide, nitrogen mustard, trimethylene thiophosphoramide, carmustine, busulfan, chlorambucil, belustine, uracil mustard, chlomaphazin, and dacarbazine. The present disclosure encompasses antimetabolites as chemotherapeutic agents. Examples of these types of agents include cytosine arabinoside, fluorouracil, methotrexate, mercaptopurine, azathioprime, and procarbazine. An additional category of chemotherapeutic cancer agents that may be used in the methods and compositions of the present disclosure include antibiotics. Examples include without limitation doxorubicin, bleomycin, dactinomycin, daunorubicin, mithramycin, mitomycin, mytomycin C, and daunomycin. There are numerous liposomal formulations commercially available for these compounds. The present disclosure further encompasses other chemotherapeutic cancer agents including without limitation anti-tumor antibodies, dacarbazine, azacytidine, amsacrine, melphalan, ifosfamide and mitoxantrone.

The disclosed compounds herein can be administered alone or in combination with other anti-tumor agents, including cytotoxic/antineoplastic agents and anti-angiogenic agents. Cytotoxic/anti-neoplastic agents are defined as agents which attack and kill cancer cells. Some cytotoxic/anti-neoplastic agents are alkylating agents, which alkylate the genetic material in tumor cells, e.g., cis-platin, cyclophosphamide, nitrogen mustard, trimethylene thiophosphoramide, carmustine, busulfan, chlorambucil, belustine, uracil mustard, chlomaphazin, and dacabazine. Other cytotoxic/anti-neoplastic agents are antimetabolites for tumor cells, e.g., cytosine arabinoside, fluorouracil, methotrexate, mercaptopuirine, azathioprime, and procarbazine. Other cytotoxic/anti-neoplastic agents are antibiotics, e.g., doxorubicin, bleomycin, dactinomycin, daunorubicin, mithramycin, mitomycin, mytomycin C, and daunomycin. There are numerous liposomal formulations commercially available for these compounds. Still other cytotoxic/anti-neoplastic agents are mitotic inhibitors (vinca alkaloids). These include vincristine, vinblastine and etoposide. Miscellaneous cytotoxic/anti-neoplastic agents include taxol and its derivatives, L-asparaginase, anti-tumor antibodies, dacarbazine, azacytidine, amsacrine, melphalan, VM-26, ifosfamide, mitoxantrone, and vindesine.

Anti-angiogenic agents are well known to those of skill in the art. Suitable anti-angiogenic agents for use in the methods and compositions of the present disclosure include anti-VEGF antibodies, including humanized and chimeric antibodies, anti-VEGF aptamers and antisense oligonucleotides. Other known inhibitors of angiogenesis include angiostatin, endostatin, interferons, interleukin 1 (including $\alpha$ and $\beta$) interleukin 12, retinoic acid, and tissue inhibitors of

metalloproteinase-1 and -2. (TIMP-1 and -2). Small molecules, including topoisomerases such as razoxane, a topoisomerase II inhibitor with anti-angiogenic activity, can also be used.

Other anti-cancer agents that can be used in combination with the disclosed compounds include, but are not limited to: acivicin; aclarubicin; acodazole hydrochloride; acronine; adozelesin; aldesleukin; altretamine; ambomycin; ametantrone acetate; aminoglutethimide; amsacrine; anastrozole; anthramycin; asparaginase; asperlin; azacitidine; azetepa; azotomycin; batimastat; benzodepa; bicalutamide; bisantrene hydrochloride; bisnafide dimesylate; bizelesin; bleomycin sulfate; brequinar sodium; bropirimine; busulfan; cactinomycin; calusterone; caracemide; carbetimer; carboplatin; carmustine; carubicin hydrochloride; carzelesin; cedefingol; chlorambucil; cirolemycin; cisplatin; cladribine; crisnatol mesylate; cyclophosphamide; cytarabine; dacarbazine; dactinomycin; daunorubicin hydrochloride; decitabine; dexormaplatin; dezaguanine; dezaguanine mesylate; diaziquone; docetaxel; doxorubicin; doxorubicin hydrochloride; droloxifene; droloxifene citrate; dromostanolone propionate; duazomycin; edatrexate; eflornithine hydrochloride; elsamitrucin; enloplatin; enpromate; epipropidine; epirubicin hydrochloride; erbulozole; esorubicin hydrochloride; estramustine; estramustine phosphate sodium; etanidazole; etoposide; etoposide phosphate; etoprine; fadrozole hydrochloride; fazarabine; fenretinide; floxuridine; fludarabine phosphate; fluorouracil; flurocitabine; fosquidone; fostriecin sodium; gemcitabine; gemcitabine hydrochloride; hydroxyurea; idarubicin hydrochloride; ifosfamide; ilmofosine; interleukin II (including recombinant interleukin II, or rIL2), interferon alfa-2a; interferon alfa-2b; interferon alfa-n1; interferon alfa-n3; interferon beta-I a; interferon gamma-I b; iproplatin; irinotecan hydrochloride; lanreotide acetate; letrozole; leuprolide acetate; liarozole hydrochloride; lometrexol sodium; lomustine; losoxantrone hydrochloride; masoprocol; maytansine; mechlorethamine hydrochloride; megestrol acetate; melengestrol acetate; melphalan; menogaril; mercaptopurine; methotrexate; methotrexate sodium; metoprine; meturedepa; mitindomide; mitocarcin; mitocromin; mitogillin; mitomalcin; mitomycin; mitosper; mitotane; mitoxantrone hydrochloride; mycophenolic acid; nocodazole; nogalamycin; ormaplatin; oxisuran; paclitaxel; pegaspargase; peliomycin; pentamustine; peplomycin sulfate; perfosfamide; pipobroman; piposulfan; piroxantrone hydrochloride; plicamycin; plomestane; porfimer sodium; porfiromycin; prednimustine; procarbazine hydrochloride; puromycin; puromycin hydrochloride; pyrazofurin; riboprine; rogletimide; safingol; safingol hydrochloride; semustine; simtrazene; sparfosate sodium; sparsomycin; spirogermanium hydrochloride; spiromustine; spiroplatin; streptonigrin; streptozocin; sulofenur; talisomycin; tecogalan sodium; tegafur; teloxantrone hydrochloride; temoporfin; teniposide; teroxirone; testolactone; thiamiprine; thioguanine; thiotepa; tiazofurin; tirapazamine; toremifene citrate; trestolone acetate; triciribine phosphate; trimetrexate; trimetrexate glucuronate; triptorelin; tubulozole hydrochloride; uracil mustard; uredepa; vapreotide; verteporfin; vinblastine sulfate; vincristine sulfate; vindesine; vindesine sulfate; vinepidine sulfate; vinglycinate sulfate; vinleurosine sulfate; vinorelbine tartrate; vinrosidine sulfate; vinzolidine sulfate; vorozole; zeniplatin; zinostatin; zorubicin hydrochloride. Other anti-cancer drugs include, but are not limited to: 20-epi-1,25 dihydroxyvitamin D3; 5-ethynyluracil; abiraterone; aclarubicin; acylfulvene; adecypenol; adozelesin; aldesleukin; ALL-TK antagonists; altretamine; ambamustine; amidox; amifostine; aminolevulinic acid; amrubicin; amsacrine; anagrelide; anastrozole; andrographolide; angiogenesis inhibitors; antagonist D; antagonist G; antarelix; anti-dorsalizing morphogenetic protein-1; antiandrogen, prostatic carcinoma; antiestrogen; antineoplaston; antisense oligonucleotides; aphidicolin glycinate; apoptosis gene modulators; apoptosis regulators; apurinic acid; ara-CDP-DL-PTBA; arginine deaminase; asulacrine; atamestane; atrimustine; axinastatin 1; axinastatin 2; axinastatin 3; azasetron; azatoxin; azatyrosine; baccatin III derivatives; balanol; batimastat; BCR/ABL antagonists; benzochlorins; benzoylstaurosporine; beta lactam derivatives; beta-alethine; betaclamycin B; betulinic acid; bFGF inhibitor; bicalutamide; bisantrene; bisaziridinylspermine; bisnafide; bistratene A; bizelesin; breflate; bropirimine; budotitane; buthionine sulfoximine; calcipotriol; calphostin C; camptothecin derivatives; canarypox IL-2; capecitabine; carboxamide-amino-triazole; carboxyamidotriazole; CaRest M3; CARN 700; cartilage derived inhibitor; carzelesin; casein kinase inhibitors (ICOS); castanospermine; cecropin B; cetrorelix; chlorlns; chloroquinoxaline sulfonamide; cicaprost; cis-porphyrin; cladribine; clomifene analogues; clotrimazole; collismycin A; collismycin B; combretastatin A4; combretastatin analogue; conagenin; crambescidin 816; crisnatol; cryptophycin 8; cryptophycin A derivatives; curacin A; cyclopentanthraquinones; cycloplatam; cypemycin; cytarabine ocfosfate; cytolytic factor; cytostatin; dacliximab; decitabine; dehydrodidemnin B; deslorelin; dexamethasone; dexifosfamide; dexrazoxane; dexverapamil; diaziquone; didemnin B; didox; diethylnorspermine; dihydro-5-azacytidine; dihydrotaxol, 9-; dioxamycin; diphenyl spiromustine; docetaxel; docosanol; dolasetron; doxifluridine; droloxifene; dronabinol; duocarmycin SA; ebselen; ecomustine; edelfosine; edrecolomab; eflornithine; elemene; emitefur; epirubicin; epristeride; estramustine analogue; estrogen agonists; estrogen antagonists; etanidazole; etoposide phosphate; exemestane; fadrozole; fazarabine; fenretinide; filgrastim; finasteride; flavopiridol; flezelastine; fluasterone; fludarabine; fluorodaunorunicin hydrochloride; forfenimex; formestane; fostriecin; fotemustine; gadolinium texaphyrin; gallium nitrate; galocitabine; ganirelix; gelatinase inhibitors; gemcitabine; glutathione inhibitors; hepsulfam; heregulin; hexamethylene bisacetamide; hypericin; ibandronic acid; idarubicin; idoxifene; idramantone; ilmofosine; ilomastat; imidazoacridones; imiquimod; immunostimulant peptides; insulin-like growth factor-1 receptor inhibitor; interferon agonists; interferons; interleukins; iobenguane; iododoxorubicin; ipomeanol, 4-; iroplact; irsogladine; isobengazole; isohomohalicondrin B; itasetron; jasplakinolide; kahalalide F; lamellarin-N triacetate; lanreotide; leinamycin; lenograstim; lentinan sulfate; leptolstatin; letrozole; leukemia inhibiting factor; leukocyte alpha interferon; leuprolide+estrogen+progesterone; leuprorelin; levamisole; liarozole; linear polyamine analogue; lipophilic disaccharide peptide; lipophilic platinum compounds; lissoclinamide 7; lobaplatin; lombricine; lometrexol; lonidamine; losoxantrone; lovastatin; loxoribine; lurtotecan; lutetium texaphyrin; lysofylline; lytic peptides; maitansine; mannostatin A; marimastat; masoprocol; maspin; matrilysin inhibitors; matrix metalloproteinase inhibitors; menogaril; merbarone; meterelin; methioninase; metoclopramide; MIF inhibitor; mifepristone; miltefosine; mirimostim; mismatched double stranded RNA; mitoguazone; mitolactol; mitomycin analogues; mitonafide; mitotoxin fibroblast growth factor-saporin; mitoxantrone; mofarotene; molgramostim; monoclonal antibody, human chorionic gonadotrophin; monophosphoryl lipid A+myobacterium cell wall sk; mopidamol; multiple drug resistance gene inhibitor; multiple tumor suppressor 1-based therapy; mustard anticancer agent; mycaperoxide B; mycobacterial cell wall extract; myriaporone; N-acetyldinaline; N-substituted benzamides; nafarelin; nagrestip; naloxone+pentazocine; napavin; naphterpin; nartograstim; nedaplatin; nemorubicin; neridronic acid; neutral endopeptidase; nilutamide; nisamycin; nitric oxide modulators; nitroxide antioxidant; nitrullyn; O6-benzylguanine; octreotide; okicenone; oligonucleotides; onapristone; ondansetron; ondansetron; oracin; oral cytokine inducer; ormaplatin; osaterone; oxaliplatin; oxaunomycin; paclitaxel; paclitaxel analogues; paclitaxel derivatives; palauamine; palmitoylrhizoxin; pamidronic acid; panaxytriol; panomifene; parabactin; pazelliptine; pegaspargase; peldesine; pentosan polysulfate sodium; pentostatin; pentrozole; perflubron; perfosfamide; perillyl alcohol; phenazinomycin; phenylacetate; phosphatase inhibitors; picibanil; pilocarpine hydrochloride; pirarubicin; piritrexim; placetin A; placetin B; plasminogen activator inhibitor; platinum complex; platinum compounds; platinum-triamine complex; porfimer sodium; porfiromycin; prednisone; propyl bis-acridone; prostaglandin J2; proteasome inhibitors; protein A-based immune modulator; protein kinase C inhibitor; protein kinase C inhibitors, microalgal; protein tyrosine phosphatase inhibitors; purine nucleoside phosphorylase inhibitors; purpurins; pyrazoloacridine; pyridoxylated hemoglobin polyoxyethylene conjugate; raf antagonists; raltitrexed; ramosetron; ras farnesyl protein transferase inhibitors; ras inhibitors; ras-GAP inhibitor; retelliptine demethylated; rhenium Re 186 etidronate; rhizoxin; ribozymes; RII retinamide; rogletimide; rohitukine; romurtide; roquinimex; rubiginone B1; ruboxyl; safingol; saintopin; SarCNU; sarcophytol A; sargramostim; Sdi 1 mimetics; semustine; senescence derived inhibitor 1; sense oligonucleotides; signal transduction inhibitors; signal transduction modulators; single chain antigen binding protein; sizofiran; sobuzoxane; sodium borocaptate; sodium phenylacetate; solverol; somatomedin binding protein; sonermin; sparfosic acid; spicamycin D; spiromustine; splenopentin; spongistatin 1; squalamine; stem cell inhibitor; stem-cell division inhibitors; stipiamide; stromelysin inhibitors; sulfinosine; superactive vasoactive intestinal peptide antagonist; suradista; suramin; swainsonine; synthetic glycosaminoglycans; tallimustine; tamoxifen methiodide; tauromustine; tazarotene; tecogalan sodium; tegafur; tellurapyrylium; telomerase inhibitors; temoporfin; temozolomide; teniposide; tetrachlorodecaoxide; tetrazomine; thaliblastine; thiocoraline; thrombopoietin; thrombopoietin mimetic; thymalfasin; thymopoietin receptor agonist; thymotrinan; thyroid stimulating hormone; tin ethyl etiopurpurin; tirapazamine; titanocene bichloride; topsentin; toremifene; totipotent stem cell factor; translation inhibitors; tretinoin; triacetyluridine; triciribine; trimetrexate; triptorelin; tropisetron; turosteride; tyrosine kinase inhibitors; tyrphostins; UBC inhibitors; ubenimex; urogenital sinus-derived growth inhibitory factor; urokinase receptor antagonists; vapreotide; variolin B; vector system, erythrocyte gene therapy; velaresol; veramine; verdins; verteporfin; vinorelbine; vinxaltine; vitaxin; vorozole; zanoterone; zeniplatin; zilascorb; and zinostatin stimalamer. In one embodiment, the anti-cancer drug is 5-fluorouracil, taxol, or leucovorin.

The term "effective amount" as used herein means "an amount of one or more phenylsulfamic acids, effective at dosages and for periods of time necessary to achieve the desired or therapeutic result." An effective amount may vary according to factors known in the art, such as the disease state, age, sex, and weight of the human or animal being treated. Although particular dosage regimes may be described in examples herein, a person skilled in the art would appreciated that the dosage regime may be altered to provide optimum therapeutic response. Thus, it is not possible to specify an exact "effective amount." For example, several divided doses may be administered daily or the dose may be proportionally reduced as indicated by the exigencies of the therapeutic situation. In addition, the compositions of the present disclosure can be administered as frequently as necessary to achieve a therapeutic amount.

Disclosed herein is a medicament comprising one or more compounds disclosed herein. Disclosed herein is the use of a disclosed compound for making a medicament suitable for use in reducing tumor volume.

While particular embodiments of the present disclosure have been illustrated and described, it would be obvious to those skilled in the art that various other changes and modifications can be made without departing from the spirit and scope of the disclosure. It is therefore intended to cover in the appended claims all such changes and modifications that are within the scope of this disclosure.

## Claims

**1.** A compound having the formula:

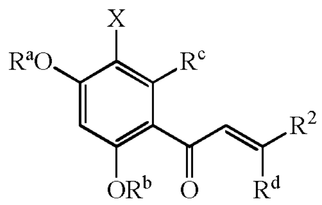

wherein:

$R^a$ and $R^b$ are each independently hydrogen or methyl;
$R^d$ is hydrogen;
$R^c$ is hydroxyl; or
$R^2$ is chosen from:

i) hydrogen;
ii) substituted or unsubstituted phenyl; or
iii) substituted or unsubstituted $C_1$-$C_5$ heteroaryl;

the substitutions on $R^2$ are each independently chosen from:

i) $C_1$-$C_4$ substituted or unsubstituted linear or branched alkyl;
ii) halogen;
iii) $OR^{10}$;
$R^{10}$ is chosen from:

a) hydrogen; or
b) $C_1$-$C_4$ substituted or unsubstituted linear or branched alkyl;

iv) $-N(R^{11a})(R^{11b})$;
$R^{11a}$ and $R^{11b}$ are each independently chosen from:

a) -H;
b) $C_1$-$C_4$ substituted or unsubstituted linear or branched alkyl;

v) -CN;
vi) $-NO_2$;

X is $-[CH_2]_yR^{23}$; $R^{23}$ is a N-substituted heterocyclic ring chosen from:

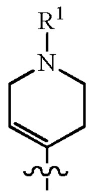

wherein $R^1$ is hydrogen or methyl; the index y is 0 or 1; or a ring having the formula:

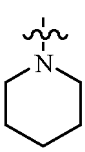

or

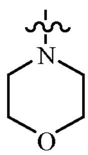

i) or
a pharmaceutically acceptable salt thereof.

**2.** The compound according to Claim 1, wherein $R^2$ is substituted or unsubstituted phenyl.

**3.** The compound according to any one of Claims 1 or 2, wherein X is a *N*-substituted heterocyclic ring having the formula:

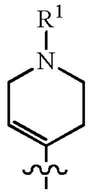

.

**4.** The compound according to any of Claims 1 to 3, wherein $R^a$ is methyl and $R^b$ is methyl.

**5.** A compound having the formula:

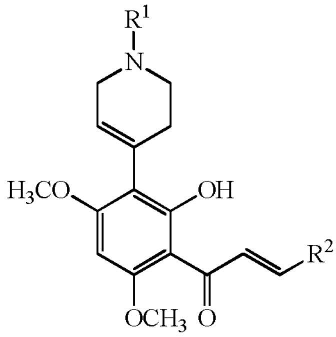

;

wherein $R^1$ is chosen from hydrogen or methyl;
$R^2$ is substituted or unsubstituted phenyl having the formula:

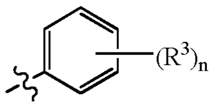

$R^3$ represents from 1 to 5 substitutions for hydrogen, each $R^3$ is independently chosen from:

i) $C_1$-$C_4$ substituted or unsubstituted linear or branched alkyl;
ii) halogen;
iii) $-OR^{10}$; wherein $R^{10}$ is hydrogen or methyl;
iv) $-N(R^{11a})(R^{11b})$; wherein $R^{11a}$ and $R^{11b}$ are each independently chosen from hydrogen or methyl;
v) $-C(O)R^{12}$; wherein $R^{12}$ is hydrogen or methyl
vi) -CN;
vii) $-NO_2$;
viii) $C_1$-$C_4$ linear or branched alkyl substituted by from 1 to 9 halogen atoms chosen from F, Cl, Br, or I; or
ix) $-SO_2NH_2$;

the index n is an integer from 0 to 5, such that when n is equal to 0, $R^3$ is absent and $R^2$ is equal to phenyl; or a pharmaceutically acceptable salt thereof.

**6.** A compound chosen from:

(E)-3-(2-chlorophenyl)-1-(2-hydroxy-4,6-dimethoxy-3-(1-methyl-1,2,3,6-tetrahydropyridine-4-yl)phenyl)-2-propylene-1-one;
(E)-1-(2-hydroxy-4,6-dimethoxy-3-(1-methyl-1,2,3,6-tetrahydropyridine-4-yl)phenyl)-3-(2-nitrophenyl)-2-propylene-1-one;

(E)-3-(2,3-dimethoxyphenyl)-1-(2-hydroxy-4,6-dimethoxy-3-(1-methyl-1,2,3,6-tetrahydropyridine-4-yl)phenyl)-2-propylene-1-one;
(E)-3-(3-chlorophenyl)-1-(2-hydroxy-4,6-dimethoxy-3-(1-methyl-1,2,3,6-tetrahydropyridine-4-yl)phenyl)-2-propylene-1-one;
(E)-1-(2-hydroxy-4,6-dimethoxy-3-(1-methyl-1,2,3,6-tetrahydropyridine-4-yl)phenyl)-3-(3-nitrophenyl)-2-propylene-1-one;
(E)-1-(2-hydroxy-4,6-dimethoxy-3-(1-methyl-1,2,3,6-tetrahydropyridine-4-yl)phenyl)-3-(4-hydroxyphenyl)-2-propylene-1-one;
(E)-3-(4-chlorophenyl)-1-(2-hydroxy-4,6-dimethoxy-3-(1-methyl-1,2,3,6-tetrahydropyridine-4-yl)phenyl)-2-propylene-1-one;
(E)-3-(3-bromophenyl)-1-(2-hydroxy-4,6-dimethoxy-3-(1-methyl-1,2,3,6-tetrahydropyridine-4-yl)phenyl)-2-propylene-1-one;
(E)-3-(4-cyanophenyl)-1-(2-hydroxy-4,6-dimethoxy-3-(1-methyl-1,2,3,6-tetrahydropyridine-4-yl)phenyl)-2-propylene-1-one;
(E)-1-(2-hydroxy-4,6-dimethoxy-3-(1-methyl-1,2,3,6-tetrahydropyridine-4-yl)phenyl)-3-(2,3-4-trimethoxyphenyl)-2-propylene-1-one;
(E)-1-(2-hydroxy-4,6-dimethoxy-3-(1-methyl-1,2,3,6-tetrahydropyridine-4-yl)phenyl)-3-(3,4,5-trimethoxyphenyl)-2-propylene-1-one;
1-(2-hydroxy-4,6-dimethoxy-3-(1,2,3,6-tetrahydropyridine-4-yl)phenyl)-2-propylene-1-one;
(E)-3-(2-chlorophenyl)-1-(2-hydroxy-4,6-dimethoxy-3-(1,2,3,6-tetrahydropyridine-4-yl)phenyl)-2-propylene-1-one;
(E)-1-(2-hydroxy-4,6-dimethoxy-3-(1,2,3,6-tetrahydropyridine-4-yl)phenyl)-3-(2-nitrophenyl)-2-propylene-1-one;
(E)-3-(2,3-dimethoxyphenyl)-1-(2-hydroxy-4,6-dimethoxy-3-(1,2,3,6-tetrahydropyridine-4-yl)phenyl)-2-propylene-1-one;
(E)-1-(2-hydroxy-4,6-dimethoxy-3-(1,2,3,6-tetrahydropyridine-4-yl)phenyl)-3-(3-methylphenyl)-2-propylene-1-one;
(E)-1-(2-hydroxy-4,6-dimethoxy-3-(1,2,3,6-tetrahydropyridine-4-yl)phenyl)-3-(3-methoxyphenyl)-2-propylene-1-one;
(E)-3-(3-chlorophenyl)-1-(2-hydroxy-4,6-dimethoxy-3-(1,2,3,6-tetrahydropyridine-4-yl)phenyl)-2-propylene-1-one;
(E)-1-(2-hydroxy-4,6-dimethoxy-3-(1,2,3,6-tetrahydropyridine-4-yl)phenyl)-3-(3-nitrophenyl)-2-propylene-1-one;
(E)-1-(2-hydroxy-4,6-dimethoxy-3-(1,2,3,6-tetrahydropyridine-4-yl)phenyl)-3-(4-methylphenyl)-2-propylene-1-one;
(E)-1-(2-hydroxy-4,6-dimethoxy-3-(1,2,3,6-tetrahydropyridine-4-yl)phenyl)-3-(4-methoxyphenyl)-2-propylene-1-one;
(E)-1-(2-hydroxy-4,6-dimethoxy-3-(1,2,3,6-tetrahydropyridine-4-yl)phenyl)-3-(4-hydroxyphenyl)-2-propylene-1-one;
(E)-3-(4-chlorophenyl)-1-(2-hydroxy-4,6-dimethoxy-3-(1,2,3,6-tetrahydropyridine-4-yl)phenyl)-2-propylene-1-one;
(E)-3-(3-bromophenyl)-1-(2-hydroxy-4,6-dimethoxy-3-(1,2,3,6-tetrahydropyridine-4-yl)phenyl)-2-propylene-1-one;
(E)-3-(4-dimethylaminophenyl)-1-(2-hydroxy-4,6-dimethoxy-3-(1,2,3,6-tetrahydropyridine-4-yl)phenyl)-2-propylene-1-one;
(E)-3-(4-cyanophenyl)-1-(2-hydroxy-4,6-dimethoxy-3-(1,2,3,6-tetrahydropyridine-4-yl)phenyl)-2-propylene-1-one;
(E)-1-(2-hydroxy-4,6-dimethoxy-3-(1,2,3,6-tetrahydropyridine-4-yl)phenyl)-3-(2,3,4-trimethoxyphenyl)-2-propylene-1-one;
(E)-1-(2-hydroxy-4,6-dimethoxy-3-(1,2,3,6-tetrahydropyridine-4-yl)phenyl)-3-(3,4,5-trimethoxyphenyl)-2-propylene-1-one;
(E)-1-(2-hydroxy-4,6-dimethoxy-3-(piperidin-1-ylmethyl)phenyl)-3-(4-methylphenyl)-2-propylene-1-one;
(E)-1-(2-hydroxy-4,6-dimethoxy-3-(piperidin-1-ylmethyl)phenyl)-3-(4-methoxyphenyl)-2-propylene-1-one;
(E)-1-(2-hydroxy-4,6-dimethoxy-3-(piperidin-1-ylmethyl)phenyl)-3-(3-methoxyphenyl)-2-propylene-1-one;
(E)-3-(3-chlorophenyl)-1-(2-hydroxy-4,6-dimethoxy-3-(piperidin-1-ylmethyl)phenyl-2-propylene-1-one;
(E)-1-(2-hydroxy-4,6-dimethoxy-3-(morpholin-4-ylmethyl)phenyl)-3-(4-methylphenyl)-2-propylene-1-one;
(E)-1-(2-hydroxy-4,6-dimethoxy-3-(morpholin-4-ylmethyl)phenyl)-3-(4-methoxyphenyl)-2-propylene-1-one;
(E)-3-(4-chlorophenyl)-1-(2-hydroxy-4,6-dimethoxy-3-(morpholin-4-ylmethyl)phenyl)-2-propylene-1-one;

(E)-3-(4-bromophenyl)-1-(2-hydroxy-4,6-dimethoxy-3-(morpholin-4-ylmethyl)phenyl)-2-propylene-1-one;
(E)-3-(3-chlorophenyl)-1-(2-hydroxy-4,6-dimethoxy-3-(morpholinemethyl)phenyl)-2-propylene-1-one;
(E)-3-(3-methoxyphenyl)-1-(2-hydroxy-4,6-dimethoxy-3-(morpholinemethyl)-phenyl)-2-propylene-1-one;
(E)-3-(furan-2-yl)-1-(2-hydroxy-4,6-dimethoxy-3-(1-methyl-1,2,3,6-tetrahydropyridine-4-yl)phenyl)-2-propylene-1-one;
(E)-3-(furan-2-yl)-1-(2-hydroxy-4,6-dimethoxy-3-(1,2,3,6-tetrahydropyridine-4-yl)phenyl)-2-propylene-1-one;
(E)-3-(furan-2-yl)-1-(2-hydroxy-4,6-dimethoxy-3-(piperidine-1-ylmethyl)phenyl)-2-propylene-1-one; and
(E)-3-(furan-2-yl)-1-(2-hydroxy-4,6-dimethoxy-3-(morpholin-4-ylmethyl)phenyl)-2-propylene-1-one.

**7.** The compound according to any of Claims 1 to 6, wherein the compounds are salts comprising anions chosen from chloride, bromide, iodide, sulfate, bisulfate, carbonate, bicarbonate, phosphate, formate, acetate, propionate, butyrate, pyruvate, lactate, oxalate, malonate, maleate, succinate, tartrate, fumarate, and citrate or wherein the compounds are salts comprising cations chosen from sodium, lithium, potassium, calcium, magnesium, and bismuth.

**8.** A composition comprising:

a) one or more compounds according to any of Claims 1 to 7; and
b) one or more pharmaceutically acceptable ingredients.

**9.** A composition comprising:

a) one or more compounds according to any one of Claims 1 to 7; and
b) an effective amount of one or more chemotherapeutic agents;

wherein the compounds and the chemotherapeutic agents can be administered together or in any order.

**10.** The composition according to Claim 9, wherein the chemotherapeutic agent is chosen from taxol, IL-2, gemcitabine, erlotinib, doxil, irinortecan, and bevacizumab.

**11.** A compound according to any one of Claims 1 to 7, for use in treating carcinoma or for use in treating malignant tumors.

**12.** A medicament comprising one or more compounds according to any one of Claims 1 to 7.

## Patentansprüche

**1.** Verbindung mit der Formel:

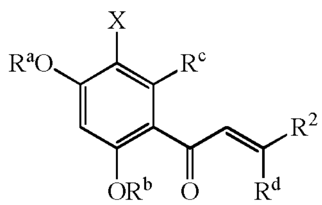

wobei:

$R^a$ und $R^b$ jeweils unabhängig Wasserstoff oder Methyl sind;
$R^d$ Wasserstoff ist;
$R^c$ Hydroxyl ist; oder
$R^2$ ausgewählt ist aus:

i) Wasserstoff;
ii) substituiertem oder unsubstituiertem Phenyl; oder
iii) substituiertem oder unsubstituiertem $C_1$-$C_5$ Heteroaryl;

wobei die Substitutionen an $R^2$ jeweils unabhängig ausgewählt sind aus:

i) $C_1$-$C_4$ substituiertem oder unsubstituiertem linearem oder verzweigtem Alkyl;
ii) Halogen;
iii) $OR^{10}$;
wobei $R^{10}$ ausgewählt ist aus:

a) Wasserstoff; oder
b) $C_1$-$C_4$ substituiertem oder unsubstituiertem linearem oder verzweigtem Alkyl;

iv) $-N(R^{11a})(R^{11b})$;
wobei $R^{11a}$ und $R^{11b}$ jeweils unabhängig ausgewählt sind aus:

a) -H;
b) $C_1$-$C_4$ substituiertem oder unsubstituiertem linearem oder verzweigtem Alkyl;

v) -CN;
vi) $-NO_2$;

X $-[CH_2]_yR^{23}$ ist; wobei $R^{23}$ ein N-substituierter heterocyclischer Ring ist, ausgewählt aus:

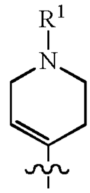

wobei $R^1$ Wasserstoff oder Methyl ist; der Index y 0 oder 1 ist; oder ein Ring mit der Formel:

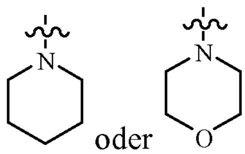
oder

i) oder
ein pharmazeutisch verträgliches Salz davon.

2. Verbindung nach Anspruch 1, wobei $R^2$ substituiertes oder unsubstituiertes Phenyl ist.

3. Verbindung nach irgendeinem der Ansprüche 1 oder 2, wobei X ein *N*-substituierter heterocyclischer Ring ist mit der Formel:

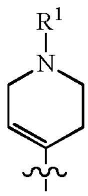
.

4. Verbindung nach irgendeinem der Ansprüche 1 bis 3, wobei $R^a$ Methyl ist und $R^b$ Methyl ist.

5. Verbindung mit der Formel:

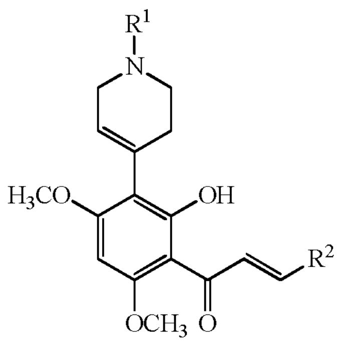

;

wobei $R^1$ ausgewählt ist aus Wasserstoff oder Methyl;
$R^2$ substituiertes oder unsubstituiertes Phenyl ist mit der Formel:

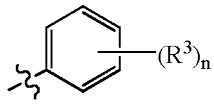

wobei $R^3$ 1 bis 5 Substitutionen für Wasserstoff darstellt, wobei jedes $R^3$ unabhängig ausgewählt ist aus:

i) $C_1$-$C_4$ substituiertem oder unsubstituiertem linearem oder verzweigtem Alkyl;
ii) Halogen;
iii) -$OR^{10}$; wobei $R^{10}$ Wasserstoff oder Methyl ist;
iv) -$N(R^{11a})(R^{11b})$; wobei $R^{11a}$ und $R^{11b}$ jeweils unabhängig ausgewählt sind aus Wasserstoff oder Methyl;
v) -$C(O)R^{12}$; wobei $R^{12}$ Wasserstoff oder Methyl ist
vi) -CN;
vii) -$NO_2$;
viii) $C_1$-$C_4$ linearem oder verzweigtem Alkyl, substituiert durch 1 bis 9 Halogenatome ausgewählt aus F, Cl, Br, oder I; oder
ix) -$SO_2NH_2$;

der Index n eine ganze Zahl von 0 bis 5 ist, so dass, wenn n gleich 0 ist, $R^3$ nicht vorhanden ist, und $R^2$ gleich Phenyl ist; oder
ein pharmazeutisch verträgliches Salz davon.

**6.** Verbindung, ausgewählt aus:

(E)-3-(2-Chlorphenyl)-1-(2-hydroxy-4,6-dimethoxy-3-(1-methyl-1,2,3,6-tetrahydropyridin-4-yl)phenyl)-2-propylen-1-on;
(E)-1-(2-Hydroxy-4,6-dimethoxy-3-(1-methyl-1,2,3,6-tetrahydropyridin-4-yl)phenyl)-3-(2-nitrophenyl)-2-propylen-1-on;
(E)-3-(2,3-Dimethoxyphenyl)-1-(2-hydroxy-4,6-dimethoxy-3-(1-methyl-1,2,3,6-tetrahydropyridin-4-yl)phenyl)-2-propylen-1-on;
(E)-3-(3-Chlorphenyl)-1-(2-hydroxy-4,6-dimethoxy-3-(1-methyl-1,2,3,6-tetrahydropyridin-4-yl)phenyl)-2-propylen-1-on;
(E)-1-(2-Hydroxy-4,6-dimethoxy-3-(1-methyl-1,2,3,6-tetrahydropyridin-4-yl)phenyl)-3-(3-nitrophenyl)-2-propylen-1-on;
(E)-1-(2-Hydroxy-4,6-dimethoxy-3-(1-methyl-1,2,3,6-tetrahydropyridin-4-yl)phenyl)-3-(4-hydroxyphenyl)-2-propylen-1-on;
(E)-3-(4-Chlorphenyl)-1-(2-hydroxy-4,6-dimethoxy-3-(1-methyl-1,2,3,6-tetrahydropyridin-4-yl)phenyl)-2-propylen-1-on;
(E)-3-(3-Bromphenyl)-1-(2-hydroxy-4,6-dimethoxy-3-(1-methyl-1,2,3,6-tetrahydropyridin-4-yl)phenyl)-2-propylen-1-on;
(E)-3-(4-Cyanophenyl)-1-(2-hydroxy-4,6-dimethoxy-3-(1-methyl-1,2,3,6-tetrahydropyridin-4-yl)phenyl)-2-propylen-1-on;

(E)-1-(2-Hydroxy-4,6-dimethoxy-3-(1-methyl-1,2,3,6-tetrahydropyridin-4-yl)phenyl)-3-(2,3-4-trimethoxyphenyl)-2-propylen-1-on;
(E)-1-(2-Hydroxy-4,6-dimethoxy-3-(1-methyl-1,2,3,6-tetrahydropyridin-4-yl)phenyl)-3-(3,4,5-trimethoxyphenyl)-2-propylen-1-on;
1-(2-Hydroxy-4,6-dimethoxy-3-(1,2,3,6-tetrahydropyridin-4-yl)phenyl)-2-propylen-1-on;
(E)-3-(2-Chlorphenyl)-1-(2-hydroxy-4,6-dimethoxy-3-(1,2,3,6-tetrahydropyridin-4-yl)phenyl)-2-propylen-1-on;
(E)-1-(2-Hydroxy-4,6-dimethoxy-3-(1,2,3,6-tetrahydropyridin-4-yl)phenyl)-3-(2-nitrophenyl)-2-propylen-1-on;
(E)-3-(2,3-Dimethoxyphenyl)-1-(2-hydroxy-4,6-dimethoxy-3-(1,2,3,6-tetrahydropyridin-4-yl)phenyl)-2-propylen-1-on;
(E)-1-(2-Hydroxy-4,6-dimethoxy-3-(1,2,3,6-tetrahydropyridin-4-yl)phenyl)-3-(3-methylphenyl)-2-propylen-1-on;
(E)-1-(2-Hydroxy-4,6-dimethoxy-3-(1,2,3,6-tetrahydropyridin-4-yl)phenyl)-3-(3-methoxyphenyl)-2-propylen-1-on;
(E)-3-(3-Chlorphenyl)-1-(2-hydroxy-4,6-dimethoxy-3-(1,2,3,6-tetrahydropyridin-4-yl)phenyl)-2-propylen-1-on;
(E)-1-(2-Hydroxy-4,6-dimethoxy-3-(1,2,3,6-tetrahydropyridin-4-yl)phenyl)-3-(3-nitrophenyl)-2-propylen-1-on;
(E)-1-(2-Hydroxy-4,6-dimethoxy-3-(1,2,3,6-tetrahydropyridin-4-yl)phenyl)-3-(4-methylphenyl)-2-propylen-1-on;
(E)-1-(2-Hydroxy-4,6-dimethoxy-3-(1,2,3,6-tetrahydropyridin-4-yl)phenyl)-3-(4-methoxyphenyl)-2-propylen-1-on;
(E)-1-(2-Hydroxy-4,6-dimethoxy-3-(1,2,3,6-tetrahydropyridin-4-yl)phenyl)-3-(4-hydroxyphenyl)-2-propylen-1-on;
(E)-3-(4-Chlorphenyl)-1-(2-hydroxy-4,6-dimethoxy-3-(1,2,3,6-tetrahydropyridin-4-yl)phenyl)-2-propylen-1-on;
(E)-3-(3-Bromphenyl)-1-(2-hydroxy-4,6-dimethoxy-3-(1,2,3,6-tetrahydropyridin-4-yl)phenyl)-2-propylen-1-on;
(E)-3-(4-Dimethylaminophenyl)-1-(2-hydroxy-4,6-dimethoxy-3-(1,2,3,6-tetrahydropyridin-4-yl)phenyl)-2-propylen-1-on;
(E)-3-(4-Cyanophenyl)-1-(2-hydroxy-4,6-dimethoxy-3-(1,2,3,6-tetrahydropyridin-4-yl)phenyl)-2-propylen-1-on;
(E)-1-(2-Hydroxy-4,6-dimethoxy-3-(1,2,3,6-tetrahydropyridin-4-yl)phenyl)-3-(2,3,4-trimethoxyphenyl)-2-propylen-1-on,
(E)-1-(2-Hydroxy-4,6-dimethoxy-3-(1,2,3,6-tetrahydropyridin-4-yl)phenyl)-3-(3,4,5-trimethoxyphenyl)-2-propylen-1-on,
(E)-1-(2-Hydroxy-4,6-dimethoxy-3-(piperidin-1-ylmethyl)phenyl)-3-(4-methylphenyl)-2-propylen-1-on;
(E)-1-(2-Hydroxy-4,6-dimethoxy-3-(piperidin-1-ylmethyl)phenyl)-3-(4-methoxyphenyl)-2-propylen-1-on;
(E)-1-(2-Hydroxy-4,6-dimethoxy-3-(piperidin-1-ylmethyl)phenyl)-3-(3-methoxyphenyl)-2-propylen-1-on;
(E)-3-(3-Chlorphenyl)-1-(2-hydroxy-4,6-dimethoxy-3-(piperidin-1-ylmethyl)phenyl-2-propylen-1-on;
(E)-1-(2-Hydroxy-4,6-dimethoxy-3-(morpholin-4-ylmethyl)phenyl)-3-(4-methylphenyl)-2-propylen-1-on;
(E)-1-(2-Hydroxy-4,6-dimethoxy-3-(morpholin-4-ylmethyl)phenyl)-3-(4-methoxyphenyl)-2-propylen-1-on;
(E)-3-(4-Chlorphenyl)-1-(2-hydroxy-4,6-dimethoxy-3-(morpholin-4-ylmethyl)phenyl)-2-propylen-1-on;
(E)-3-(4-Bromphenyl)-1-(2-hydroxy-4,6-dimethoxy-3-(morpholin-4-ylmethyl)phenyl)-2-propylen-1-on;
(E)-3-(3-Chlorphenyl)-1-(2-hydroxy-4,6-dimethoxy-3-(morpholinmethyl)phenyl)-2-propylen-1-on;
(E)-3-(3-Methoxyphenyl)-1-(2-hydroxy-4,6-dimethoxy-3-(morpholinmethyl)-phenyl)-2-propylen-1-on;
(E)-3-(Furan-2-yl)-1-(2-hydroxy-4,6-dimethoxy-3-(1-methyl-1,2,3,6-tetrahydropyridin-4-yl)phenyl)-2-propylen-1-on,
(E)-3-(Furan-2-yl)-1-(2-hydroxy-4,6-dimethoxy-3-(1,2,3,6-tetrahydropyridin-4-yl)phenyl)-2-propylen-1-on;
(E)-3-(Furan-2-yl)-1-(2-hydroxy-4,6-dimethoxy-3-(piperidin-1-ylmethyl)phenyl)-2-propylen-1-on; und
(E)-3-(Furan-2-yl)-1-(2-hydroxy-4,6-dimethoxy-3-(morpholin-4-ylmethyl)phenyl)-2-propylen-1-on.

**7.** Verbindung nach einem der Ansprüche 1 bis 6, wobei die Verbindungen Salze sind, die Anionen aufweisen, ausgewählt aus Chlorid, Bromid, Iodid, Sulfat, Bisulfat, Carbonat, Bicarbonat, Phosphat, Formiat, Acetat, Propionat, Butyrat, Pyruvat, Lactat, Oxalat, Malonat, Maleat, Succinat, Tartrat, Fumarat, und Citrat, oder wobei die Verbindungen Salze sind, die Kationen aufweisen, ausgewählt aus Natrium, Lithium, Kalium, Calcium, Magnesium, und Bismut.

**8.** Zusammensetzung, die

a) eine oder mehrere Verbindungen nach einem der Ansprüche 1 bis 7; und
b) einen oder mehrere pharmazeutisch verträgliche Bestandteile aufweist.

**9.** Zusammensetzung, die

a) eine oder mehrere Verbindungen nach einem der Ansprüche 1 bis 7; und
b) eine wirksame Menge eines oder mehrerer Chemotherapeutika aufweist;

wobei die Verbindungen und die Chemotherapeutika zusammen oder in beliebiger Reihenfolge verabreicht werden können.

**10.** Zusammensetzung nach Anspruch 9, wobei das Chemotherapeutikum ausgewählt ist aus Taxol, IL-2, Gemcitabin, Erlotinib, Doxil, Irinortecan, und Bevacizumab.

**11.** Verbindung nach einem der Ansprüche 1 bis 7 zur Verwendung bei der Behandlung von Karzinomen oder zur Verwendung bei der Behandlung von malignen Tumoren.

**12.** Medikament, das eine oder mehrere Verbindungen nach einem der Ansprüche 1 bis 7 aufweist.

## Revendications

**1.** Composé possédant la formule :

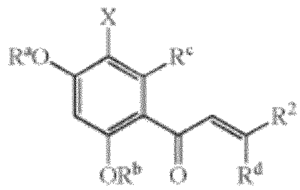

dans laquelle :

$R^a$ et $R^b$ sont chacun indépendamment hydrogène ou méthyle ;
$R^d$ est hydrogène ;
$R^c$ est hydroxyle ; ou
$R^2$ est choisi parmi :

i) hydrogène ;
ii) phényle substitué ou non substitué ; ou
iii) hétéroaryle en $C_1$-$C_5$ substitué ou non substitué ;

les substituants sur $R^2$ sont chacun indépendamment choisis parmi :

i) alkyle en $C_1$-$C_4$ linéaire ou ramifié substitué ou non substitué ;
ii) halogène ;
iii) $OR^{10}$ ;
$R^{10}$ est choisi parmi :

a) hydrogène ; ou
b) alkyle en $C_1$-$C_4$ linéaire ou ramifié substitué ou non substitué ;

iv) -N($R^{11a}$) ($R^{11b}$) ;
$R^{11a}$ et $R^{11b}$ sont chacun indépendamment choisis parmi :

a) -H ;
b) alkyle en $C_1$-$C_4$ linéaire ou ramifié substitué ou non substitué ;

v) -CN ;
vi) $-NO_2$ ;

X est $-[CH_2]_yR^{23}$ ; $R^{23}$ est un cycle hétérocyclique N-substitué choisi parmi :

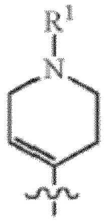

dans lequel $R^1$ est hydrogène ou méthyle ; l'indice y est 0 ou 1 ; ou un cycle possédant la formule :

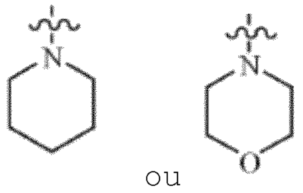

ou

un sel pharmaceutiquement acceptable de celui-ci.

**2.** Composé selon la revendication 1, dans lequel $R^2$ est un phényle substitué ou non substitué.

**3.** Composé selon l'une quelconque des revendications 1 ou 2, dans lequel X est un cycle hétérocyclique N-substitué possédant la formule :

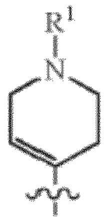

**4.** Composé selon l'une quelconque des revendications 1 à 3, dans lequel $R^a$ est méthyle et $R^b$ est méthyle.

**5.** Composé possédant la formule :

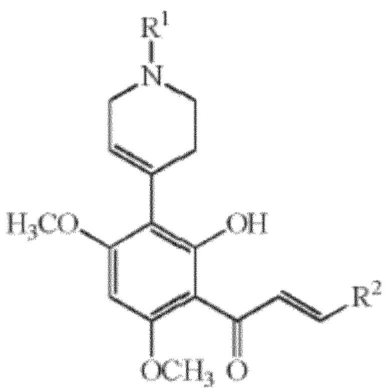 ;

dans laquelle $R^1$ est choisi parmi hydrogène ou méthyle ;
$R^2$ est un phényle substitué ou non substitué possédant la formule :

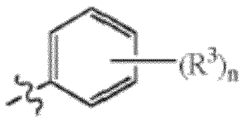

$R^3$ représente de 1 à 5 substituants de l'hydrogène, chaque $R^3$ est indépendamment choisi parmi :

i) alkyle en $C_1$-$C_4$ linéaire ou ramifié substitué ou non substitué ;
ii) halogène ;
iii) -$OR^{10}$ ; $R^{10}$ étant hydrogène ou méthyle ;
iv) -$N(R^{11a})(R^{11b})$ ; $R^{11a}$ et $R^{11b}$ étant chacun indépendamment choisis parmi hydrogène ou méthyle ;
v) -$C(O)R^{12}$ ; $R^{12}$ étant hydrogène ou méthyle ;
vi) -CN ;
vii) -$NO_2$ ;
viii) alkyle en $C_1$-$C_4$ linéaire ou ramifié substitué par 1 à 9 atomes d'halogène choisis parmi F, Cl, Br ou I ; ou
ix) -$SO_2NH_2$ ;

l'indice n est un entier de 0 à 5, de telle sorte que lorsque n est égal à 0, $R^3$ est absent et $R^2$ est égal à phényle ; ou un sel pharmaceutiquement acceptable de celui-ci.

**6.** Composé choisi parmi :

(E)-3-(2-chlorophényl)-1-(2-hydroxy-4,6-diméthoxy-3-(1-méthyl-1,2,3,6-tétrahydropyridin-4-yl)phényl)-2-propylén-1-one ;
(E)-1-(2-hydroxy-4,6-diméthoxy-3-(1-méthyl-1,2,3,6-tétrahydropyridin-4-yl)phényl)-3-(2-nitrophényl)-2-propylén-1-one ;
(E)-3-(2,3-diméthoxyphényl)-1-(2-hydroxy-4,6-diméthoxy-3-(1-méthyl-1,2,3,6-tétrahydropyridin-4-yl)phényl)-2-propylén-1-one ;
(E)-3-(3-chlorophényl)-1-(2-hydroxy-4,6-diméthoxy-3-(1-méthyl-1,2,3,6-tétrahydropyridin-4-yl)phényl)-2-propylén-1-one ;
(E)-1-(2-hydroxy-4,6-diméthoxy-3-(1-méthyl-1,2,3,6-tétrahydropyridin-4-yl)phényl)-3-(3-nitrophényl)-2-propylén-1-one ;
(E)-1-(2-hydroxy-4,6-diméthoxy-3-(1-méthyl-1,2,3,6-tétrahydropyridin-4-yl)phényl)-3-(4-hydroxyphényl)-2-propylén-1-one ;
(E)-3-(4-chlorophényl)-1-(2-hydroxy-4,6-diméthoxy-3-(1-méthyl-1,2,3,6-tétrahydropyridin-4-yl)phényl)-2-propylén-1-one ;
(E)-3-(3-bromophényl)-1-(2-hydroxy-4,6-diméthoxy-3-(1-méthyl-1,2,3,6-tétrahydropyridin-4-yl)phényl)-2-propylén-1-one ;
(E)-3-(4-cyanophényl)-1-(2-hydroxy-4,6-diméthoxy-3-(1-méthyl-1,2,3,6-tétrahydropyridin-4-yl)phényl)-2-propylén-1-one ;
(E)-1-(2-hydroxy-4,6-diméthoxy-3-(1-méthyl-1,2,3,6-tétrahydropyridin-4-yl)phényl)-3-(2,3,4-triméthoxyphényl)-2-propylén-1-one ;
(E)-1-(2-hydroxy-4,6-diméthoxy-3-(1-méthyl-1,2,3,6-tétrahydropyridin-4-yl)phényl)-3-(3,4,5-triméthoxyphényl)-2-propylén-1-one ;
1-(2-hydroxy-4,6-diméthoxy-3-(1,2,3,6-tétrahydropyridin-4-yl)phényl)-2-propylén-1-one ;
(E)-3-(2-chlorophényl)-1-(2-hydroxy-4,6-diméthoxy-3-(1,2,3,6-tétrahydropyridin-4-yl)phényl)-2-propylén-1-one ;
(E)-1-(2-hydroxy-4,6-diméthoxy-3-(1,2,3,6-tétrahydropyridin-4-yl)phényl)-3-(2-nitrophényl)-2-propylén-1-one ;
(E)-3-(2,3-diméthoxyphényl)-1-(2-hydroxy-4,6-diméthoxy-3-(1,2,3,6-tétrahydropyridin-4-yl)phényl)-2-propylén-1-one ;
(E)-1-(2-hydroxy-4,6-diméthoxy-3-(1,2,3,6-tétrahydropyridin-4-yl)phényl)-3-(3-méthylphényl)-2-propylén-1-one ;
(E)-1-(2-hydroxy-4,6-diméthoxy-3-(1,2,3,6-tétrahydropyridin-4-yl)phényl)-3-(3-méthoxyphényl)-2-propylén-1-one ;
(E)-3-(3-chlorophényl)-1-(2-hydroxy-4,6-diméthoxy-3-(1,2,3,6-tétrahydropyridin-4-yl)phényl)-2-propylén-1-one ;
(E)-1-(2-hydroxy-4,6-diméthoxy-3-(1,2,3,6-tétrahydropyridin-4-yl)phényl)-3-(3-nitrophényl)-2-propylén-1-one ;
(E)-1-(2-hydroxy-4,6-diméthoxy-3-(1,2,3,6-tétrahydropyridin-4-yl)phényl)-3-(4-méthylphényl)-2-propylén-1-one ;
(E)-1-(2-hydroxy-4,6-diméthoxy-3-(1,2,3,6-tétrahydropyridin-4-yl)phényl)-3-(4-méthoxyphényl)-2-propylén-1-one ;
(E)-1-(2-hydroxy-4,6-diméthoxy-3-(1,2,3,6-tétrahydropyridin-4-yl)phényl)-3-(4-hydroxyphényl)-2-propylén-1-one ;
(E)-3-(4-chlorophényl)-1-(2-hydroxy-4,6-diméthoxy-3-(1,2,3,6-tétrahydropyridin-4-yl)phényl)-2-propylén-1-one ;
(E)-3-(3-bromophényl)-1-(2-hydroxy-4,6-diméthoxy-3-(1,2,3,6-tétrahydropyridin-4-yl)phényl)-2-propylén-1-one ;
(E)-3-(4-diméthylaminophényl)-1-(2-hydroxy-4,6-diméthoxy-3-(1,2,3,6-tétrahydropyridin-4-yl)phényl)-2-propylén-1-one ;
(E)-3-(4-cyanophényl)-1-(2-hydroxy-4,6-diméthoxy-3-(1,2,3,6-tétrahydropyridin-4-yl)phényl)-2-propylén-1-one ;
(E)-1-(2-hydroxy-4,6-diméthoxy-3-(1,2,3,6-tétrahydropyridin-4-yl)phényl)-3-(2,3,4-triméthoxyphényl)-2-propylén-1-one ;
(E)-1-(2-hydroxy-4,6-diméthoxy-3-(1,2,3,6-tétrahydropyridin-4-yl)phényl)-3-(3,4,5-triméthoxyphényl)-2-propylén-1-one ;
(E)-1-(2-hydroxy-4,6-diméthoxy-3-(pipéridin-1-ylméthyl)phényl)-3-(4-méthylphényl)-2-propylén-1-one ;
(E)-1-(2-hydroxy-4,6-diméthoxy-3-(pipéridin-1-ylméthyl)phényl)-3-(4-méthoxyphényl)-2-propylén-1-one ;
(E)-1-(2-hydroxy-4,6-diméthoxy-3-(pipéridin-1-ylméthyl)phényl)-3-(3-méthoxyphényl)-2-propylén-1-one ;
(E)-3-(3-chlorophényl)-1-(2-hydroxy-4,6-diméthoxy-3-(pipéridin-1-ylméthyl)phényl)-2-propylén-1-one ;
(E)-1-(2-hydroxy-4,6-diméthoxy-3-(morpholin-4-ylméthyl)phényl)-3-(4-méthylphényl)-2-propylén-1-one ;
(E)-1-(2-hydroxy-4,6-diméthoxy-3-(morpholin-4-ylméthyl)phényl)-3-(4-méthoxyphényl)-2-propylén-1-one ;
(E)-3-(4-chlorophényl)-1-(2-hydroxy-4,6-diméthoxy-3-(morpholin-4-ylméthyl)phényl)-2-propylén-1-one ;
(E)-3-(4-bromophényl)-1-(2-hydroxy-4,6-diméthoxy-3-(morpholin-4-ylméthyl)phényl)-2-propylén-1-one ;
(E)-3-(3-chlorophényl)-1-(2-hydroxy-4,6-diméthoxy-3-(morpholineméthyl)phényl)-2-propylén-1-one ;
(E)-3-(3-méthoxyphényl)-1-(2-hydroxy-4,6-diméthoxy-3-(morpholineméthyl)phényl)-2-propylén-1-one ;
(E)-3-(furan-2-yl)-1-(2-hydroxy-4,6-diméthoxy-3-(1-méthyl-1,2,3,6-tétrahydropyridin-4-yl)phényl)-2-propylén-1-one ;
(E)-3-(furan-2-yl)-1-(2-hydroxy-4,6-diméthoxy-3-(1,2,3,6-tétrahydropyridin-4-yl)phényl)-2-propylén-1-one ;
(E)-3-(furan-2-yl)-1-(2-hydroxy-4,6-diméthoxy-3-(pipéridin-1-ylméthyl)phényl)-2-propylén-1-one ; et
(E)-3-(furan-2-yl)-1-(2-hydroxy-4,6-diméthoxy-3-(morpholin-4-ylméthyl)phényl)-2-propylén-1-one.

**7.** Composé selon l'une quelconque des revendications 1 à 6, les composés étant des sels comprenant des anions choisis parmi chlorure, bromure, iodure, sulfate, bisulfate, carbonate, bicarbonate, phosphate, formiate, acétate, propionate, butyrate, pyruvate, lactate, oxalate, malonate, maléate, succinate, tartrate, fumarate et citrate ou les composés étant des sels comprenant des cations choisis parmi sodium, lithium, potassium, calcium, magnésium et bismuth.

**8.** Composition comprenant :

a) un ou plusieurs composés selon l'une quelconque des revendications 1 à 7 ; et
b) un ou plusieurs ingrédients pharmaceutiquement acceptables.

**9.** Composition comprenant :

a) un ou plusieurs composés selon l'une quelconque des revendications 1 à 7 ; et
b) une quantité efficace d'un ou plusieurs agents chimiothérapeutiques ;

dans laquelle les composés et les agents chimiothérapeutiques peuvent être administrés ensemble ou dans n'importe quel ordre.

**10.** Composition selon la revendication 9, dans laquelle l'agent chimiothérapeutique est choisi parmi le taxol, l'IL-2, la gemcitabine, l'erlotinib, le doxil, l'irinotécan et le bévacizumab.

**11.** Composé selon l'une quelconque des revendications 1 à 7, pour utilisation dans le traitement de carcinomes et pour utilisation dans le traitement de tumeurs malignes.

**12.** Médicament comprenant un ou plusieurs composés selon l'une quelconque des revendications 1 à 7.

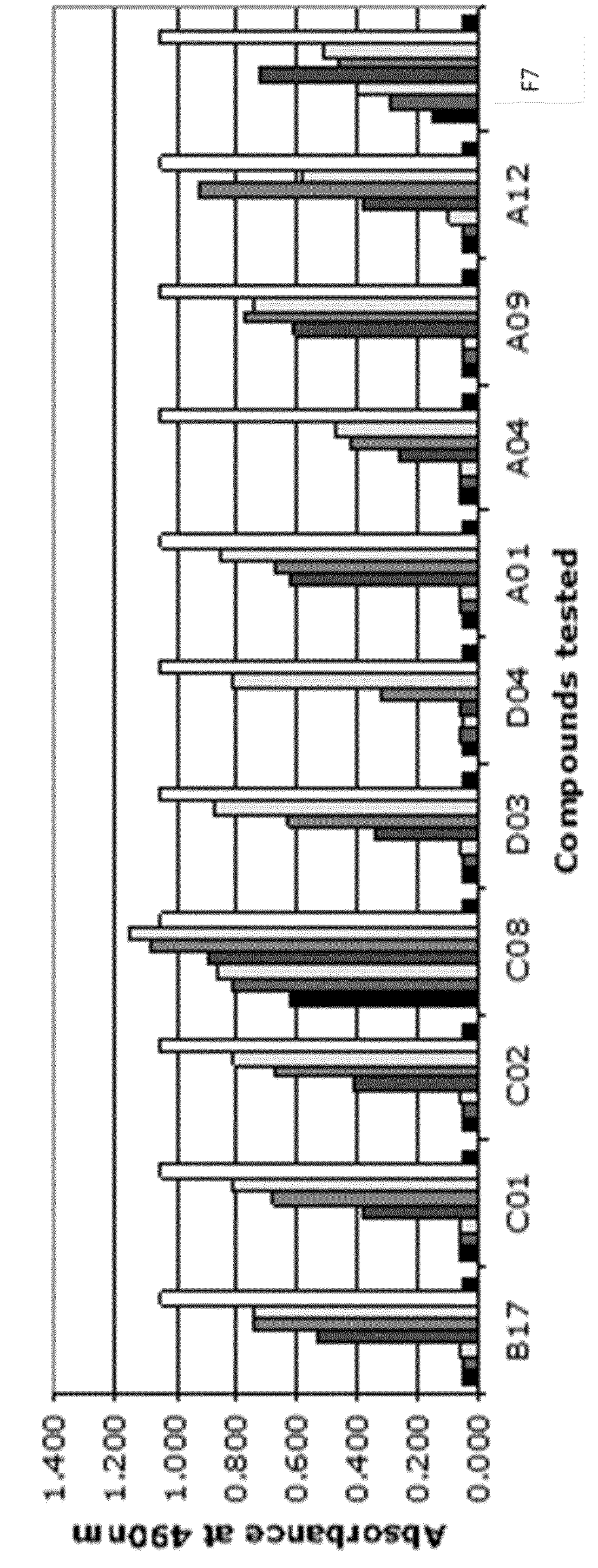
Absorbance at 490nm
1.400
1.200
1.000
0.800
0.600
0.400
0.200
0.000
B17
C01
C02
C08
D03
D04
A01
A04
A09
A12
F7
Compounds tested

**Fig. 1**

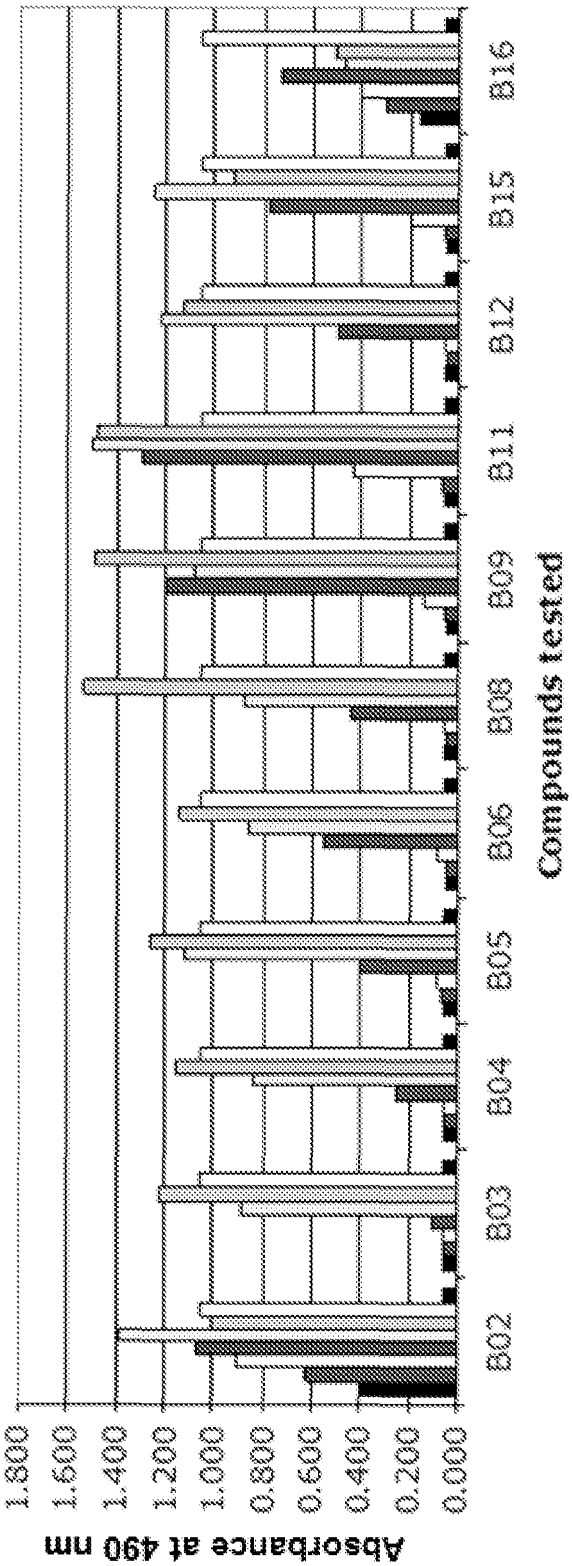
Absorbance at 490 nm
1.800
1.600
1.400
1.200
1.000
0.800
0.600
0.400
0.200
0.000
B02
B03
B04
B05
B06
B08
B09
B11
B12
B15
B16
Compounds tested

Fig. 2

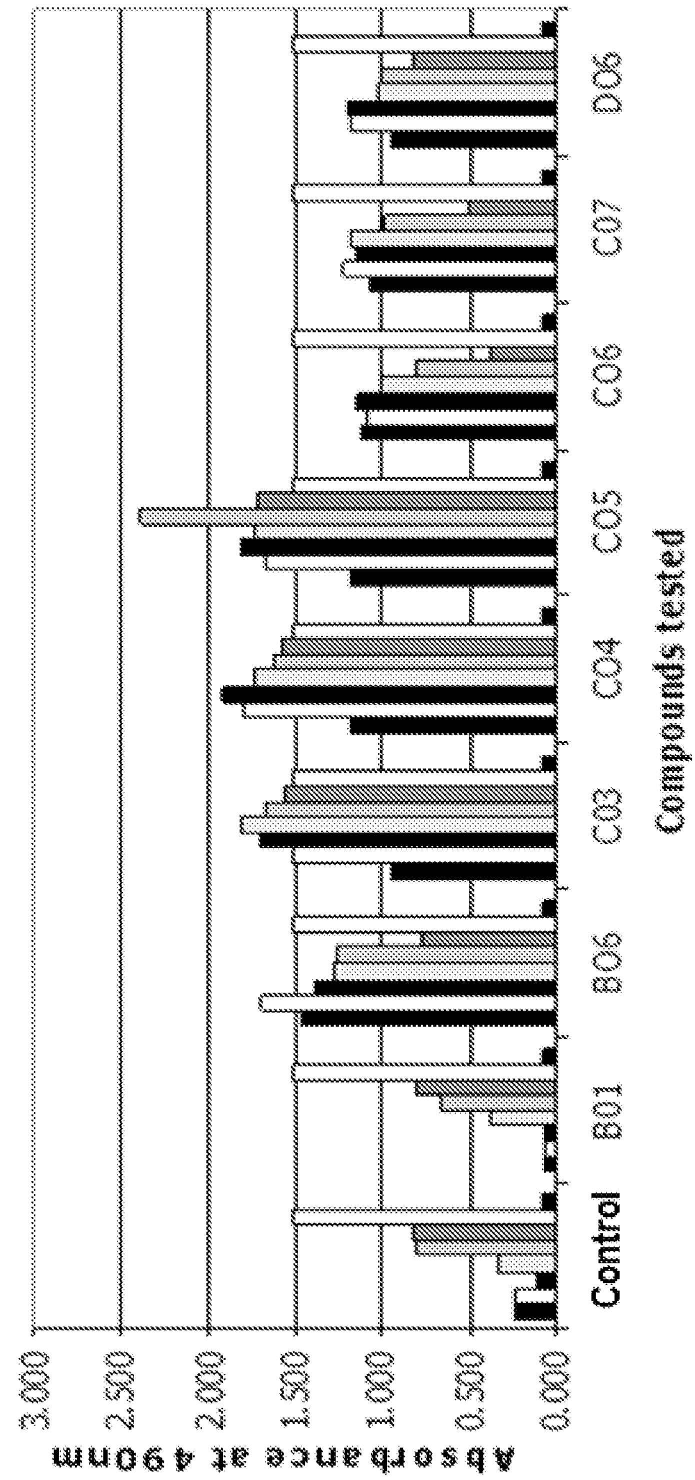
Absorbance at 490nm
3.000
2.500
2.000
1.500
1.000
0.500
0.000
Control
B01
B06
C03
C04
C05
C06
C07
D06
Compounds tested

Fig. 3

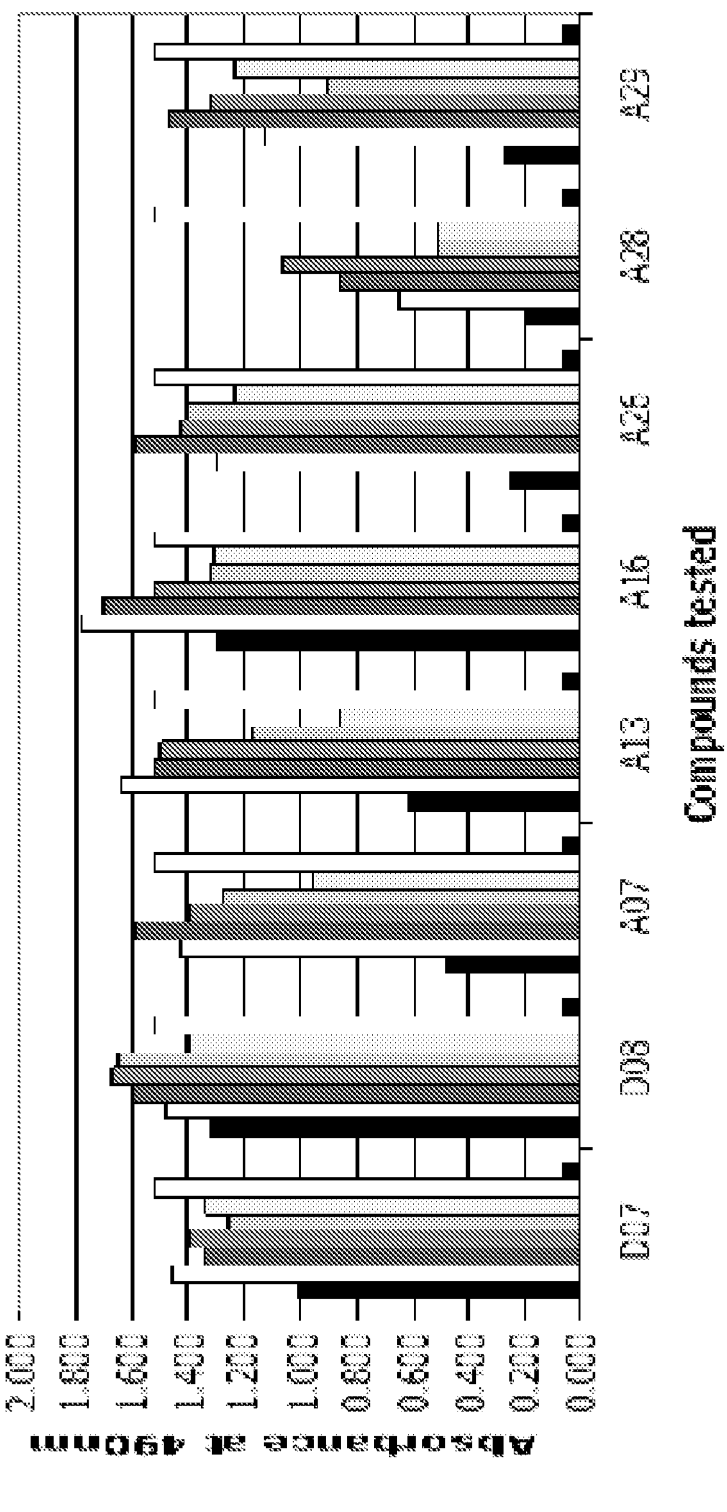
Absorbance at 490nm
2.000
1.800
1.600
1.400
1.200
1.000
0.800
0.600
0.400
0.200
0.000
D07
D08
A07
A13
A16
A26
A28
A29
Compounds tested

Fig. 4

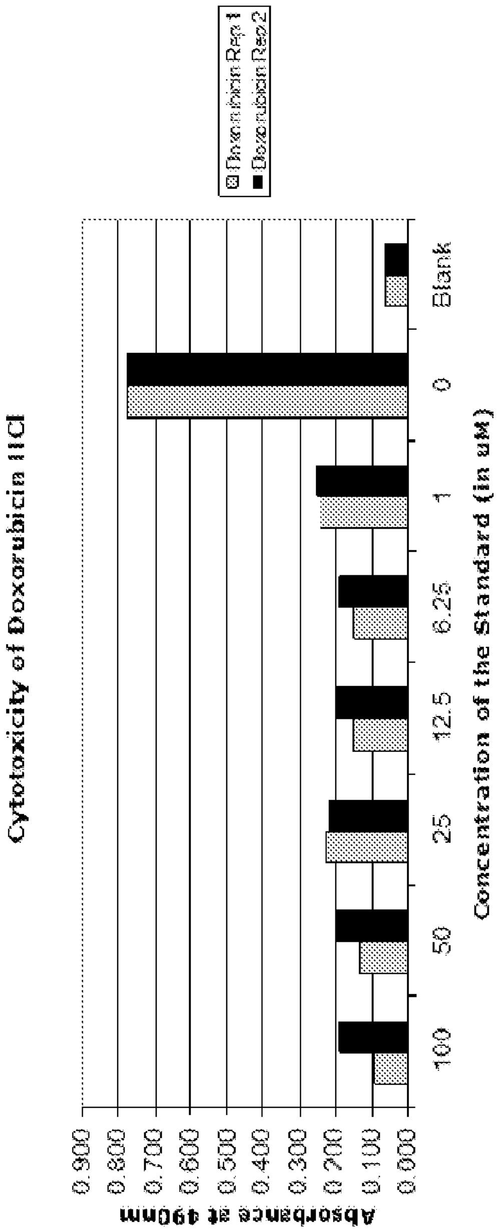
Cytotoxicity of Doxorubicin HCl
Doxorubicin Rep 1
Doxorubicin Rep 2
Absorbance at 490nm
0.900
0.800
0.700
0.600
0.500
0.400
0.300
0.200
0.100
0.000
100
50
25
12.5
6.25
1
0
Blank
Concentration of the Standard (in uM)

Fig. 5

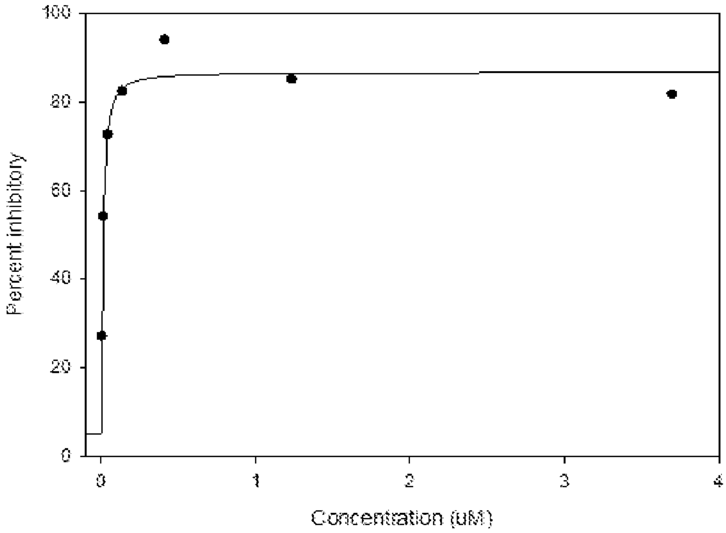


Fig. 6

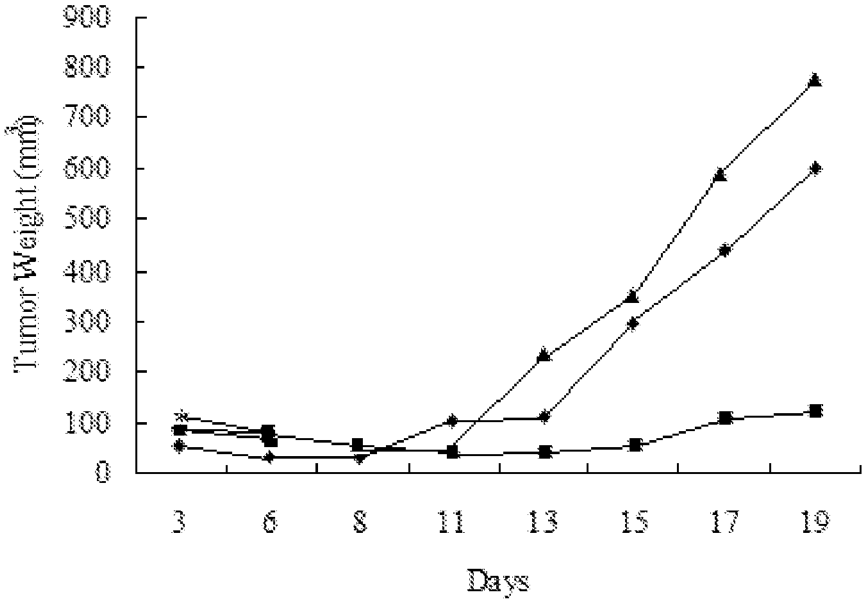
Tumor Weight (mm3)
900
800
700
600
500
400
300
200
100
0
3
6
8
11
13
15
17
19
Days

**Fig. 7**

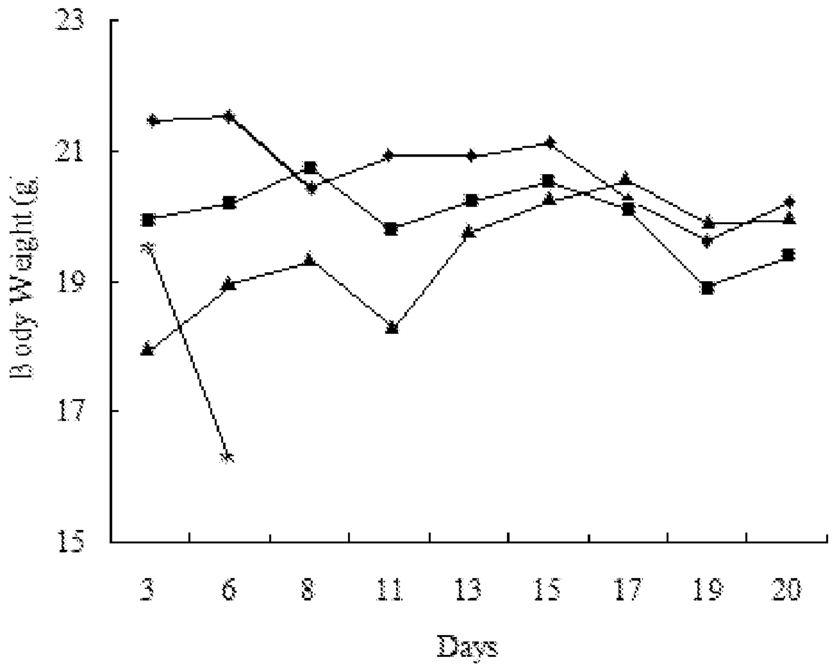
Body Weight (g)
23
21
19
17
15
3
6
8
11
13
15
17
19
20
Days

**Fig. 8**

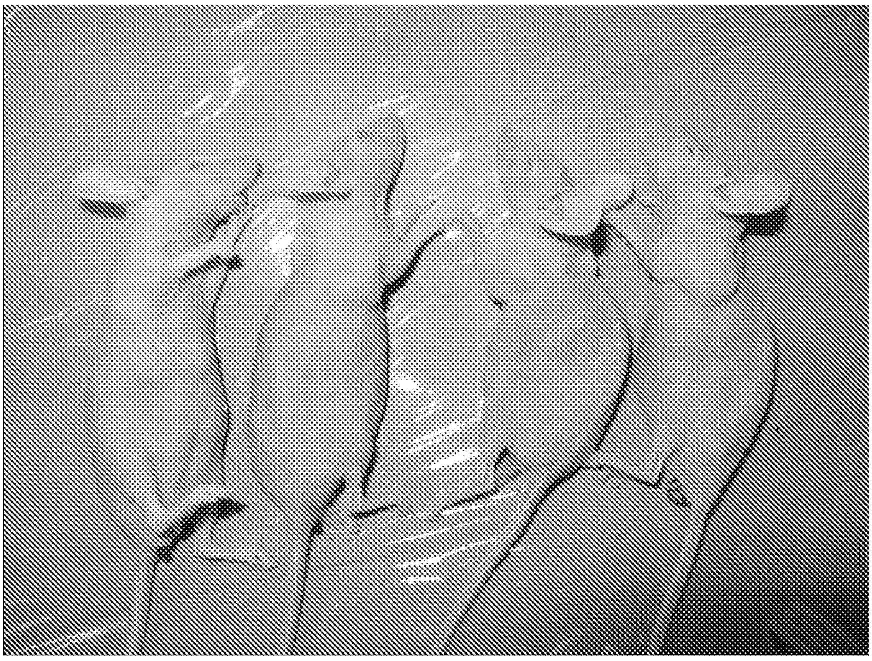

Fig. 9

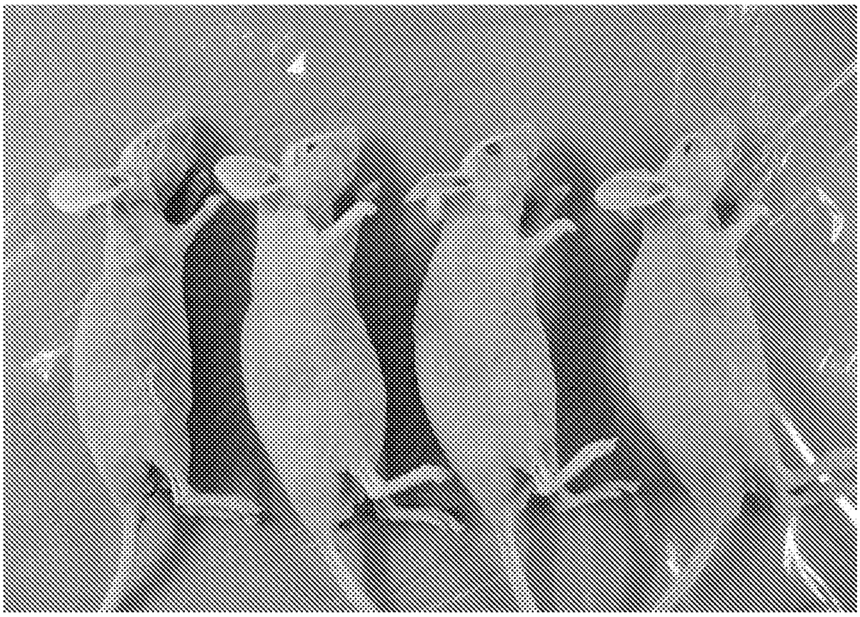

Fig. 10

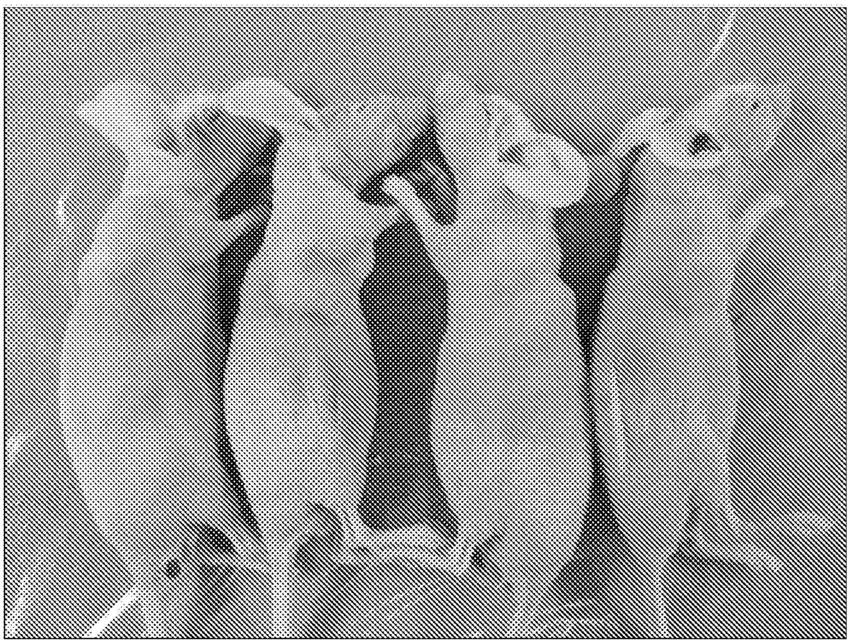

Fig. 11

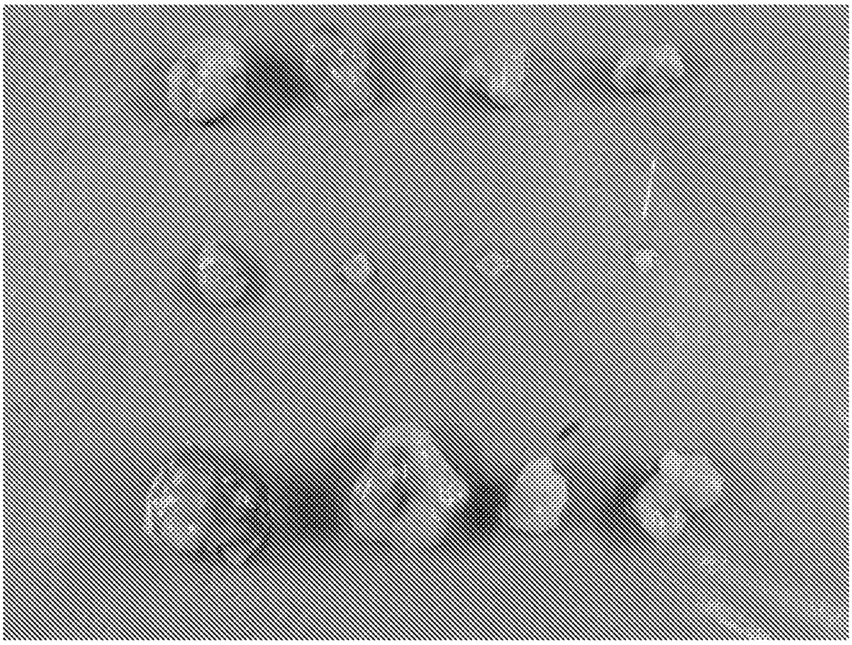

Fig. 12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20040106581 A1


### Non-patent literature cited in the description


- **REN, W. et al.** Flavonoids: promising anticancer agents. *Med Res Rev,* 2003, vol. 23 (4), 519-34
- **WANG, H.K. et al.** Recent advances in the discovery and development of flavonoids and their analogues as antitumor and anti-HIV agents. *Adv. Exp. Med. Biol.,* 1998, vol. 439, 191-225
- **WANG, H.K.** The therapeutic potential of flavonoids. *Expert Opin. Investig. Drugs,* 2000, vol. 9 (9), 2103-19
- **ZHAO, L. et al.** Mechanisms of tumor vascular shutdown induced by 5,6-dimethylxanthenone-4-acetic acid (DMXAA): Increased tumor vascular permeability. *Int. J. Cancer,* 2005, vol. 116 (2), 322-6
- **GALLO, D. et al.** Antitumour activity of the silybin-phosphatidylcholine complex, IdB 1016, against human ovarian cancer. *Eur. J. Cancer,* 2003, vol. 39 (16), 2403-10
- **DIMMOCK, J.R. et al.** Bioactivities of chalcones. *Curr. Med. Chem.,* 1999, vol. 6 (12), 1125-49
- *CHEMICAL ABSTRACTS,* 1003-29-8
- *CHEMICAL ABSTRACTS,* 10111-08-7
- *CHEMICAL ABSTRACTS,* 65373-52-6
- *CHEMICAL ABSTRACTS,* 32829-25-7
- *CHEMICAL ABSTRACTS,* 3364-80-5
- *CHEMICAL ABSTRACTS,* 98-03-3
- **MOSMANN, T.** Rapid colorimetric assay for cellular growth and survival: application to proliferation and cytotoxicity assays. *J. Immunol. Methods,* 1983, vol. 65 (1-2), 55-63